(19) Europäisches Patentamt / European Patent Office / Office européen des brevets

(11) EP 2 977 467 B1

(12) EUROPEAN PATENT SPECIFICATION

(45) Date of publication and mention of the grant of the patent:
**11.04.2018 Bulletin 2018/15**

(51) Int Cl.:
***C12Q 1/68*** *(2018.01)*

(21) Application number: **15176249.9**

(22) Date of filing: **10.07.2015**

# (54) METHOD, USE OF MARKER, AND DETERMINATION DEVICE FOR OBTAINING INFORMATION ON PLURAL TYPES OF CANCERS

VERFAHREN, VERWENDUNG VON MARKER UND BESTIMMUNGSVORRICHTUNG ZUR GEWINNUNG VON INFORMATIONEN ÜBER EINE VIELZAHL VON KREBSARTEN

PROCÉDÉ, UTILISATION DE MARQUEUR ET DISPOSITIF DE DÉTERMINATION POUR OBTENIR DES INFORMATIONS SUR PLUSIEURS TYPES DE CANCERS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.07.2014 JP 2014143326**

(43) Date of publication of application:
**27.01.2016 Bulletin 2016/04**

(73) Proprietors:
- **Sysmex Corporation**
  **Kobe-shi, Hyogo 651-0073 (JP)**
- **The University of Tokyo**
  **Bunkyo-ku,**
  **Tokyo 113-8654 (JP)**

(72) Inventors:
- **Sakai, Ayako**
  **Kobe-shi**
  **Hyogo 651-0073 (JP)**
- **Tai, Kaya**
  **Kobe-shi**
  **Hyogo 651-0073 (JP)**
- **Nagae, Genta**
  **Tokyo 113-8654 (JP)**
- **Aburatani, Hiroyuki**
  **Kobe-shi**
  **Hyogo 651-0073 (JP)**

(74) Representative: **Hoffmann Eitle**
**Patent- und Rechtsanwälte PartmbB**
**Arabellastraße 30**
**81925 München (DE)**

(56) References cited:
**WO-A1-2009/128453 WO-A2-2012/167145**

- **SHAMES DAVID S ET AL: "A genome-wide screen for promoter methylation in lung cancer identifies novel methylation markers for multiple malignancies", PLOS MEDICINE, PUBLIC LIBRARY OF SCIENCE, US, vol. 3, no. 12, 1 December 2006 (2006-12-01), XP002527542, ISSN: 1549-1676, DOI: 10.1371/JOURNAL.PMED.0030486**
- **M. EHRICH ET AL: "Cytosine methylation profiling of cancer cell lines", PROCEEDINGS OF THE NATIONAL ACADEMY OF SCIENCES, vol. 105, no. 12, 25 March 2008 (2008-03-25), pages 4844-4849, XP055102833, ISSN: 0027-8424, DOI: 10.1073/pnas.0712251105**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 977 467 B1

**Description**

TECHNICAL FIELD

The present invention relates to a method, use of marker, and a determination device for obtaining information on plural types of cancers in a subject.

BACKGROUND

In recent years, it has become apparent that abnormal DNA methylation, i.e., gene silencing due to DNA methylation, is involved in development and progress of diseases such as cancers. Attempts thus have been made to diagnose the diseases such as cancers by analyzing the methylation status of DNA for various genes.

For instance, US 2012/0190024 A describes that a DNA sample extracted from a biological sample is used to analyze the methylation status of CpG sites in a gene region such as PCDHGA10, PCDHB6, or LBX2 to determine the presence or absence of cells derived from epithelial cancer such as breast cancer in the biological sample.

Furthermore, US 2012/0178634 A describes that a DNA sample extracted from a biological sample is used to analyze the methylation status of CpG sites in a gene region such as COL4A2, AOX1, DUSP26, ELMO1, STOX2, EDIL3, ZNF447, or EFHD1 to determine the presence or absence of cells derived from colon cancer, breast cancer, or the like in the biological sample.

Moreover, US 2013/0130242 A describes that a DNA sample extracted from a biological sample is used to analyze the methylation status of CpG sites in a gene region such as HOXA9, KCNQ1DN, RAPSN, FZD9, or CDH22 to determine the presence or absence of cells derived from colon cancer or the like in the biological sample. US 2013/0130242 A also describes that a DNA sample extracted from a biological sample is used to analyze the methylation status of CpG sites in a gene region such as LBX2, PAX9, ADD3, CCDC61, ZIC4, CGB7, TOX, or TNNI3 to determine the presence or absence of cells derived from breast cancer or the like in the biological sample.

WO 2009128453 A1 describes the use of the methylation of genomic DNA of a Zar1 gene as a marker for proliferative disease.

Advances have been made in the search for a marker for the specific type of cancer to assist definite diagnosis. However, the search for a marker applicable to various types of cancers has barely advanced. Thus, there is desired a further development of a novel molecular marker useful in a method for detecting cancer cells from various types of cancers using the methylation analysis of genes. It is thus an object of the present invention to provide a method for obtaining information on cancer cells derived from various cancers by identifying such a novel marker and analyzing methylation status of the marker. It is another object of the present disclosure to provide a kit suitably used for the method.

SUMMARY OF THE INVENTION

The present inventors thoroughly conducted researches, and have come to complete the present invention by identifying a genetic region of GDF7 gene as a novel marker.

The present invention is defined by the appended claims. Also disclosed is the following:

(1) A method for obtaining information on plural types of cancers includes the steps of: preparing a DNA sample from a biological sample collected from a subject; analyzing methylation status of a CpG site in at least one gene selected from a group consisting of GDF7, ZNF132, CPXM1, RPL39L, DOK1, FUZ, MGAT3, and EHD3 genes in the DNA sample obtained in the preparing step; and obtaining information on plural types of cancers in the subject based on an analysis result obtained in the analyzing step.

(2) The method according to (1), wherein the plural types of cancers are two or more cancers selected from a group consisting of brain tumor, hepatocellular cancer, colon cancer, gastric cancer, uterine body cancer, lung cancer, breast cancer, prostate cancer, and renal cell cancer.

(3) The method according to (1) or (2), wherein the analyzing step is a step of analyzing the presence or absence of methylation of at least one CpG site.

(4) The method according to (3), wherein the information on the plural types of cancers is information related to the presence or absence of cancer cells; and the information obtaining step is a step of obtaining information indicating that cancer cells derived from any of the plural types of cancers exist in the biological sample when the analysis result indicating that a methylated CpG site is present is obtained.

(5) The method according to (1) or (2), wherein the analyzing step is a step of analyzing methylation frequency.

(6) The method according to (5), wherein the information on the plural types of cancers is information related to the presence or absence of cancer cells; and the information obtaining step is a step of obtaining information indicating that cancer cells derived from any of the plural types of cancers exist in the biological sample when the methylation frequency obtained in the analyzing step is higher than a predetermined threshold value or is the same as the threshold value.

(7) The method according to (1) to (6), wherein when analyzing the methylation status of the CpG site of GDF7 in the analyzing step, the plural types of cancers are two or more cancers selected from a group consisting of brain tumor, hepatocellular cancer, colon cancer, gastric cancer, uterine body cancer, lung cancer, breast cancer, prostate cancer, and renal cell cancer, and the information on the plural types of cancers is information indicating that cancer cells derived from any of the plural types of cancers exist in the biological sample; when analyzing the methylation status of the CpG site of ZNF132 in the analyzing step, the plural types of cancers are two or more cancers selected from a group consisting of hepatocellular cancer, colon cancer, gastric cancer, uterine body cancer, lung cancer, renal cell cancer, and breast cancer, and the information on the plural types of cancers is information indicating that cancer cells derived from any of the plural types of cancers exist in the biological sample; when analyzing the methylation status of the CpG site of CPXM1 in the analyzing step, the plural types of cancers are two or more cancers selected from a group consisting of hepatocellular cancer, colon cancer, gastric cancer, uterine body cancer, lung cancer, renal cell cancer, prostate cancer, and breast cancer, and the information on the plural types of cancers is information indicating that cancer cells derived from any of the plural types of cancers exist in the biological sample; when analyzing the methylation status of the CpG site of RPL39L in the analyzing step, the plural types of cancers are two or more cancers selected from a group consisting of brain tumor, breast cancer, lung cancer, gastric cancer, colon cancer, hepatocellular cancer, renal cell cancer, uterine body cancer, and prostate cancer, and the information on the plural types of cancers is information indicating that cancer cells derived from any of the plural types of cancers exist in the biological sample; when analyzing the methylation status of the CpG site of DOK1 in the analyzing step, the plural types of cancers are two or more cancers selected from a group consisting of hepatocellular cancer, breast cancer, and prostate cancer, and the information on the plural types of cancers is information indicating that cancer cells derived from any of the plural types of cancers exist in the biological sample; when analyzing the methylation status of the CpG site of FUZ in the analyzing step, the plural types of cancers are two or more cancers selected from a group consisting of uterine body cancer, hepatocellular cancer, colon cancer, and gastric cancer, and the information on the plural types of cancers is information indicating that cancer cells derived from any of the plural types of cancers exist in the biological sample; when analyzing the methylation status of the CpG site of MGAT3 in the analyzing step, the plural types of cancers are two or more cancers selected from a group consisting of breast cancer, lung cancer, colon cancer, and gastric cancer, and the information on the plural types of cancers is information indicating that cancer cells derived from any of the plural types of cancers exist in the biological sample; and when analyzing the methylation status of the CpG site of EHD3 in the analyzing step, the plural types of cancers are colon cancer and gastric cancer, and the information on the plural types of cancers is information indicating that cancer cells derived from any of colon cancer and gastric cancer exist in the biological sample.

(8) The present disclosure provides in vitro use of a marker for obtaining information on plural types of cancers by methylation analysis, which is at least one CpG site selected from CpG sites located in a promoter region of each gene of GDF7, ZNF132, CPXM1, RPL39L, DOK1, FUZ, MGAT3, and EHD3.

(9) The in vitro use of the marker according to (9), wherein the marker is a polynucleotide obtained by subjecting an isolated DNA to bisulfite treatment, the isolated DNA having a contiguous base sequence in an entire or partial promoter region of any one gene selected from a group consisting of GDF7, ZNF132, CPXM1, RPL39L, DOK1, FUZ, MGAT3 and EHD3 and containing at least one CpG site in the promoter region and at least one cytosine not included in CpG sites.

(10) The present disclosure provides a determination device for obtaining information on plural types of cancers comprising a computer including a processor and a memory, the memory being controlled by the processor wherein the memory is recorded with a computer program for causing the computer to execute the steps of: obtaining an analysis result on methylation status of a CpG site in at least one gene selected from a group consisting of GDF7, ZNF132, CPXM1, RPL39L, DOK1, FUZ, MGAT3, and EHD3 genes in a DNA sample derived from a subject; determining the presence or absence of any of the plural types of cancers in the subject based on the analysis result; and outputting a determination result.

(11) The determination device according to (10), wherein the analysis result is presence or absence of methylation of at least one CpG site.

(12) The determination device according to (11), wherein the information on the plural types of cancers is information related to the presence or absence of cancer cells; and the step of outputting information is the step of outputting information indicating that cancer cells derived from any of the plural types of cancers exist in the biological sample when the analysis result indicates that the presence of a methylated CpG site.

(13) The determination device according to (10) to (12), wherein the analysis result is methylation frequency.

(14) The determination device according to (10) to (13) further comprises a measurement device which measures a DNA sample from a biological sample collected from a subject, wherein the measurement device is connected to the computer.

(15) The determination device according to (14), wherein the measurement device is a mass spectrometer or a fluorescence image scanner.

The present invention can provide a method, in vitro use of a marker, and a determination device for obtaining information on plural types of cancers in a subject by analyzing methylation status.

BRIEF DESCRIPTION OF THE DRAWINGS

Fig. 1A is a graph illustrating a methylation positive rate in a promoter region of GDF7 gene in DNA extracted from various types of specimens;
Fig. 1B is a graph illustrating a methylation positive rate of a promoter region of ZNF132 gene in DNA extracted from various types of specimens;
Fig. 1C is a graph illustrating a methylation positive rate of a promoter region of CPXM1 gene in DNA extracted from various types of specimens;
Fig. 1D is a graph illustrating a methylation positive rate of a promoter region of RPL39L gene in DNA extracted from various types of specimens;
Fig. 1E is a graph illustrating a methylation positive rate of a promoter region of DOK1 gene in DNA extracted from various types of specimens;
Fig. 1F is a graph illustrating a methylation positive rate of a promoter region of FUZ gene in DNA extracted from various types of specimens;
Fig. 1G is a graph illustrating a methylation positive rate of a promoter region of MGAT3 gene in DNA extracted from various types of specimens;
Fig. 1H is a graph illustrating a methylation positive rate of a promoter region of EHD3 gene in DNA extracted from various types of specimens;
Fig. 2A is a graph illustrating a methylation score of a promoter region of GDF7 gene in DNA extracted from various types of tissues;
Fig. 2B is a graph illustrating a methylation score of a promoter region of ZNF132 gene in DNA extracted from various types of tissues;
Fig. 2C is a graph illustrating a methylation score of a promoter region of CPXM1 gene in DNA extracted from various types of tissues;
Fig. 2D is a graph illustrating a methylation score of a promoter region of RPL39L gene in DNA extracted from various types of tissues;
Fig. 2E is a graph illustrating a methylation score of a promoter region of DOK1 gene in DNA extracted from various types of tissues;
Fig. 2F is a graph illustrating a methylation score of a promoter region of FUZ gene in DNA extracted from various types of tissues;
Fig. 2G is a graph illustrating a methylation score of a promoter region of EHD3 gene in DNA extracted from various types of tissues;
Fig. 3A is an image describing the results of methylation-specific PCR (MSP) amplification of DNA extracted from various types of tissues using the primer set for FUZ;
Fig. 3B is an image describing the results of methylation-specific PCR (MSP) amplification of DNA extracted from various types of tissues using the primer set for EHD3;
Fig. 3C is an image describing the results of methylation-specific PCR (MSP) amplification of DNA extracted from various types of tissues using the primer set for MGAT3;
Fig. 4 is a schematic view illustrating one example of a determination device for providing information on plural types of cancers in a subject;
Fig. 5 is a block diagram illustrating the functionality configuration of the determination device of Fig. 4;
Fig. 6 is a block diagram illustrating the hardware configuration of the determination device illustrated in Fig. 4; and
Fig. 7 is a flowchart of determination for providing information on plurality of cancers in a subject using the determination device illustrated in Fig. 4.

DETAILED DESCRIPTION OF THE PREFERRED EMBODIMENTS

In the method for obtaining information on plural types of cancers of an embodiment (hereinafter also merely referred to as "method"), a DNA sample is first prepared from a biological sample collected from a subject.
Here, examples of "cancers" include brain tumor, hepatocellular cancer, colon cancer, gastric cancer, uterine body cancer, lung cancer, breast cancer, prostate cancer, and renal cell cancer. In the embodiment, at least two different types of cancers are selected from these cancers as the plural types of cancers.

In the embodiment, the biological sample is not particularly limited as long as it is a biological sample containing DNA of a subject, but is preferably a sample containing a genomic DNA such as a clinical specimen. Examples of the clinical specimen include body fluid, urine, and tissues obtained by operations or biopsies. Examples of the body fluid include blood, serum, plasma, lymph fluid, ascitic fluid, bone marrow fluid, and nipple discharge. The biological sample may also be a culture obtained by culturing cells or tissues collected from a subject.

The DNA sample can be prepared by extracting DNA from the biological sample. A method for extracting DNA from a biological sample is well-known in the art. DNA can be extracted by, for example, mixing the biological sample with a treatment solution containing a surfactant for solubilization of cells or tissues (such as sodium cholate and sodium dodecyl sulfate) and subjecting the resulting mixture to physical procedure (such as stirring, homogenization, and ultrasonication) to release DNA contained in the biological sample into the mixture. In this case, a supernatant containing DNA released by centrifuging the mixture to precipitate cell debris is preferably used in a later-described analyzing step. The obtained supernatant may be purified by any well-known method in the art. DNA can also be extracted from the biological sample and purified by using a commercially-available kit.

Preferably, the above-described preparing step further comprises a step of fragmenting the extracted DNA. By fragmenting the DNA to have appropriate length, methylated DNA immunoprecipitation (MeDIP) and non-methylated cytosine conversion as described below can be effectively performed.

Fragmentation of DNA may be performed by ultrasonication, alkaline treatment, restriction enzyme treatment, or the like. When DNA is fragmented by alkaline treatment, for example, a sodium hydroxide solution is added to a DNA solution to obtain a final concentration of 0.1 to 1.0N and the mixture is incubated at 10 to 40°C for 5 to 15 minutes to fragment the DNA. When DNA is fragmented by the restriction enzyme treatment, the restriction enzyme is appropriately selected based on the base sequence of DNA, which may be MseI or BamHI, for example.

In the method of the embodiment, the methylation status of a CpG site in a promoter region of GDF7 gene in the DNA sample obtained in the preparing step is analyzed.

The term "CpG site" used herein means a site of a sequence in which cytosine (C) and guanine (G) are adjacent in this order from 5' to 3' in the base sequence. The letter "p" in "CpG" represents a phosphodiester bond between cytosine and guanine.

As used herein, "analyzing the methylation status" means analyzing the presence or absence of methylation of a CpG site located in a promoter region of at least one gene selected from a group consisting of GDF7, ZNF132, CPXM1, RPL39L, DOK1, FUZ, MGAT3 and EHD3 genes or analyzing methylation frequency in the promoter region.

GDF7 (Growth Differentiating Factor 7) is known in the art as a gene that encodes one of the cytokine, which is a member of a β type transforming growth factor (TGF-β) family. In the present embodiment, if the GDF7 is selected in the analyzing step, the information on plural types of cancers obtained by analyzing the methylation status of the CpG site in the GDF7 is preferably related to at least two plural types of cancers selected from brain tumor, hepatocellular cancer, colon cancer, gastric cancer, uterine body cancer, lung cancer, breast cancer, prostate cancer, and renal cell cancer.

ZNF132 (Zinc Finger Protein 132) is known in the art as a gene that encodes one of the zinc finger proteins. In the present embodiment of the disclosure, if the ZNF132 is selected in the analyzing step, the information on plural types of cancers obtained by analyzing the methylation status of the CpG site in the ZNF132 is preferably related to at least two plural types of cancers selected from hepatocellular cancer, colon cancer, gastric cancer, uterine body cancer, lung cancer, renal cell cancer, and breast cancer.

CPXM1 (Carboxy Peptidase X Member 1) is known in the art as a gene that encodes a protein assumed to be associated with intercellular interaction. In the present embodiment of the disclosure, if the CPXM1 is selected in the analyzing step, the information on plural types of cancers obtained by analyzing the methylation status of the CpG site in the CPXM1 is preferably related to at least two plural types of cancers selected from hepatocellular cancer, colon cancer, gastric cancer, uterine body cancer, lung cancer, renal cell cancer, prostate cancer, and breast cancer.

RPL39L (Ribosomal Protein L-39 Like) is known in the art as a gene that encodes a protein, which has an amino acid sequence similar to the ribosome protein L-39 but which function is unknown. In the present embodiment of the disclosure, if the RPL39L is selected in the analyzing step, the information on plural types of cancers obtained by analyzing the methylation status of the CpG site in the RPL39L is preferably related to at least two plural types of cancers selected from brain tumor, breast cancer, lung cancer, gastric cancer, colon cancer, renal cell cancer, uterine body cancer, and prostate cancer.

DOK1 (DOcKing protein 1) is known in the art as a gene that encodes an adapter molecule associated with the induction of Th2 cytokine by coupling to protein kinase C-θ. In the present embodiment of the disclosure, if the DOK1 is selected in the analyzing step, the information on plural types of cancers obtained by analyzing the methylation status of the CpG site in the DOK1 is preferably related to at least two plural types of cancers selected from hepatocellular cancer, breast cancer, and prostate cancer.

FUZ (FUZzy homolog) is known in the art as a gene that encodes a protein, which function is unknown, in a human. In the present embodiment of the disclosure, if the FUZ is selected in the analyzing step, the information on plural types of cancers obtained by analyzing the methylation status of the CpG site in the FUZ is preferably related to at least two plural types of cancers selected from uterine body cancer, hepatocellular cancer, colon cancer, and gastric cancer.

MGAT3 (Mannosyl (beta-1,4-) - Glycoprotein beta-1,4 - N - AcetylglucosaminylTransferase) is known in the art as a gene that encodes a protein associated with the removal of an amyloidβ that forms a characteristic plaque in Alzheimer disease. In the present embodiment of the disclosure, if the MGAT3 is selected in the analyzing step, the information on plural types of cancers obtained by analyzing the methylation status of the CpG site in the MGAT3 is preferably related to at least two plural types of cancers selected from breast cancer, lung cancer, colon cancer, and gastric cancer.

EHD3 (EH-domain containing 3) is known in the art as a gene that encodes a protein associated with a cell cytoskeleton formation by an epidermal growth factor stimulation. In the present embodiment of the disclosure, if the EHD3 is selected in the analyzing step, the information on plural types of cancers obtained by analyzing the methylation status of the CpG site in the EHD3 is preferably related to colon cancer and gastric cancer.

The base sequence itself in the promotor region of each gene of GDF7, ZNF132, CPXM1, RPL39L, DOK1, FUZ, MGAT3, and EHD3 is well-known in the art. Such a base sequence can be obtained from a well-known database provided by, for example, the National Center for Biotechnology Information (NCBI) (http://www.ncbi.nlm.nih.gov/). The ID numbers of the genes described above are shown in Table 1. The base sequence of the promotor region of each gene is shown in Table 1 (base sequences having SEQ. ID NOs: 1 to 3 and 5 to 8 are sequences of positive strand, and base sequences having SEQ. ID NO: 4 is sequence of negative strand).

[Table 1]

| Gene symbol | Unigene ID | Entrez Gene ID | Transcript ID | SEQ ID NO: |
|---|---|---|---|---|
| GDF7 | Hs.447688 | 151449 | NM_182828 | 1 |
| ZNF132 | Hs.156169 | 7691 | NM_003433 | 2 |
| CPXM1 | Hs.659346 | 56265 | NM_019609 | 3 |
| | | | NM_001184699 | |
| RPL39L | Hs.647900 | 116832 | NM_052969 | 4 |
| DOK1 | Hs.103854 | 1796 | NM_001381 | 5 |
| | | | NM_001197260 | |
| FUZ | Hs.288800 | 80199 | NM_025129 | 6 |
| | | | NM_001171937 | |
| MGAT3 | Hs.276808 | 4248 | NM_002409 | 7 |
| | | | NM_001098270 | |
| EHD3 | Hs.368808 | 30845 | NM_014600 | 8 |

In the embodiment of the present disclosure, the analyzing step may be a step of analyzing the presence or absence of methylation of at least one CpG site among CpG sites located in a promoter region of at least one gene selected from a group consisting of GDF7, ZNF132, CPXM1, RPL39L, DOK1, FUZ, MGAT3 and EHD3 genes. The term "presence or absence of methylation" means whether or not cytosine in a CpG site located in the promoter region is methylated. In the embodiment, only one CpG site may be analyzed, but a plurality of CpG sites is preferably analyzed for the presence or absence of methylation. The CpG sites may be selected in a promoter region of one gene or in each of promoter regions of a plurality of genes.

In another embodiment of the present disclosure, the analyzing step may be a step of analyzing methylation frequency in a promoter region of at least one gene selected from a group consisting of GDF7, ZNF132, CPXM1, RPL39L, DOK1, FUZ, MGAT3 and EHD3 genes. The term "methylation frequency" means a ratio of the number of methylated CpG sites relative to the number of CpG sites located in the promoter region. In this embodiment, a target for analysis may be the entire promoter region or a part of the promoter region including at least one CpG site. The target for analysis may contain only one CpG site, but the target for analysis preferably contains a plurality of CpG sites. The target for analysis may be selected in a promoter region of any one of the above genes or in promoter regions of the genes. The positions and number of CpG sites located in the promoter regions of GDF7, ZNF132, CPXM1, RPL39L, DOK1, FUZ, MGAT3 and EHD3 genes are already known, and thus, in the embodiment, the number of methylated CpG sites itself in the promoter regions can be used as the methylation frequency.

The methylation frequency may be a "methylation score" obtained by analyzing methylation status of a CpG site in DNA with mass spectrometry such as MassARRAY® as described below. MassARRAY® allows calculation of a methylation score based on a ratio between the area of a peak derived from methylated DNA fragment and the area of a peak derived from non-methylated DNA fragment obtained through measurement of DNA fragments.

In the embodiment of the present disclosure, the target for analysis may is not particularly limited, and may be any CpG sites or certain regions including the CpG sites in the promoter regions of GDF7, ZNF132, CPXM1, RPL39L, DOK1, FUZ, MGAT3 and EHD3 genes. The positions and number of CpG sites located in the promoter regions of these genes are already known. Thus, the target CpG sites or regions may be selected by a person skilled in the art based on the results of routine experiments according to the well-known analysis method described below.

Various methods for analyzing methylation status are well-known in the art. The analysis method to be used in the embodiment is not particularly limited, but preferably includes a step of differentiating methylated DNA from non-methylated DNA, a step of amplifying DNA, and a step of detecting methylated DNA and/or non-methylated DNA.

The step of differentiating methylated DNA from non-methylated DNA may include a step of performing methylation sensitive restriction enzyme treatment, a MeDIP method, non-methylated cytosine converting treatment, or the like.

The step of amplifying DNA may include a step of performing PCR, quantitative PCR, IVT (in vitro transcription) amplification, SPIA™ amplification methods, or the like.

The step of detecting methylated DNA and/or non-methylated DNA may include a step of performing electrophoresis, sequence analysis, microarray analysis, mass spectrometry, Southern hybridization, or the like.

The MeDIP method is used to enrich for methylated DNA in a biological sample by immunoprecipitation using an anti-methylated cytosine antibody or an anti-methylated cytidine antibody, or an antibody which specifically recognizes a methylated DNA-binding protein. In the embodiment, the analyzing step may be a step of enriching for methylated DNA in DNA obtained in the extracting step by the MeDIP method and analyzing methylation status of the obtained methylated DNA. The methylated DNA enriched by the MeDIP method may be amplified by, for example, IVT amplification, and the methylation status of the obtained amplified product may be analyzed by using a microarray. This analysis method is referred to as "MeDIP on chip."

The non-methylated cytosine converting treatment is used to react DNA extracted from a biological sample with a non-methylated cytosine conversion agent so as to convert non-methylated cytosine in the DNA to a different base (uracil, thymine, adenine or guanine). The non-methylated cytosine conversion agent is a substance that can react with DNA and convert non-methylated cytosine in the DNA to a different base (uracil, thymine, adenine or guanine). The non-methylated cytosine conversion agent may be, for example, bisulfite such as sodium, potassium, calcium or magnesium bisulfite. In the treatment using bisulfite, non-methylated cytosine in DNA is converted to uracil due to deamination reaction, while methylated cytosine does not undergo such a base conversion. Thus, the difference in methylation status of a CpG site in DNA is converted to the difference in a base sequence (C and U) by the non-methylated cytosine converting treatment using bisulfite. The non-methylated cytosine converting treatment using bisulfite is referred to as "bisulfite treatment."

When the bisulfite treatment is performed, the additive amount (concentration) of bisulfite is not specifically limited as long as it can sufficiently convert non-methylated cytosine in DNA. For example, the final concentration in a solution containing DNA is 1M or higher, preferably 1M to 15M, and more preferably 3M to 10M. The incubation condition (temperature and time) after addition of bisulfite may be appropriately selected depending on the additive amount of bisulfite. For example, when bisulfite is added at a final concentration of 6M, the incubation is carried out at 50 to 80°C for 10 to 90 minutes.

Methylation status of CpG sites in DNA can be analyzed by analyzing the sequence of DNA after bisulfite treatment and detecting the difference in base sequence from the original sequence. This method is referred to as "bisulfite sequencing."

The methylation status of CpG sites can be alternatively analyzed by mass spectrometry. Specifically, DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is cleaved with RNase A, and the difference in mass (16 Da) due to difference between G and A in the obtained digested fragments is detected using a MALDI-TOF (matrix assisted laser desorption/ionization time-of-flight) mass spectrometer to analyze methylation status of the DNA. This method is referred to as "MassARRAY® analysis."

It is known that the site of IVT product cleaved with RNase A is between an arbitrary base sequence and the adjacent uracil (U) or thymine (T). Thus, the base sequence and mass of the IVT product cleaved with RNase A can be predicted based on the base sequence of the template DNA. Accordingly, it is possible to identify a portion of the base sequence of the template DNA from which each peak obtained in MassARRAY® is originated. For example, when one CpG site is methylated in a DNA fragment, a peak obtained in MassARRAY® shifts to the side with an increased mass for 16 Da. In analysis of a DNA fragment containing plural CpG sites, for example, a shift of 32 Da is shown when two CpG sites are methylated, and a shift of 48 Da is shown when three methylated CpG sites are methylated.

In mass spectrometry such as MassARRAY®, the methylation score of the analyzed DNA fragment can be calculated. For example, when the ratio between the area of the peak of the non-methylated DNA fragment and the area of the peak of the methylated DNA fragment in a chart obtained from the analysis of a DNA fragment having a certain sequence is 1 : 3, the methylation score of the DNA fragment is 0.75 (= 3/(1 + 3)). The methylation score is theoretically 1 when all CpG sites are methylated, and 0 when not all CpG sites are methylated.

The methylation status of CpG sites can be analyzed by a methylation-specific PCR (MSP) method. The MSP method is a method of analyzing the methylation status of CpG sites (the presence or absence of methylation) by amplifying DNA after bisulfite treatment by PCR using a primer set described below and determining the presence or absence of a PCR product.

The MSP method utilizes a primer set (M primer) that can amplify a base sequence where a CpG site to be analyzed is methylated (i.e. cytosine is not converted to uracil), but cannot amplify a base sequence where a CpG site is not methylated (i.e. cytosine is converted to uracil). According to the MSP method using such a primer set, the presence of a PCR product indicates the methylation of the CpG site to be analyzed. The specific M primer set includes the primer set of SEQ. ID NOs: 23 and 24, the primer set of SEQ. ID NOs: 27 and 28, and the primer set of SEQ. ID NOs: 31 and 32.

The MSP method may also utilize a primer set (U primer) that cannot amplify a base sequence where cytosine in a CpG site to be analyzed is not converted to uracil, but can amplify a base sequence where cytosine in a CpG site is converted to uracil. In this case, the absence of a PCR product indicates the methylation of the CpG site to be analyzed. The specific U primer set includes the primer set of SEQ. ID NOs: 25 and 26, the primer set of SEQ. ID NOs: 29 and 30, and the primer set of SEQ. ID NOs: 33 and 34.

Each primer in the primer set used for the MSP method may be appropriately designed by a person skilled in the art based on the base sequence including a CpG site to be analyzed, and it is preferably designed so as to contain cytosine of the CpG site to be analyzed at the 3' end of the primer or in the vicinity thereof.

The methylation status of CpG sites may alternatively be analyzed with a microarray. In this case, the microarray for analysis may be prepared by immobilizing a nucleic probe complementary to the base sequence of a promoter region of each of GDF7, ZNF132, CPXM1, RPL39L, DOK1, FUZ, MGAT3 and EHD3 genes on a substrate. The microarray can be prepared according to a well-known method in the art.

In the analysis using a microarray, DNA extracted from a biological sample is preferably labeled with a labeling substance well-known in the art. Thus, the determination method of the embodiment preferably further includes a step of labeling the extracted DNA. The labeling step is advantageously carried out after the DNA amplifying step because all DNA in the biological sample can be labeled. Examples of the labeling substance include fluorescent substances, haptens such as biotin, and radioactive substances. Examples of the fluorescent substances include Cy3, Cy5, FITC, and Alexa Fluor™. Labeling of DNA facilitates measurement of a signal from a probe on the microarray. The method for labeling DNA with the labeling substance is well-known in the art.

The above signal may be any suitable signal depending on the type of microarrays. For example, the signal may be an electric signal generated when a DNA fragment hybridizes to a probe on the microarray, or a fluorescence or luminescence signal generated from a labeling substance when DNA to be analyzed is labeled as described above. The signal can be detected using a scanner included in a normal microarray analyzer. Examples of the scanner include GeneChip® Scanner3000 7G (Affymetrix, Inc.), and Illumina® BeadArray Reader (Illumina, Inc.).

In the method of the embodiment, the information on plural types of cancers in a subject is obtained based on the analysis result obtained in the analyzing step. The information on cancer described herein is not particularly limited as long as it may be an index on diagnosis of cancer. The information is preferably information indicative of occurrence or state of cancer or both of them in a subject. For example, information indicative of high or low possibility of any one of plural types of cancers may be provided. In this case, the one of the plural types of cancers is not specified. In other words, the primary lesion is not specified, but the information on the possibility of being affected to cancer can be provided. Such information is useful in screening the cancer. If the blood is used as a sample and the methylation is detected, the cells derived from the primary lesion are assumed to be circulating in the blood. Such information can be also used to monitor relapse for the cancer patient whose type of cancer is already known. The determination made by a doctor or the like on the presence or absence and the extent of cancer is assisted by obtaining the information and providing the information to the doctor or the like.

In the embodiment, when the analysis result in the analyzing step indicates the presence of methylated CpG sites, information indicating the occurrence of any one of plural types of cancers or indicating that the status of any one of plural types of cancers is poor (or aggravated) can be obtained. Alternatively, such information can be obtained when the methylation frequency obtained in the analyzing step is higher than or equal to a certain threshold. For example, the information indicative of high possibility of occurrence of any one of the plural types of cancers can be obtained.

When the result in the analyzing step indicates the absence of methylated CpG sites, information suggesting no occurrence of any one of plural types of cancers or information indicating that any one of plural types of cancers is in a preferable status can be obtained. Alternatively, such information can be obtained when the methylation frequency obtained in the analyzing step is lower than a certain threshold. For example, information indicative of low possibility of occurrence of any one of the plural types of cancers can be obtained.

The threshold is not particularly limited and may be empirically set based on accumulated data on various biological samples. The threshold may be set as follows. First, methylation frequency is analyzed for DNA extracted from a biological sample which is confirmed to be devoid of cancer cells derived from any one of plural types of cancers (normal tissues or normal cells), and a biological sample containing a cancer cell derived from any one of plural types of cancers. Next, based on the obtained analysis results, a threshold is set within a range that is higher than the methylation frequency of the biological sample devoid of cancer cells derived from any one of plural types of cancers and lower than the methylation frequency of the biological sample containing the cancer cell derived from any one of plural types of cancers. Preferably, the threshold is set as a value that can highly accurately differentiate between the biological sample devoid of cancer cells derived from any one of plural types of cancers and the biological sample containing the cancer cell derived from any one of plural types of cancers.

The scope of the present disclosure encompasses a kit for obtaining information on plural types of cancers (also simply referred to as "kit"). The kit of the embodiment of the present disclosure includes a primer set for analysis of methylation status of at least one CpG site selected from CpG sites located in a promoter region of each gene of GDF7, ZNF132, CPXM1, RPL39L, DOK1, FUZ, MGAT3 and EHD3.

In the embodiment of the present disclosure, the primer set included in the kit may be any primer set for analysis of methylation status of CpG sites according to mass spectrometry such as MassARRAY® or an analysis method involving PCR amplification such as the MSP method and the bisulfite sequencing method, but is preferably a primer set used for mass spectrometry such as Mass ARRAY® or for the MSP. The base sequence of each primer in the primer set may be appropriately selected by a person skilled in the art based on the base sequence in the promoter region. Examples of the primer set include a primer set of primers respectively having base sequences SEQ ID NOs: 9 and 10, a primer set of primers respectively having base sequences SEQ ID NOs: 11 and 12, a primer set of primers respectively having base sequences SEQ ID NOs: 13 and 14, a primer set of primers respectively having base sequences SEQ ID NOs: 15 and 16, a primer set of primers respectively having base sequences SEQ ID NOs: 17 and 18, a primer set of primers respectively having base sequences SEQ ID NOs: 19 and 20, a primer set of primers respectively having base sequences SEQ ID NOs: 21 and 22, a primer set of primers respectively having base sequences SEQ ID NOs: 23 and 24, a primer set of primers respectively having base sequences SEQ ID NOs: 27 and 28, and a primer set of primers respectively having base sequences SEQ ID NOs: 31 and 32.

The scope of the present invention also encompasses a marker for obtaining information on plural types of cancers by methylation analysis (also simply referred to as "marker").

The marker of the embodiment is at least one CpG site located in a promoter region of GDF7.

In the embodiment, the methylation status of the marker in a DNA sample prepared from a biological sample collected from a subject may be analyzed, and information on plural types of cancers in the subject can be obtained based on the analysis result. The analysis of methylation status and the obtainment of information on plural types of cancers are the same as previously described.

In another embodiment, the marker includes a contiguous base sequence in the entire or partial promoter region of the gene. In the present disclosure, the promoter region is a promotor region of any one gene selected from a group consisting of GDF7, ZNF132, CPXM1, RPL39L, DOK1, FUZ, MGAT3 and EHD3. The marker is a polynucleotide obtained by bisulfite treatment on an isolated DNA including at least one CpG site in the promoter region and at least one cytosine not included in CpG sites (hereinafter simply referred to as "polynucleotide"). The cytosine not included in CpG sites described herein may be any cytosine other than those in CpG sites, and may include, for example, cytosine in a base sequence (i.e., CA, CT, or CC) in which cytosine (C), and adenine (A), thymine (T) or the cytosine (C) are adjacent in this order in the direction from 5' to 3'.

In the polynucleotide of the embodiment, a non-methylated cytosine in the isolated DNA is converted to uracil by bisulfate treatment, while a methylated cytosine is not converted. In the embodiment, the methylation status of a CpG site can be analyzed by analyzing the sequence of the CpG site in the polynucleotide. Based on the analysis result, the information on plural types of cancers can be obtained. The isolated DNA can be obtained in the same manner as that described for preparation of the DNA sample. The bisulfite treatment, the analysis of the methylation status, and the obtainment of information on plural types of cancers are also the same as previously described.

The size of the polynucleotide of the embodiment is not particularly limited as long as it allows analysis of methylation status by the MSP method, sequencing or mass spectrometry, but is preferably 50 to 200 bases and more preferably 70 to 200 bases. Examples of the polynucleotide of the embodiment of the present disclosure include a polynucleotide having any of base sequences SEQ ID NOs: 47 to 54.

The polynucleotide of SEQ ID NO: 47 is obtained by bisulfite treatment on a part of the promoter region of GDF7 gene of a non-cancerous subject. The polynucleotide having such sequence can be used for analysis of the methylation status by mass spectrometry.

The polynucleotide of SEQ ID NO: 48 is obtained by bisulfite treatment on a part of the promoter region of ZNF132 gene of a non-cancerous subject. The polynucleotide having such sequence can be used for analysis of the methylation status by mass spectrometry.

The polynucleotide of SEQ ID NO: 49 is obtained by bisulfite treatment on a part of the promoter region of CPXM1 gene of a non-cancerous subject. The polynucleotide having such sequence can be used for analysis of the methylation status by mass spectrometry.

The polynucleotide of SEQ ID NO: 50 is obtained by bisulfite treatment on a part of the promoter region of RPL39L gene of a non-cancerous subject. The polynucleotide having such sequence can be used for analysis of the methylation status by mass spectrometry.

The polynucleotide of SEQ ID NO: 51 is obtained by bisulfite treatment on a part of the promoter region of DOK1 gene of a non-cancerous subject. The polynucleotide having such sequence can be used for analysis of the methylation status by mass spectrometry.

The polynucleotide of SEQ ID NO: 52 is obtained by bisulfite treatment on a part of the promoter region of FUZ gene of a non-cancerous subject. The polynucleotide having such sequence can be used for analysis of the methylation status by the MSP method.

The polynucleotide of SEQ ID NO: 53 is obtained by bisulfite treatment on a part of the promoter region of EHD3 gene of a non-cancerous subject. The polynucleotide having such sequence can be used for analysis of the methylation status by the MSP method

The polynucleotide of SEQ ID NO: 54 is obtained by bisulfite treatment on a part of the promoter region of MGAT3 gene of a non-cancerous subject. The polynucleotide having such sequence can be used for analysis of the methylation status by the MSP method.

The polynucleotide of SEQ ID NO: 55 is obtained by bisulfite treatment on a part of the promoter region of FUZ gene of a non-cancerous subject. The polynucleotide having such sequence can be used for analysis of the methylation status by mass spectrometry.

The polynucleotide of SEQ ID NO: 56 is obtained by bisulfite treatment on a part of the promoter region of EHD3 gene of a non-cancerous subject. The polynucleotide having such sequence can be used for analysis of the methylation status by mass spectrometry.

The present invention also includes a system suitable for providing information on plural types of cancers in a subject.

The system of the embodiment includes a computer with a processor and a memory controlled by the processor.

The memory of the system is recorded with a computer program for causing the computer to execute the steps of claim 1 and the step of outputting the determination result.

The present invention also includes a computer program product for causing a computer to provide information on plural types of cancers in a subject. The computer program product includes a program that can be downloaded through the Internet or the like, and a medium recorded with such program.

For example, an exemplary program of the present disclosure causes the computer to execute the steps of:

obtaining an analysis result on methylation status of a CpG site in at least one gene selected from a group consisting of GDF7, ZNF132, CPXM1, RPL39L, DOK1, FUZ, MGAT3, and EHD3 genes in a DNA sample derived from a subject;
determining the presence or absence of cancer in the subject based on the analysis result; and
outputting the determination result.

Hereinafter, an embodiment of a suitable device for carrying out the method of the embodiment will be described with reference to the drawings. However, the present invention is not limited to this embodiment. Fig. 4 is a schematic view of an example of a determination device for providing information on plural types of cancers in a subject. A determination device 1 illustrated in Fig. 4 includes a measurement device 2 and a computer system 3 connected to the measurement device 2.

In the embodiment, the measurement device 2 is a MALDI-TOF mass spectrometer. The measurement device 2 obtains mass spectrometric information such as the time of flight or the mass-to-charge ratio (m/z value) of a substance to be analyzed. The measurement device 2, onto which a measurement sample prepared from a DNA sample derived from a subject is mounted, obtains mass spectrometric information of a nucleic acid in the measurement sample and sends the mass spectrometric information to the computer system 3.

The measurement device 2 may be, when methylation status is analyzed by the MSP method, a gel imaging device such as a fluorescence image scanner. In this case, the measurement device 2, onto which a gel obtained by electrophoresis of a reaction solution after nucleic acid amplification by the MSP method is mounted, detects amplification products. The measurement device 2 then obtains the band intensity data of the amplification products and sends the obtained data to the computer system 3.

The computer system 3 includes a computer main body 3a, an input device 3b, and a display unit 3c for displaying sample information, determination results and the like. The computer system 3 receives the mass spectrometric information from the measurement device 2. The processor in the computer system 3 executes, based on the mass spectrometric information, a program for providing information on plural types of cancers in a subject.

Fig. 5 is a block diagram illustrating the functionality configuration of the determination device of Fig. 4. As illustrated in Fig. 5, the computer system 3 includes an acquisition unit 301, a storage unit 302, a calculation unit 303, a determination unit 304, and an output unit 305. The acquisition unit 301 is communicably connected to the measurement device 2 through a network. The calculation unit 303 and the determination unit 304 are included in a control unit 306.

The acquisition unit 301 obtains information from the measurement device 2. The storage unit 302 stores a threshold necessary for determination and a formula for calculating a methylation score. The calculation unit 303 calculates the methylation score from the information obtained at the acquisition unit 301 according to the formula stored in the storage unit 302. The determination unit 304 determines whether or not the methylation score calculated at the calculation unit 303 is lower than the threshold stored at the storage unit 302. The output unit 305 outputs the determination result from the determination unit 304 as information on plural types of cancers in the subject (e.g., the presence or absence of cancer cells derived from any of the plural types of cancers in the biological sample collected from the subject).

Fig. 6 is a block diagram illustrating the hardware configuration of the determination device in Fig. 4. As illustrated in Fig. 6, the computer main body 3a includes a central processing unit (CPU) 30, a read only memory (ROM) 31, a random access memory (RAM) 32, a hard disk 33, an input/output interface 34, a readout device 35, a communication interface 36, and an image output interface 37. The CPU 30, ROM 31, RAM 32, the hard disk 33, the input/output interface 34, the readout device 35, the communication interface 36, and the image output interface 37 are data-communicably connected via a bus 38.

The CPU 30 can execute a computer program stored in the ROM 31 and a computer program loaded with the RAM 32. When the CPU 30 executes the application program, the functional blocks described above may be executed. Accordingly, the computer system serves as a terminal that is a determination device for providing information on plural types of cancers in a subject.

The ROM 31 may be mask ROM, PROM, EPROM, EEPROM, or the like. The ROM 31 stores the computer program executed by the CPU 30 and data used for the execution.

The RAM 32 may be SRAM, DRAM, or the like. The RAM 32 is used to read out the computer program stored in the ROM 31 and the hard disk 33. The RAM 32 is also used as a work area of the CPU 30 in executing these computer programs.

The computer programs, such as an operating system and an application program (a computer program for providing information on plural types of cancers in a subject), to be executed by the CPU 30, and data for executing the computer programs are installed on the hard disk 33.

The readout device 35 may be a flexible disk drive, a CD-ROM drive, a DVD-ROM drive, or the like. The readout device 35 can read out the computer program or data recorded in a portable recording medium 40.

The input/output interface 34 may be a serial interface such as USB, IEEE 1394, and RS-232C, a parallel interface such as SCSI, IDE, and IEEE 1284, an analog interface formed by a D/A converter and an A/D converter, and the like. An input device 3b such as a keyboard, a mouse, and the like is connected to the input/output interface 34. The operator uses the input device 3b to input data to the computer main body 3a.

The communication interface 36 is, for example, an Ethernet® interface. The computer system 3 can send printing data to a printer by the communication interface 36.

The image output interface 37 is connected to the display unit 3c including a LCD, a CRT and the like. Accordingly, the display unit 3c can output an image signal according to image data from the CPU 30. The display unit 3c displays an image (on a screen) according to the input image signal.

Subsequently, the processing procedure performed by the determination device 1 for providing information on plural types of cancers in a subject will be described. Fig. 7 is a flow chart for providing information on plural types of cancers using the determination device of Fig. 4. In an illustrated example, a peak area is calculated based on mass spectrometric information of a nucleic acid in a measurement sample prepared from a DNA sample derived from a subject, and a methylation score is calculated from the obtained peak area, so as to determine whether or not the methylation score is lower than a threshold. However, the present invention is not limited to this embodiment.

In the step S1-1, the acquisition unit 301 in the determination device 1 obtains mass spectrometric information from the measurement device 2. In the step S1-2, the calculation unit 303 calculates a peak area from the mass spectrometric information obtained at the acquisition unit 301 and sends the peak area to the storage unit 302. In the step S1-3, the calculation unit 303 calculates a methylation score based on the peak area stored in the storage unit 302 according to the formula stored in the storage unit 302.

In the step S1-4, the determination unit 304 determines whether or not the methylation score calculated at the calculation unit 303 is lower than the threshold stored in the storage unit 302. When the methylation score is lower than the threshold, the process proceeds to the step S1-5 and the determination unit 304 sends, to the output unit 305, a determination result indicating that the biological sample collected from the subject does not contain cancer cells. When the methylation score is not lower than the threshold (i.e., the methylation score is the threshold or more), the determination unit 304 sends, to the output unit 305, a determination result indicating that the biological sample collected from the subject contains cancer cells.

In the step S1-7, the output unit 305 outputs the determination result as information on plural types of cancers in the subject, so that the display unit 3c displays the result and/or the printer prints out the result. Accordingly, the determination device can provide, to a physician or the like, information assisting the physician or the like to judge whether or not the subject has cancer.

Hereinafter, the present invention will be described in detail with reference to Examples, but the present invention is not limited thereto.

## Examples

### Example 1: Identification of Novel Marker

#### (1) Biological Sample

In the present example, the specimens shown in Tables 2 to 4 below were used as biological samples. Among such specimens, the healthy blood cells were purchased by BioChain Inc. Other specimens were collected from patients or healthy subjects. After being collected, the tissues were immediately frozen with liquid nitrogen and stored at -80°C until use.

[Table 2]

| Cancer/tumor tissue specimen | |
|---|---|
| Tissue | The number of specimens |
| Brain tumor (Brain) | 283 |
| Colon Cancer (Colon) | 236 |
| Breast cancer (Breast) | 186 |
| Lung cancer (Lung) | 59 |
| Gastric cancer (Gastric) | 82 |
| Hepatocellular cancer (Liver) | 100 |
| Renal cell cancer (Kidney) | 219 |
| Uterus | 70 |
| Prostate cancer (Prostase) | 93 |

[Table 3]

| Non-cancerous tissue | |
|---|---|
| Tissue | The number of specimens |
| Colonic mucosa (Colon) | 16 |
| Lung | 59 |
| Liver | 39 |
| Kidney | 199 |
| Prostate | 87 |

[Table 4]

| Normal specimen | |
|---|---|
| Tissue | The number of specimens |
| Normal brain (Brain) | 2 |
| Normal oral mucosa (Oral) | 2 |
| Normal lung (Lung) | 2 |
| Normal colonic mucosa (Colon) | 2 |
| Normal liver (Liver) | 2 |
| Normal skeletal muscle (Skeletal) | 2 |
| Normal testis (Testis) | 1 |
| Normal stomach (Stomach) | 2 |
| Normal pancreas (Pancreas) | 1 |
| Normal spleen (Spleen) | 1 |
| Normal kidney (Kidney) | 2 |
| Normal uterus (Uterus) | 1 |
| Healthy blood cell (Blood) | 2 |
| Healthy peripheral blood (Blood) | 93 |

Among the specimens, the methylation data of cancer tissue specimens derived from gastric cancer, cancer tissue specimens derived from hepatocellular cancer, non-cancerous tissue specimens derived from hepatocellular cancer, and normal specimens (excluding normal uterus and healthy peripheral blood) were measured by Infinium Methylation Assay using Infinium HumanMethylation27 BeadChip (Illumina, Inc.) in a manner described below in (2) to (3).

The methylation data of lung cancer, prostate cancer, and peripheral blood from healthy subjects were obtained using Infinium HumanMethylation27 BeadChip (Illumina, Inc.) published in the following documents.

- Lung cancer: Selamat SA et al., Genome-scale analysis of DNA methylation in lung adenocarcinoma and integration with mRNA expression. Genome Res. Jul;22(7):1197-211(2012)
- Prostate cancer: Kobayashi Y et al., DNA methylation profiling reveals novel biomarkers and important roles for DNA methyl transferases in prostate cancer. Genome Res. Jul 21(7):1017-27(2011)
- Data of healthy peripheral blood for cases of 93 healthy subjects: Salhia B et al., DNA methylation analysis determines the high frequency of genic hypomethylation and low frequency of hypermethylation events in plasma cell tumors. Cancer Res. Sep 1;70(17):6934-44(2010)

The data of other specimens were obtained from TCGA (The Cancer Genome Atlas : http://tcga-data.nci.nih.gov/tcga/tcgaHome2.jsp).

The methylation data described herein is the methylation rate (mCpG) of CpG sites of GDF7, ZNF132, CPXM1, RPL39L, DOK1, FUZ, MGAT3, and EHD3 obtained by Infinium Methylation Assay using Infinium HumanMethylation27 BeadChip (Illumina, Inc.) in a manner described below in (2) to (3).

(2) DNA Extraction and Bisulfite Treatment

Genomic DNA was extracted from each of cancerous tissue specimens derived from gastric cancer, cancerous tissue specimens derived from hepatocellular cancer, non-cancerous tissue specimens derived from hepatocellular cancer, and normal specimens (excluding normal uterus and healthy peripheral blood) using QIAamp DNA Mini Kit (QIAGEN). The obtained genomic DNA (500 ng) was subjected to bisulfite treatment using EZ DNA Methylation Kit (Zymo Research), and the genomic DNA after the treatment was dissolved in sterilized distilled water (10μl). The genomic DNA contained in an obtained DNA solution (4μl) was fragmented by Bioruptor (COSMO BIO Co., Ltd).

(3) Identification of Novel Marker

Methylation data for each specimen was obtained by Infinium Methylation Assay using Infinium HumanMethylation27 BeadChip (Illumina, Inc.) on the fragmented genomic DNA. A novel marker which was methylated specifically to cancerous tissue of various types of cancers was detected using the obtained methylation data, and the methylation data published in the documents described above and the data of the TCGA. The specific operation was carried out according to the manual attached to the used chip.

The Infinium HumanMethylation27 BeadChip includes probes for methylated CpG sites and probes for non-methylated CpG sites of 27,578 CpG sites on human genome. In this example, the signal intensity (signal M) from the probes for methylated CpG sites and the signal intensity (signal U) from the probes for non-methylated CpG sites were detected on Bead Array Reader, and the methylation rate (mCpG) of CpG sites in the respective genes was calculated according to the following calculation formula:

$$(\mathrm{mCpG}) = (\mathrm{signal\ M})/\{(\mathrm{signal\ M}) + (\mathrm{signal\ U})\}$$

The threshold was set to "0.2" for the obtained methylation rate (mCpG) of each gene. A specimen with the methylation rate of the threshold value or higher was referred to as "methylation positive specimen." Based on the number of methylation positive specimens, the methylation positive rate (%) of the genes in various tissues was calculated by the following formula:

$$\text{Methylation positive rate (\%)} = (\text{the number of methylation positive specimens/the total number of specimens}) \times 100$$

When a statistically significant difference was recognized between the cancerous tissue specimen and the non-cancerous tissue specimen or the normal specimen, such gene was defined as a marker which is methylated specifically to the cancerous tissue.

As a result, the promoter region of each gene of GDF7, ZNF132, CPXM1, RPL39L, DOK1, FUZ, MGAT3, and EHD3 was identified as a marker which is highly methylated in cancerous tissue of at least one cancer of the various types of cancers (see Figs. 1A to 1H, and Table 5 below). In Figs. 1A to 1H, the methylation positive rate of "normal specimens" is an average value of the methylation positive rates of 13 types of normal specimens, and the methylation positive rate of "normal blood" is an average value of the methylation positive rates of peripheral blood from 93 healthy subjects (Salhia B, et al.). According to Figs. 1A to 1H, the average value of the methylation positive rates of the normal specimens and the normal blood was zero in the marker of the embodiment, and thus the methylation was not observed in 13 types of normal specimens and normal blood. On the other hand, the methylation positive rate higher than the non-cancerous parts and normal specimens was confirmed in a plurality of predetermined cancers. This suggests that the methylation analysis of the gene in this example is useful as an index for diagnosis of any one of plural types of cancers.

[Table 5]

| Gene name | hg18 chromosome number | Probe position of Infinium HumanMethylation27 BeadChip |
|---|---|---|
| GDF7 | 2 | 20,729,328 |
| ZNF132 | 19 | 63,643,484 |
| CPXM1 | 20 | 2,729,489 |
| RPL39L | 3 | 188,340,169 |
| DOK1 | 2 | 74,634,604 |
| FUZ | 19 | 55,008,270 |
| MGAT3 | 22 | 38,183,536 |
| EHD3 | 2 | 31,310,293 |

As shown in Table 5, GDF7 is a gene existing in the chromosome 2, and the probe position in the Infinium HumanMethylation27 BeadChip is 20,729,328. As illustrated in Fig. 1A, when GDF7 was used, a high methylation positive rate was obtained in cancerous tissue of brain tumor, breast cancer, lung cancer, colon cancer, hepatocellular cancer (liver cancer), gastric cancer, renal cell cancer, uterine body cancer, and prostate cancer. These results suggest that the methylation analysis of the promoter region of GDF7 serves an index of existence of any cancer of brain tumor, breast cancer, lung cancer, colon cancer, hepatocellular cancer, gastric cancer, renal cell cancer, uterine body cancer, and prostate cancer, and is useful to screen for cancer.

As shown in Table 5, ZNF132 is a gene existing in the chromosome 19, and the probe position in the Infinium HumanMethylation27 BeadChip is 63,643,484. As illustrated in Fig. 1B, when ZNF132 was used, a high methylation positive rate was obtained in cancerous tissue of breast cancer, lung cancer, gastric cancer, colon cancer, hepatocellular cancer, renal cell cancer, and uterine body cancer. These results suggest that the methylation analysis of the promoter region of ZNF132 serves an index of existence of any cancer of breast cancer, lung cancer, gastric cancer, colon cancer, hepatocellular cancer, renal cell cancer, and uterine body cancer, and is useful to screen for cancer.

As shown in Table 5, CPXM1 is a gene existing in the chromosome 20, and the probe position in the Infinium HumanMethylation27 BeadChip is 2,729,489. As illustrated in Fig. 1c, when CPXM1 was used, a high methylation positive rate was obtained in cancerous tissue of lung cancer, gastric cancer, colon cancer, hepatocellular cancer, renal cell cancer, uterine body cancer, and prostate cancer. These results suggest that the methylation analysis of the promoter region of CPXM1 serves an index of existence of any cancer of breast cancer, lung cancer, gastric cancer, colon cancer, hepatocellular cancer, renal cell cancer, uterine body cancer, and prostate cancer, and is useful to screen for cancer.

As shown in Table 5, RPL39L is a gene existing in the chromosome 3, and the probe position in the Infinium HumanMethylation27 BeadChip is 188,340,169. As illustrated in Fig. 1D, when RPL39L was used, a high methylation positive rate was obtained in cancerous tissue of brain tumor, breast cancer, lung cancer, gastric cancer, colon cancer, hepatocellular cancer, renal cell cancer, uterine body cancer, and prostate cancer. These results suggest that the methylation analysis of the promoter region of RPL39L serves an index of existence of any cancer of brain tumor, breast cancer, lung cancer, gastric cancer, colon cancer, hepatocellular cancer, renal cell cancer, uterine body cancer, and prostate cancer, and is useful to screen for cancer.

As shown in Table 5, DOK1 is a gene existing in the chromosome 2, and the probe position in the Infinium HumanMethylation27 BeadChip is 74,634,604. As illustrated in Fig. 1E, when DOK1 was used, a high methylation positive rate was obtained in cancerous tissue of breast cancer, hepatocellular cancer, and prostate cancer. These results suggest that the methylation analysis of the promoter region of DOK1 serves an index of existence of any cancer of breast cancer, hepatocellular cancer, and prostate cancer, and is useful to screen for cancer.

As shown in Table 5, FUZ is a gene existing in the chromosome 19, and the probe position in the Infinium HumanMethylation27 BeadChip is 55,008,270. As illustrated in Fig. 1F, when FUZ was used, a high methylation positive rate was obtained in cancerous tissue of gastric cancer, colon cancer, hepatocellular cancer, and uterine body cancer. These results suggest that the methylation analysis of the promoter region of FUZ serves an index of existence of any cancer of gastric cancer, colon cancer, hepatocellular cancer, and uterine body cancer, and is useful to screen for cancer.

As shown in Table 5, MGAT3 is a gene existing in the chromosome 22, and the probe position in the Infinium HumanMethylation27 BeadChip is 38,183,536. As illustrated in Fig. 1G, when MGAT3 was used, a high methylation positive rate was obtained in cancerous tissue of breast cancer, lung cancer, gastric cancer, and colon cancer. These results suggest that the methylation analysis of the promoter region of MGAT3 serves an index of existence of any cancer of breast cancer, lung cancer, gastric cancer, and colon cancer, and is useful to screen for cancer.

As shown in Table 5, EHD3 is a gene existing in the chromosome 2, and the probe position in the Infinium HumanMethylation27 BeadChip is 31,310,293. As illustrated in Fig. 1H, when EHD3 was used, a high methylation positive rate was obtained in cancerous tissue of gastric cancer and colon cancer. These results suggest that the methylation analysis of the promoter region of EHD3 serves an index of existence of any cancer of gastric cancer and colon cancer, and is useful to screen for cancer.

Example 2: Comparison of Methylation Data (MassARRAY) of Promoter Region of GDF7 Gene

(1) Biological Sample

In this example, clinical specimens including a cancerous part and non-cancerous tissue specimen of hepatocellular cancer collected by hepatectomy from a patient different from that in Example 1, as well as a cancerous tissue specimen of gastric cancer and a normal stomach tissue specimen collected from a patient different from that in Example 1 were used as biological samples. The clinical specimens included normal brain tissue (1 specimen), brain tumor tissue (8 specimens), normal stomach tissue (2 specimens), cancerous tissue of gastric cancer (6 specimens), normal colon tissue (4 specimens), cancerous tissue of colon cancer (5 specimens), normal liver tissue (2 specimens), non-cancerous tissue of hepatocellular cancer (5 specimens), cancerous tissue of hepatocellular cancer (6 specimens), normal uterus tissue (4 specimens), cancerous tissue of uterine body cancer (4 specimens), non-cancerous tissue of breast cancer (3 specimens), and cancerous tissue of breast cancer (4 specimens). After being collected, these tissues were immediately frozen with liquid nitrogen, and stored at -80°C until use.

(2) Preparation of Measurement Samples and Control Samples

(i) Extraction of Genomic DNA

Genomic DNA was extracted from each tissue using QIAamp DNA Mini Kit (QIAGEN). The genome DNA contained in an obtained DNA solution was fragmented by Bioruptor (COSMO BIO Co., Ltd). For preparing calibration curves in mass spectrometry, genomic DNA of human peripheral blood lymphocytes was used as the control genomic DNA. The genomic DNA from human peripheral blood lymphocytes was amplified using GenomiPhi v2DNA amplification kit (GE Healthcare Life Sciences). The obtained amplified product consisted of a solution of non-methylated DNA fragments (0% methylated DNA). Then, a part of the solution of the non-methylated DNA fragments was subjected to reaction with SssI methylase (New England Biolabs) to methylate all cytosines in CG sequences, and a solution of methylated DNA fragments (100% methylated DNA) was obtained. The 0% methylated DNA solution and the 100% methylated DNA solution were mixed at a certain proportion to obtain a solution of 25%, 50%, and 75% methylated DNA.

(ii) Bisulfite Treatment

The respective DNA fragments (500 ng) obtained as described above were subjected to bisulfite treatment using EZ DNA Methylation Kit (Zymo Research), and the genome DNA after the treatment was dissolved in sterilized distilled water (80 μl).

(iii) Amplification by PCR and IVT

The methylated cytosine and uracil contained in the respective bisulfate-treated DNA fragments were converted to guanine and adenine by the PCR and IVT amplification.

Incidentally, it has been confirmed by MassARRAY® analysis using control samples described later that the primer set used in the PCR amplifies the methylated DNA and non-methylated DNA evenly. The sequence of the primer set for the present marker is shown in Table 5. The base sequence (sequence of positive stand) in the region to be analyzed by the primer set is shown in SEQ ID NO: 37. The base sequence after the bisulfite conversion in the region amplified with primer set for methylation detection of GDF7 is shown in SEQ ID NO: 47. The base sequence of SEQ ID NO: 47 represents the base sequence when all CpG sites existing in the region to be amplified with each primer set for methylation detection.

[Table 6]

| Marker | Base sequence of primer | SEQ ID NO: |
|---|---|---|
| GDF7 | Forward: AGGAAGAGAGGAAAATGTAATTTTAAAGGAAAAGG | 9 |
| | Reverse: CAGTAATACGACTCACTATAGGGAGAAGGCTCRAAAACCAAATCTCAAAAAAAC | 10 |

R represents A or G

The following tag sequence and T7 promoter sequence were added to the 5' end of the forward and reverse primers of the primer set for IVT reaction.

- Tag sequence: AGGAAGAGAG (SEQ ID NO: 35)
- T7 promoter sequence: CAGTAATACGACTCACTATAGGGAGAAGGCT

(SEQ ID NO: 36)

A PCR reaction solution was prepared by mixing the following reagents.

| | |
|---|---|
| 10x Hot Star buffer (QIAGEN) | 0.5 μL |
| 25 mM dNTP mix | 0.04 μL |
| Hot Star Taq (5U/μl) (QIAGEN) | 0.04 μL |

(continued)

| | |
|---|---|
| Primer mix | 2.0 μL |
| DNA solution | 1.0 μL |
| Water | 1.42 μL |
| Total | 5 μL |

The PCR reaction was carried out in the above reaction solution under the following conditions:

1 cycle of 94°C for 15 minutes;
45 cycles of 94°C for 20 seconds, 52°C for 30 seconds, and 72°C for 1 minute; and
72°C for 3 minutes

The obtained PCR products were dephosphorylated by SAP (Shrimp Alkaline Phosphatase) contained in a MassCLEAVE™ Reagent kit (Sequenom, Inc.). Then, the following reaction solution prepared by the kit was added to the PCR products.

5x T7 R&DNA polymerase buffer 0.89 μL

| | |
|---|---|
| T Cleavage mix | 0.24 μL |
| 100 mM DTT | 0.22 μL |
| T7 R&DNA polymerase | 0.44 μL |
| RNase A | 0.06 μL |
| RNase free water | 3.15 μL |
| Total | 5 μL |

The obtained mixture was incubated at 37°C for three hours to induce the IVT reaction and urasil- or thymine-specific cleavage. The resulting reaction products were purified by Clean Resin (Sequenom, Inc.) to obtain samples for mass spectrometry. These samples, in which samples derived from genomic DNA extracted from the clinical specimens were designated as measurement samples and samples derived from control genomic DNA were designated as control samples, were used for mass spectrometry described later.

(3) Analysis of Methylation Status by Mass Spectrometry using MassARRAY®

(i) Preparation of Calibration Curves

The control samples obtained as described above were subjected to the mass spectrometric analysis twice independently. The calibration curves were prepared for the respective primer sets based on the obtained analysis results, and correlation coefficients were calculated. Accordingly, it was confirmed that the used primer sets amplified the methylated DNA and non-methylated DNA evenly.

(ii) Analysis of Measurement Samples

The obtained measurement samples obtained as described above were subjected to the mass spectrometric analysis to obtain peaks of DNA fragments included in the measurement samples. Then, a portion of the base sequence of GDF7, from which each obtained peak was originated, was identified. A methylation score was calculated from the ratio between the area of the peaks of the fragments including the methylated CpG sites and the area of peaks of the fragments not including the methylated CpG sites in the fragments originating from the same base sequence. The results are illustrated in Fig. 2A.

According to Fig. 2A, the samples having the methylation score exceeded 0.1 in the promoter region of GDF7 gene were, in the cancerous tissue, 4 specimens/8 specimens in brain tumor tissue, 6 specimens/6 specimens in gastric cancer tissue, 5 specimens/5 specimens in colon cancer tissue, 4 specimens/6 specimens in hepatocellular cancer tissue, 4 specimens/4 specimens in uterine body cancer tissue, and 3 specimens/4 specimens in breast cancer tissue. In non-cancerous tissue, such samples were 5 specimens/5 specimens in non-cancerous tissue of hepatocellular cancer, and 0 specimens/3 specimens in non-cancerous tissue of breast cancer. In normal tissue, such samples were 0 specimens/1 specimens in brain tissue, 0 specimens/2 specimens in stomach tissue, 0 specimens/4 specimens in colon tissue, 0 specimens/2 specimens in liver tissue, and 3 specimens/4 specimens in uterus tissue. These results are summarized in the following Table 7.

[Table 7]

| | | Methylation score > 0 (specimen) | The number of all specimens (specimen) |
|---|---|---|---|
| Cancerous tissue | Brain tumor | 4 | 8 |
| | Gastric cancer | 6 | 6 |
| | Colon cancer | 5 | 5 |
| | Hepatocellular cancer | 4 | 6 |
| | Uterine body cancer | 4 | 4 |
| | Breast cancer | 3 | 4 |
| Non-cancerous tissue | Hepatocellular cancer | 5 | 5 |
| | Breast cancer | 0 | 3 |
| Normal tissue | Brain | 0 | 1 |
| | Stomach | 0 | 2 |
| | Colon | 0 | 4 |
| | Liver | 0 | 2 |
| | Uterus | 3 | 4 |

As is evident from Fig. 2A and the results listed in Table 7, the analysis of methylation frequency using GDF7 can be used in the assistance of diagnosis and relapse monitoring of brain tumor, gastric cancer, colon cancer, hepatocellular cancer, uterine body cancer, breast cancer, and the like based on the difference in methylation frequency of the cancerous tissue specimen, the non-cancerous tissue specimen, and the normal specimen.

Example 3 (reference example): Comparison of Methylation Data (MassARRAY) of Promoter Region of ZNF132 gene

The methylation score was calculated in a manner similar to Example 2 except that normal stomach tissue (2 specimens), cancerous tissue (6 specimens) of gastric cancer, non-cancerous tissue (4 specimens) of colon cancer, cancerous tissue (5 specimens) of colon cancer, normal liver tissue (2 specimens), non-cancerous tissue (5 specimens) of hepatocellular cancer, cancerous tissue (6 specimens) of hepatocellular cancer, normal uterus tissue (4 specimens), cancerous tissue (4 specimens) of uterine body cancer, non-cancerous tissue (3 specimens) of breast cancer, and cancerous tissue (4 specimens) of breast cancer were used as clinical specimens, and those shown in Table 8 were used for the MassARRAY analysis (base sequence (sequence of positive strand) of the region analyzed with such primer set is represented by SEQ ID NO: 38). The base sequence after bisulfite conversion of the region amplified with the primer set for methylation detection of ZNF132 was represented by SEQ ID NO: 48. The base sequence of SEQ ID NO: 48 represents the base sequence when all CpG sites existing in the region amplified with each primer set for methylation detection are methylated.

[Table 8]

| Marker | Base sequence of primer | SEQ ID NO: |
|---|---|---|
| ZNF132 | Forward: AGGAAGAGAGTTGTTAAGGTGATTGAGGAAT | 11 |
| | Reverse: CAGTAATACGACTCACTATAGGGAGAAGGCTCCAAACTATAATTAACCAAC | 12 |

The tag sequence of SEQ ID NO: 35 and the T7 promoter sequence of SEQ ID NO: 36 are added to the 5' end of the forward primer and the reverse primer of the primer set for IVT reaction.

The results are shown in Fig. 2B. According to Fig. 2B, the samples having the methylation score exceeded 0.1 in the promoter region of ZNF12 gene were, in the cancerous tissue, 6 specimens/6 specimens in gastric cancer tissue, 5 specimens /5 specimens in colon cancer tissue, 4 specimens/6 specimens in hepatocellular cancer tissue, 4 specimens/4 specimens in uterine body cancer tissue, and 3 specimens/4 specimens in breast cancer tissue. In the non-cancerous tissue, such samples were 0 specimens/4 specimens in non-cancerous tissue of colon cancer, 0 specimens/5 specimens in non-cancerous tissue of hepatocellular cancer, and 0 specimens/3 specimens in non-cancerous tissue of breast cancer. In the normal tissue, the samples were 0 specimens/2 specimens in stomach tissue, 0 specimens/2 specimens in liver tissue, and 0 specimens/4 specimens in uterus tissue. These results are summarized in Table 9 below.

[Table 9]

| | | Methylation score > 0.1 (specimen) | The number of all specimens (specimen) |
|---|---|---|---|
| Cancerous tissue | Gastric cancer | 6 | 6 |
| | Colon cancer | 5 | 5 |
| | Hepatocellular cancer | 4 | 6 |
| | Uterine body cancer | 4 | 4 |
| | Breast cancer | 3 | 4 |
| Non-cancerous tissue | Colon cancer | 0 | 4 |
| | Hepatocellular cancer | 0 | 5 |
| | Breast cancer | 0 | 3 |
| Normal tissue | Stomach | 0 | 2 |
| | Liver | 0 | 2 |
| | Uterus | 0 | 4 |

As is evident from Fig. 2B and the results listed in Table 9, in the analysis of methylation frequency using ZNF132, it can be used in the assistance of diagnosis and relapse monitoring of gastric cancer, colon cancer, hepatocellular cancer, uterine body cancer, breast cancer, and the like based on the difference in methylation frequency of the cancerous tissue specimen, the non-cancerous tissue specimen, and the normal specimen.

Example 4 (reference example): Comparison of Methylation Data (MassARRAY) of Promoter Region of CPXM1 Gene

The methylation score was calculated in a manner similar to Example 2 except that the normal stomach tissue (2 specimens), the cancerous tissue (6 specimens) of gastric cancer, the non-cancerous tissue (4 specimens) of colon cancer, the cancerous tissue (5 specimens) of colon cancer, the normal liver tissue (2 specimens), the non-cancerous tissue (5 specimens) of hepatocellular cancer, the cancerous tissue (6 specimens) of hepatocellular cancer, the normal uterus tissue (4 specimens), the cancerous tissue (4 specimens) of uterine body cancer, the non-cancerous tissue (3 specimens) of breast cancer, and the cancerous tissue (4 specimens) of breast cancer were used as clinical specimens, and those shown in Table 10 were used for the MassARRAY analysis (base sequence (sequence of positive strand) of the region analyzed with the primer set is represented by SEQ ID NO: 39). The base sequence after bisulfite conversion of the region amplified with the primer set for methylation detection of the CPXM1 is represented by SEQ ID NO: 49. The base sequence of SEQ ID NO: 49 represents the base sequence when all the CpG sites existing in the region amplified with each primer set for methylation detection are methylated.

[Table 10]

| Marker | Base sequence of primer | SEQ ID NO: |
|---|---|---|
| CPXM1 | Forward: AGGAAGAGAGGGGGTTGATTTTTAAGGGGT | 13 |
| | Reverse: CAGTAATACGACTCACTATAGGGAGAAGGCTCCACCAAATAATCTCTAATCTCTTCA | 14 |

The tag sequence of SEQ ID NO: 35 and the T7 promoter sequence of SEQ ID NO: 36 arc added to the 5' end of the forward primer and the reverse primer of the primer set for IVT reaction.

The results are shown in Fig. 2C. According to Fig. 2C, the samples having the methylation score exceeded 0.1 in the promoter region of CPXM1 gene were, in the cancerous tissue, 6 specimens/6 specimens in gastric cancer tissue, 3 specimens/5 specimens in colon cancer tissue, 5 specimens/6 specimens in hepatocellular cancer tissue, 4 specimens/4 specimens in uterine body cancer tissue, and 3 specimens/4 specimens in breast cancer tissue. In the non-cancerous tissue, such samples were 0 specimens/4 specimens in non-cancerous tissue of colon cancer, 0 specimens/5 specimens in non-cancerous tissue of hepatocellular cancer, and 0 specimens/3 specimens in non-cancerous tissue of breast cancer. In the normal tissue, the samples were 0 specimens/2 specimens in stomach tissue, 0 specimens/2 specimens in liver tissue, and 0 specimens/4 specimens in uterus tissue. These results are summarized in Table 11 below.

[Table 11]

| | | Methylation score > 0.1 (specimen) | The number of all specimens (specimen) |
|---|---|---|---|
| Cancerous tissue | Gastric cancer | 6 | 6 |
| | Colon cancer | 3 | 5 |
| | Hepatocellular cancer | 5 | 6 |
| | Uterine body cancer | 4 | 4 |
| | Breast cancer | 3 | 4 |
| Non-cancerous tissue | Colon cancer | 0 | 4 |
| | Hepatocellular cancer | 0 | 5 |
| | Breast cancer | 0 | 3 |
| Normal tissue | Stomach | 0 | 2 |
| | Liver | 0 | 2 |
| | Uterus | 0 | 4 |

As is evident from Fig. 2C and the results listed in Table 11, in the analysis of the methylation frequency using CPXM1, it can be used in the assistance of diagnosis and relapse monitoring of gastric cancer, colon cancer, hepatocellular cancer, uterine body cancer, breast cancer, and the like based on the difference in methylation frequency of the cancerous tissue specimen, the non-cancerous tissue specimen, and the normal specimen.

Example 5 (reference example) : Comparison of Methylation Data (MassARRAY) of Promoter Region of RPL39L Gene

The methylation score was calculated in a manner similar to Example 2 except that normal stomach tissue (1 specimen), brain tumor tissue (8 specimens), normal stomach tissue (2 specimens), cancerous tissue (6 specimens) of gastric cancer, non-cancerous tissue (4 specimens) of colon cancer, cancerous tissue (5 specimens) of colon cancer, normal liver tissue (2 specimens), non-cancerous tissue (5 specimens) of hepatocellular cancer, cancerous tissue (6 specimens) of hepatocellular cancer, normal uterus tissue (4 specimens), and cancerous tissue (4 specimens) of uterine body cancer were used as clinical specimens, and those shown in Table 12 were used for the MassARRAY analysis (base sequence (sequence of positive strand) of the region analyzed with the primer set is represented by SEQ ID NO: 40). The base sequence after bisulfite conversion of the region amplified with the primer set for methylation detection of RPL39L is represented by SEQ ID NO: 50. The base sequence of SEQ ID NO: 50 represents the base sequence when all CpG sites existing in the region amplified with each primer set for methylation detection are methylated.

[Table 12]

| Marker | Base sequence of primer | SEQ ID NO: |
|---|---|---|
| RPL39L | Forward: AGGAAGAGAGTTTAAGTTGTGAGTTTTTGG | 15 |
| | Reverse: CAGTAATACGACTCACTATAGGGAGAAGGCTATCCCCCATTCTTCCTTAATT | 16 |

The tag sequence of SEQ ID NO: 35 and the T7 promoter sequence of SEQ ID NO: 36 are added to the 5' end of the forward primer and the reverse primer of the primer set for IVT reaction.

The results are shown in Fig. 2D. According to Fig. 2D, the samples having the methylation score exceeded 0.1 in the promoter region of RPL39L gene were, in cancerous tissue, 7 specimens/8 specimens in brain tumor tissue, 4 specimens/6 specimens in gastric cancer tissue, 2 specimens/5 specimens in colon cancer tissue, 4 specimens/6 specimens in hepatocellular cancer tissue, and 3 specimens/4 specimens in uterine body cancer tissue. In non-cancerous tissue, such samples were 1 specimens/4 specimens in non-cancerous tissue of colon cancer, and 0 specimens/5 specimens in non-cancerous tissue of hepatocellular cancer. In normal tissue, the samples were 0 specimens/1 specimens in brain tissue, 0 specimens/2 specimens in stomach tissue, 0 specimens/2 specimens in liver tissue, and 0 specimens/4 specimens in uterus tissue. These results are summarized in Table 13 below.

[Table 13]

| | | Methylation score > 0.1 (specimen) | The number of all specimens (specimen) |
|---|---|---|---|
| Cancerous tissue | Brain tumor | 7 | 8 |
| | Gastric cancer | 4 | 6 |
| | Colon cancer | 2 | 5 |
| | Hepatocellular cancer | 4 | 6 |
| | Uterine body cancer | 3 | 4 |
| Non-cancerous tissue | Colon cancer | 1 | 4 |
| | Hepatocellular cancer | 0 | 5 |
| Normal tissue | Brain | 0 | 1 |
| | Stomach | 0 | 2 |
| | Liver | 0 | 2 |
| | Uterus | 0 | 4 |

As is evident from Fig. 2D and the results listed in Table 13, in the analysis of the methylation frequency using RPL39L, it can be used in the assistance of diagnosis and relapse monitoring of brain tumor, gastric cancer, colon cancer, hepatocellular cancer, uterine body cancer, and the like based on the difference in methylation frequency of the cancerous tissue specimen, the non-cancerous tissue specimen, and the normal specimen.

Example 6 (reference example) : Comparison of Methylation Data (MassARRAY) of Promoter Region of DOK1 Gene

The methylation score was calculated in a manner similar to Example 2 except that that normal liver tissue (2 specimens), non-cancerous tissue (5 specimens) of hepatocellular cancer, cancerous tissue (6 specimens) of hepatocellular cancer, non-cancerous tissue (3 specimens) of breast cancer, and cancerous tissue (4 specimens) of breast cancer were used as clinical specimens, and those shown in Table 14 were used for the MassARRAY analysis (base sequence (sequence of positive strand) of the region analyzed with the primer set is represented by SEQ ID NO: 41). The base sequence after bisulfite conversion of the region amplified with the primer set for methylation detection of DOK1 is represented by SEQ ID NO: 51. The base sequence of SEQ ID NO: 51 represents the base sequence when all CpG sites existing in the region amplified with each primer set for methylation detection are methylated.

[Table 14]

| Marker | Base sequence of primer | SEQ ID NO: |
|---|---|---|
| DOK1 | Forward: AGGAAGAGAGAGAGTTTAGAAATAGGGAGA | 17 |
| | Reverse: CAGTAATACGACTCACTATAGGGAGAAGGCTAACTTCCAAACRAAATTAAAAAC | 18 |

The tag sequence of SEQ ID NO: 35 and the T7 promoter sequence of SEQ ID NO: 36 are added to the 5' end of the forward primer and the reverse primer of the primer set for IVT reaction.

The results are shown in Fig. 2E. According to Fig. 2E, the samples having the methylation score exceeded 0.1 in the promoter region of the DOK1 gene were, in cancerous tissue, 6 specimens/6 specimens in hepatocellular cancer tissue, and 3 specimens/4 specimens in breast cancer tissue. In non-cancerous tissue, such samples were 0 specimen/5 specimens in non-cancerous tissue of hepatocellular cancer, and 0 specimens/3 specimens in non-cancerous tissue of breast cancer. In normal tissue, the samples were 0 specimens/2 specimens in liver tissue. These results are summarized in Table 15 below.

[Table 15]

| | | Methylation score > 0.1 (specimen) | The number of all specimens (specimen) |
|---|---|---|---|
| Cancerous tissue | Hepatocellular cancer | 6 | 6 |
| | Breast cancer | 3 | 4 |
| Non-cancerous tissue | Hepatocellular cancer | 0 | 5 |
| | Breast cancer | 0 | 3 |
| Normal tissue | Liver | 0 | 2 |

As is evident from Fig, 2E and the results listed in Table 15, in the analysis of the methylation frequency using DOK1, it can be used in the assistance of diagnosis and relapse monitoring of hepatocellular cancer, breast cancer, and the like based on the difference in methylation frequency of the cancerous tissue specimen, the non-cancerous tissue specimen, and the normal specimen.

Example 7 (reference example) : Comparison of Methylation Data (MassARRAY) of Promoter Region of FUZ Gene

The methylation score was calculated in a manner similar to Example 2 except that that normal stomach tissue (2 specimens), cancerous tissue (6 specimens) of gastric cancer, non-cancerous tissue (4 specimens) of colon cancer, cancerous tissue (5 specimens) of colon cancer, normal liver tissue (2 specimens), non-cancerous tissue (5 specimens) of hepatocellular cancer, and cancerous tissue (6 specimens) of hepatocellular cancer were used as clinical specimens, and those shown in Table 16 were used for the MassARRAY analysis (base sequence (sequence of positive strand) of the region analyzed with the primer set is represented by SEQ ID NO: 42). The base sequence after bisulfite conversion of the region amplified with the primer set for methylation detection of FUZ is represented by SEQ ID NO: 55. The base sequence of SEQ ID NO: 55 represents the base sequence when all CpG sites existing in the region amplified with each primer set for methylation detection are methylated.

[Table 16]

| Marker | Base sequence of primer | SEQ ID NO: |
|---|---|---|
| FUZ | Forward: AGGAAGAGAGAGGTATAGTAGATGTATAGT | 19 |
| | Reverse: CAGTAATACGACTCACTATAGGGAGAAGGCTAAATTAATCCTCTTCCAATC | 20 |

The tag sequence of SEQ ID NO: 35 and the T7 promoter sequence of SEQ ID NO: 36 are added to the 5' end of the forward primer and the reverse primer of the primer set for IVT reaction.

The results are shown in Fig. 2F. According to Fig. 2F, the samples having the methylation score exceeded 0.1 in the promoter region of FUZ gene were, in cancerous tissue, 4 specimens/6 specimens in gastric cancer tissue, 3 specimens/5 specimens in colon cancer tissue, and 4 specimens/6 specimens in hepatocellular cancer tissue. In non-cancerous tissue, such samples were 0 specimen/4 specimens in non-cancerous tissue of colon cancer, and 0 specimen/5 specimens in non-cancerous tissue of hepatocellular cancer. In normal tissue, the samples were 0 specimen/2 specimens in stomach tissue, and 0 specimen/2 specimens in liver tissue. These results are summarized in Table 17 below.

[Table 17]

| | | Methylation score > 0.1 (specimen) | The number of all specimens (specimen) |
|---|---|---|---|
| Cancerous tissue | Gastric cancer | 4 | 6 |
| | Colon cancer | 3 | 5 |
| | Hepatocellular cancer | 4 | 6 |
| Non-cancerous tissue | Colon cancer | 0 | 4 |
| | Hepatocellular cancer | 0 | 5 |
| Normal tissue | Stomach | 0 | 2 |
| | Liver | 0 | 2 |

As is evident from Fig. 2F and the results of Table 17, in the analysis of the methylation frequency using FUZ, it can be used in the assistance of diagnosis and relapse monitoring of gastric cancer, colon cancer, hepatocellular cancer, and the like based on the difference in methylation frequency of the cancerous tissue specimen, the non-cancerous tissue specimen, and the normal specimen.

Example 8 (reference example) : Comparison of Methylation Data (MassARRAY) of Promoter Region of EHD3 Gene

The methylation score was calculated in a manner similar to Example 2 except that that normal stomach tissue (2 specimens), cancerous tissue (6 specimens) of gastric cancer, non-cancerous tissue (4 specimens) of colon cancer, and cancerous tissue (5 specimens) of colon cancer were used as clinical specimens, and those shown in Table 18 were used for the MassARRAY analysis (base sequence (sequence of positive strand) of the region analyzed with the primer set is represented by SEQ ID NO: 43). The base sequence after bisulfite conversion of the region amplified with the primer set for methylation detection of EHD3 is represented by SEQ ID NO: 56. The base sequence of SEQ ID NO: 56 represents the base sequence when all CpG sites existing in the region amplified with each primer set for methylation detection are methylated.

[Table 18]

| Marker | Base sequence of primer | SEQ ID NO: |
|---|---|---|
| EHD3 | Forward: AGGAAGAGAGGATTTTAGTAATTGAAGGAG | 21 |
| | Reverse: CAGTAATACGACTCACTATAGGGAGAAGGCTAATACTAACCCAATCCAATA | 22 |

The tag sequence of SEQ ID NO: 35 and the T7 promoter sequence of SEQ ID NO: 36 are added to the 5' end of the forward primer and the reverse primer of the primer set for IVT reaction.

The results are shown in Fig. 2G. According to Fig. 2G, the samples having the methylation score exceeded 0.1 in the promoter region of EHD3 gene were, in cancerous tissue, 5 specimens/6 specimens in gastric cancer tissue, and 3 specimens/5 specimens in colon cancer tissue. In non-cancerous tissue, such samples were 0 specimen/4 specimens in non-cancerous tissue of colon cancer. In normal tissue, the samples were 0 specimen/2 specimens in stomach tissue. These results are summarized in Table 19 below.

[Table 19]

| | | Methylation score > 0.1 (specimen) | The number of all specimens (specimen) |
|---|---|---|---|
| Cancerous tissue | Gastric cancer | 5 | 6 |
| | Colon cancer | 3 | 5 |
| Non-cancerous tissue | Colon cancer | 0 | 4 |

(continued)

| | | Methylation score > 0.1 (specimen) | The number of all specimens (specimen) |
|---|---|---|---|
| Normal tissue | Gastric cancer | 0 | 2 |

As is evident from Fig. 2G and the results of Table 19, in the analysis of the methylation frequency using EHD3, it can be used in the assistance of diagnosis and relapse monitoring of gastric cancer, colon cancer, and the like based on the difference in methylation frequency of the cancerous tissue specimen, the non-cancerous tissue specimen, and the normal specimen.

Example 9 (reference example) : Comparison of Methylation Data (MSP) of Promoter Region of FUZ Gene (1) Biological Sample

In this example, the non-cancerous tissue (2 specimens) of gastric cancer, the cancerous tissue (4 specimens) of gastric cancer, the non-cancerous tissue (4 specimens) of colon cancer, the cancerous tissue (5 specimens) of colon cancer, the normal liver tissue (2 specimens), the non-cancerous tissue (5 specimens) of hepatocellular cancer, and the cancerous tissue (6 specimens) of hepatocellular cancer were used as biological samples.

(2) Preparation of Measurement Samples and Control Samples

(i) Extraction of Genomic DNA

Genomic DNA was extracted from each tissue described above using QIAamp DNA Mini Kit (QIAGEN). The genomic DNA contained in an obtained DNA solution was fragmented by Bioruptor (COSMO BIO Co., Ltd).

Further, genomic DNA of human peripheral blood lymphocytes was used as the control genomic DNA. The genomic DNA from human peripheral blood lymphocytes was amplified using GenomiPhi v2DNA amplification kit (GE Healthcare Life Sciences). The obtained amplified product consisted of non-methylated DNA. The amplified product was then fragmented with Bioruptor (COSMO BIO Co., Ltd.) to obtain a solution of non-methylated DNA fragments (0% methylated DNA). A part of the solution of the non-methylated DNA fragments was subjected to reaction with SssI methylase (New England Biolabs) to methylate all cytosines in CG sequences, and a solution of methylated DNA fragments (100% methylated DNA) was obtained.

(ii) Bisulfite Treatment

The respective DNA fragments (500 ng) obtained as described above were subjected to bisulfite treatment using EZ DNA Methylation Kit (Zymo Research), and the genomic DNA after the treatment was dissolved in sterilized distilled water (80 μl).

(3) Methylation-specific PCR (MSP)

The MSP was conducted using the measurement samples and the control samples (DNA after bisulfite treatment) obtained as described above in (2). The composition of the PCR reagent used in the MSP, the primer set, and the reaction conditions of the PCR are described below.

<PCR reagent>

| | |
|---|---|
| DDW (sterilized water) | 16.75 μL |
| 10 × PCR buffer with MgC12 (Roche) | 2.5 μL |
| 2 mM dNTP mix | 2.5μL |
| 10μM sense primer | 1.0 μL |
| 10μM anti-sense primer | 1.0 μL |
| Faststart Taq polymerase (Roche) | 0.25 μL |

(continued)

| | |
|---|---|
| Measurement sample or control sample | 1.0 μL |
| Total | 25 μL |

<Primer set>

The primer set used in the MSP is shown in Table 20. Such primer sets are primer sets that can obtain the amplified product when DNA in the region to be amplified is methylated. The base sequences of the region analyzed with the primer set shown in Table 20 for the promoter region of FUZ gene are respectively represented by SEQ ID NO: 44. The base sequence of SEQ ID NO: 44 is the sequence of positive strand. The base sequence after bisulfite conversion in the region amplified with the primer set for methylation detection (M primer of SEQ ID NOs: 23 and 24) of FUZ is represented by SEQ ID NO: 52. The base sequence of SEQ ID NO: 52 represents the base sequence when all CpG sites existing in the region amplified with each primer set for methylation detection are methylated. The base sequence of after bisulfite conversion in the region amplified with the primer set for non-methylation detection (U primer of SEQ ID NOs: 25 and 26) of FUZ is represented by SEQ ID NO: 57.

[Table 20]

| Gene symbol | | Primer | Base sequence of primer | SEQ ID NO: | Strand | Size of PCR product (bp) | Annealing temperature (°C) | Cycle (Y) |
|---|---|---|---|---|---|---|---|---|
| FUZ | M | FUZ_MF | ATAGTAGATGTATAGTGTCGTTCGT | 23 | TOP | 123 | 54 | 36 |
| | | FUZ_MR | CAAACCGAATTACTCTAATTACCG | 24 | TOP | | | |
| | U | FUZ_UF | GTATAGTAGATGTATAGTGTTGTTTGT | 25 | TOP | 123 | 52 | 36 |
| | | FUZ_UR | AACCAAATTACTCTAATTACCACC | 26 | TOP | | | |

<PCR reaction condition>

95°C for 6 minutes;
Y cycles of 95°C for 30 seconds, X°C for 30 seconds, and 72°C for 30 seconds;
72°C for 7 minutes; and
keep at 16°C

In the above reaction conditions, "X" and "Y" represent the annealing temperature and the number of cycles, respectively, shown in Table 20.

(4) Result Analysis of Methylation-specific PCR (MSP)

The amplified product obtained with the MSP described above was checked with 2% agarose gel electrophoresis. The result is shown in Fig. 3A. In the figure, "0" and "100" indicated in "control" represent 0% methylated control sample and 100% methylated control sample, respectively.

In the PCR for FUZ gene, the band derived from the methylated CpG was not detected in the non-cancerous tissue of gastric cancer, the non-cancerous tissue of colon cancer, the normal liver tissue, and the non-cancerous tissue of hepatocellular cancer. On the other hand, the band of the methylated DNA was detected in 3 specimens/4 specimens for the cancerous tissue of gastric cancer, 3 specimens/5 specimens for the cancerous tissue of colon cancer, and 4 specimens/6 specimens for the cancerous tissue of hepatocellular cancer. As is evident from Fig. 3A, in the analysis of the methylation frequency using FUZ, it can be used in the assistance of diagnosis and relapse monitoring of gastric cancer, colon cancer, hepatocellular cancer, and the like based on the difference in methylation frequency of the cancerous tissue specimen and the normal specimen.

Example 10 (reference example) : Comparison of Methylation Data (MSP) of Promoter Region of EHD3 Gene

The amplified product was obtained by the MSP in a manner similar to Example 9 except that the non-cancerous tissue (2 specimens) of gastric cancer, the cancerous tissue (4 specimens) of gastric cancer, the non-cancerous tissue (4 specimens) of colon cancer, and the cancerous tissue (5 specimens) of colon cancer were used as biological samples, and that the primer set shown in Table 21 was used (base sequence of region to be analyzed is represented by SEQ ID NO: 45, base sequence of SEQ ID NO: 45 is sequence of positive strand). The base sequence after bisulfite conversion of the region amplified with the primer set for methylation detection (M primer of SEQ ID NOs: 27 and 28) of EHD3 is represented by SEQ ID NO: 53. The base sequence of SEQ ID NO: 53 represents the base sequence when all CpG sites existing in the region amplified with each primer set for methylation detection are methylated. The base sequence after bisulfite conversion of the region amplified with the primer set for non-methylation detection (U primer of SEQ ID NOs: 29 and 30) of EHD3 is represented by SEQ ID NO: 58.

[Table 21]

| Gene symbol | | Primer | Base sequence of primer | SEQ ID NO: | Strand | Size of PCR product (bp) | Annealing temperature (°C) | Cycle (Y) |
|---|---|---|---|---|---|---|---|---|
| EHD3 | M | EHD3_MF | ATTTTAGTAATTGAAGGAGAGGCG | 27 | TOP | 150 | 68 | 36 |
| | | EHD3_MR | CCAATCCAATAAAAAAATACCGAT | 28 | TOP | | | |
| | U | EHD3_UF | ATTTTAGTAATTGMGGAGAGGTGA | 29 | TOP | 152 | 56 | 36 |
| | | EHD3_UR | ACCCAATCCAATAAAAAAATACCA | 30 | TOP | | | |

The amplified product obtained with the MSP described above was checked with 2% agarose gel electrophoresis. The result is shown in Fig. 3B. In the figure, "0" and "100" indicated in "control" represent 0% methylated control sample and 100% methylated control sample, respectively.

In the PCR for EHD3 gene, the band derived from the methylated CpG was not detected in the non-cancerous tissue of gastric cancer and the non-cancerous tissue of colon cancer. On the other hand, the band of the methylated DNA was detected in 3 specimens/4 specimens for the cancerous tissue of gastric cancer and 5 specimens/5 specimens for the cancerous tissue of colon cancer. As is evident from Fig. 3B, in the analysis of the methylation frequency using the EHD3, it can be used in the assistance of diagnosis and relapse monitoring of gastric cancer, colon cancer, and the like based on the difference in methylation frequency of the cancerous tissue specimen and the non-cancerous tissue specimen.

Example 11 (reference example) : Comparison of Methylation Data (MSP) of Promoter Region of MGAT3 Gene

The amplified product was obtained by the MSP in a manner similar to Example 9 except that the non-cancerous tissue (2 specimens) of gastric cancer, the cancerous tissue (6 specimens) of gastric cancer, the non-cancerous tissue (4 specimens) of colon cancer, and the cancerous tissue (5 specimens) of colon cancer were used for the biological sample, and that the primer set shown in Table 22 was used (base sequence of region to be analyzed is represented by SEQ ID NO: 46, base sequence of SEQ ID NO: 46 is sequence of positive strand). The base sequence after bisulfite conversion of the region amplified with the primer set for methylation detection (M primer of SEQ ID NOs: 31 and 32) of MGAT3 is represented by SEQ ID NO: 54. The base sequence of SEQ ID NO: 54 represents the base sequence when all CpG sites existing in the region amplified with each primer set for methylation detection are methylated. The base sequence after bisulfite conversion of the region amplified with the detection primer set for non-methylation detection (U primer of SEQ ID NOs: 33 and 34) of MGAT3 is represented by SEQ ID NO: 59.

[Table 22]

| Gene symbol | | Primer | Base sequence of primer | SEQ ID NO: | Strand | Size of PCR product (bp) | Annealing temperature (°C) | Cycle (Y) |
|---|---|---|---|---|---|---|---|---|
| MGAT 3 | M | MGAT3_MF | TCGTTGTTGAGATTTAGC | 31 | TOP | 74 | 58 | 36 |
| | | MGAT3_MR | AAAATAAAAAAACCGCGAC | 32 | TOP | | | |
| | U | MGAT3_UF | TTGTTGTTGAGATTTAGT | 33 | TOP | 73 | 52 | 34 |
| | | MGAT3_UR | AAATAAAAAAACCACAACAC | 34 | TOP | | | |

The amplified product obtained with the MSP described above was checked with 2% agarose gel electrophoresis. The result is shown in Fig. 3C. In the figure, "0" and "100" indicated in "control" represent 0% methylated control sample and 100% methylated control sample, respectively.

In the PCR for MGAT3 gene, the band derived from the methylated CpG was not detected in the non-cancerous tissue of gastric cancer and the non-cancerous tissue of colon cancer. On the other hand, the band of the methylated DNA was detected in 5 specimens/6 specimens for the cancerous tissue of gastric cancer and 4 specimens/5 specimens for the cancerous tissue of colon cancer. As is evident from Fig. 3C, in the analysis of the methylation frequency using the MGAT3, it can be used in the assistance of diagnosis and relapse monitoring of gastric cancer, colon cancer, and the like based on the difference in methylation frequency of the cancerous tissue specimen and the non-cancerous tissue specimen.

## Claims

1. A method for obtaining information on plural types of cancers comprising the steps of:

   preparing a DNA sample from a biological sample collected from a subject;
   analyzing methylation status of a CpG site in a promoter region of GDF7 gene in the DNA sample obtained in the preparing step; and
   obtaining information on plural types of cancers in the subject based on an analysis result obtained in the analyzing step;
   wherein the plural types of cancers are two or more cancers selected from a group consisting of brain tumor, hepatocellular cancer, colon cancer, gastric cancer, uterine body cancer, lung cancer, breast cancer, prostate cancer, and renal cell cancer.

2. The method according to claim 1, wherein the analyzing step is a step of analyzing the presence or absence of methylation of at least one CpG site.

3. The method according to claim 2, wherein
   the information on the plural types of cancers is information related to the presence or absence of cancer cells; and the information obtaining step is a step of obtaining information indicating that cancer cells derived from any of the plural types of cancers exist in the biological sample when the analysis result indicating that a methylated CpG site is present is obtained.

4. The method according to claim 1, wherein the analyzing step is a step of analyzing methylation frequency.

5. The method according to claim 4, wherein
   the information on the plural types of cancers is information related to the presence or absence of cancer cells; and the information obtaining step is a step of obtaining information indicating that cancer cells derived from any of the plural types of cancers exist in the biological sample when the methylation frequency obtained in the analyzing step is higher than a predetermined threshold value or is the same as the threshold value.

6. The method according to any one of claims 1 to 5, wherein

   the plural types of cancers are two or more cancers selected from a group consisting of brain tumor, hepatocellular cancer, colon cancer, gastric cancer, uterine body cancer, lung cancer, breast cancer, prostate cancer, and renal cell cancer, and
the information on the plural types of cancers is information indicating that cancer cells derived from any of the plural types of cancers exist in the biological sample.

**7.** The method according to any one of claims 1 to 6, wherein the analyzing step is a step of analyzing the methylation status of a CpG site in a region of the GDF7 gene having base sequence SEQ ID NO: 37 in the DNA sample obtained in the preparing step.

**8.** In vitro use of a marker for obtaining information on plural types of cancers by methylation analysis, which marker is at least one CpG site located in a promoter region of GDF7.

**9.** The in vitro use of the marker according to claim 8, wherein the marker is a polynucleotide obtained by subjecting an isolated DNA to bisulfite treatment, the isolated DNA having a contiguous base sequence in an entire or partial promoter region of GDF7 and containing at least one CpG site in the promoter region and at least one cytosine not included in CpG sites.

**10.** A determination device for obtaining information on plural types of cancers comprising
a computer including a processor and a memory, the memory being controlled by the processor wherein
the memory is recorded with a computer program for causing the computer to execute the steps of claim 1 and the step of outputting a determination result.

**11.** The determination device according to claim 10, wherein the analysis result is presence or absence of methylation of at least one CpG site.

**12.** The determination device according to claim 11, wherein
the information on the plural types of cancers is information related to the presence or absence of cancer cells; and
the step of outputting information is the step of outputting information indicating that cancer cells derived from any of the plural types of cancers exist in the biological sample when the analysis result indicates that the presence of a methylated CpG site.

**13.** The determination device according to claims 10 to 12, wherein the analysis result is methylation frequency.

**14.** The determination device according to claims 10 to 13 further comprises a measurement device which measures a DNA sample from a biological sample collected from a subject, wherein the measurement device is connected to the computer.

**15.** The determination device according to claim 14, wherein the measurement device is a mass spectrometer or a fluorescence image scanner.

## Patentansprüche

**1.** Verfahren zum Erhalten von Information über mehrere Typen von Krebs, umfassend die folgenden Schritte:

Herstellen einer DNA-Probe aus einer biologischen Probe, gewonnen von einem Subjekt;
Analysieren des Methylierungsstatus einer CpG-Stelle in einer Promotorregion des GDF7-Gens in der DNA-Probe, erhalten in dem Herstellungsschritt; und
Erhalten von Information über mehrere Typen von Krebs in dem Subjekt auf Grundlage eines Analyseergebnisses, erhalten in dem Analyseschritt;
wobei die mehreren Typen von Krebs zwei oder mehr Krebsarten sind, ausgewählt aus einer Gruppe, die besteht aus Gehirntumor, hepatozellulärem Krebs, Darmkrebs, Magenkrebs, Gebärmutterkrebs (uterus body cancer), Lungenkrebs, Brustkrebs, Prostatakrebs und Nierenzellkrebs (renal cell cancer).

**2.** Verfahren gemäß Anspruch 1, wobei der Analyseschritt ein Schritt des Analysierens des Vorliegens oder Nichtvorliegens von Methylierung wenigstens einer CpG-Stelle ist.

**3.** Verfahren gemäß Anspruch 2, wobei
die Information über die mehreren Typen von Krebs Information bezüglich des Vorliegens oder Nichtvorliegens von Krebszellen ist; und
der Schritt des Erhaltens von Information ein Schritt des Erhaltens von Information ist, die darauf hinweist, dass Krebszellen, abgeleitet von irgendeinem der mehreren Typen von Krebs, in der biologischen Probe vorliegen, wenn das Analyseergebnis erhalten wird, das darauf hinweist, dass eine methylierte CpG-Stelle vorliegt.

**4.** Verfahren gemäß Anspruch 1, wobei der Analyseschritt ein Schritt des Analysierens der Methylierungsfrequenz ist.

**5.** Verfahren gemäß Anspruch 4, wobei
die Information über die mehreren Typen von Krebs Information bezüglich des Vorliegens oder Nichtvorliegens von Krebszellen ist; und
der Schritt des Erhaltens von Information ein Schritt des Erhaltens von Information ist, die darauf hinweist, dass Krebszellen, abgeleitet von irgendeinem der mehreren Typen von Krebs, in der biologischen Probe vorliegen, wenn die in dem Analyseschritt erhaltene Methylierungsfrequenz höher als ein vorbestimmter Schwellwert ist oder gleich dem Schwellwert ist.

**6.** Verfahren gemäß irgendeinem der Ansprüche 1 bis 5, wobei
die mehreren Typen von Krebs zwei oder mehr Krebsarten sind, ausgewählt aus einer Gruppe, die besteht aus Gehirntumor, hepatozellulärem Krebs, Darmkrebs, Magenkrebs, Gebärmutterkrebs (uterus body cancer), Lungenkrebs, Brustkrebs, Prostatakrebs und Nierenzellkrebs (renal cell cancer), und
die Information über die mehreren Typen von Krebs Information ist, die darauf hinweist, dass Krebszellen, abgeleitet von irgendeinem der mehreren Typen von Krebs, in der biologischen Probe vorliegen.

**7.** Verfahren gemäß irgendeinem der Ansprüche 1 bis 6, wobei der Analyseschritt ein Schritt des Analysierens des Methylierungsstatus einer CpG-Stelle in einer Region des GDF7-Gens mit der Basensequenz SEQ ID NO: 37 in der DNA-Probe, erhalten in dem Herstellungsschritt, ist.

**8.** *In vitro*-Verwendung eines Markers zum Erhalten von Information über mehrere Typen von Krebs durch Methylierungsanalyse, wobei der Marker wenigstens eine CpG-Stelle ist, die in einer Promotorregion von GDF7 liegt.

**9.** *In vitro*-Verwendung des Markers gemäß Anspruch 8, wobei der Marker ein Polynukleotid ist, das erhalten wird, indem eine isolierte DNA Bisulfitbehandlung unterzogen wird, wobei die isolierte DNA eine zusammenhängende Basensequenz in einer ganzen oder partiellen Promotorregion von GDF7 hat und wenigstens eine CpG-Stelle in der Promotorregion und wenigstens ein Cytosin, das nicht in CpG-Stellen eingeschlossen ist, enthält.

**10.** Bestimmungsvorrichtung zum Erhalten von Information über mehrere Typen von Krebs, umfassend
einen Computer, einschließlich eines Prozessors und eines Speichers, wobei der Speicher durch den Prozessor gesteuert wird, wobei
der Speicher mit einem Computerprogramm aufgezeichnet wird, das den Computer dazu bringt, die Schritte von Anspruch 1 und den Schritt des Ausgebens eines Bestimmungsergebnisses auszuführen.

**11.** Bestimmungsvorrichtung gemäß Anspruch 10, wobei das Analyseergebnis das Vorliegen oder Nichtvorliegen von Methylierung an wenigstens einer CpG-Stelle ist.

**12.** Bestimmungsvorrichtung gemäß Anspruch 11, wobei
die Information über die mehreren Typen von Krebs Information bezüglich des Vorliegens oder Nichtvorliegens von Krebszellen ist; und
der Schritt des Ausgebens von Information der Schritt des Ausgebens von Information ist, die darauf hinweist, dass Krebszellen, abgeleitet von irgendeinem der mehreren Typen von Krebs, in der biologischen Probe vorliegen, wenn das Analyseergebnis darauf hinweist, dass eine methylierte CpG-Stelle vorliegt.

**13.** Bestimmungsvorrichtung gemäß den Ansprüchen 10 bis 12, wobei das Analyseergebnis Methylierungsfrequenz ist.

**14.** Bestimmungsvorrichtung gemäß den Ansprüchen 10 bis 13, weiterhin umfassend eine Messvorrichtung, die eine DNA-Probe aus einer biologischen Probe, gewonnen von einem Subjekt, misst, wobei die Messvorrichtung mit dem Computer verbunden ist.

**15.** Bestimmungsvorrichtung gemäß Anspruch 14, wobei die Messvorrichtung ein Massenspektrometer oder ein Fluoreszenzbildscanner ist.

## Revendications

1. Procédé d'obtention d'informations sur plusieurs types de cancers comprenant les étapes suivantes :

    la préparation d'un échantillon d'ADN à partir d'un échantillon biologique prélevé sur un sujet ;
    l'analyse de l'état de méthylation d'un site CpG dans une région de promoteur du gène GDF7 dans l'échantillon d'ADN obtenu dans l'étape de préparation ; et
    l'obtention d'informations sur plusieurs types de cancers chez le sujet sur la base d'un résultat d'analyse obtenu dans l'étape d'analyse ;
    dans lequel les plusieurs types de cancers sont deux ou plus de deux cancers sélectionnés dans un groupe constitué d'une tumeur cérébrale, d'un cancer hépatocellulaire, d'un cancer du côlon, d'un cancer gastrique, d'un cancer du corps utérin, d'un cancer du poumon, d'un cancer du sein, d'un cancer de la prostate, et d'un cancer des cellules rénales.

2. Procédé selon la revendication 1, dans lequel l'étape d'analyse est une étape d'analyse de la présence ou de l'absence de méthylation d'au moins un site CpG.

3. Procédé selon la revendication 2, dans lequel
    les informations sur les plusieurs types de cancers sont des informations relatives à la présence ou à l'absence de cellules cancéreuses ; et
    l'étape d'obtention d'informations est une étape d'obtention d'informations indiquant que des cellules cancéreuses dérivées de l'un quelconque des plusieurs types de cancers existent dans l'échantillon biologique quand le résultat d'analyse indiquant qu'un site CpG méthylé est présent est obtenu.

4. Procédé selon la revendication 1, dans lequel l'étape d'analyse est une étape d'analyse de fréquence de méthylation.

5. Procédé selon la revendication 4, dans lequel
    les informations sur les plusieurs types de cancers sont des informations relatives à la présence ou à l'absence de cellules cancéreuses ; et
    l'étape d'obtention d'informations est une étape d'obtention d'informations indiquant que des cellules cancéreuses dérivées de l'un quelconque des plusieurs types de cancers existent dans l'échantillon biologique quand la fréquence de méthylation obtenue dans l'étape d'analyse est supérieure à une valeur seuil prédéterminée ou est identique à la valeur seuil.

6. Procédé selon l'une quelconque des revendications 1 à 5, dans lequel
    les plusieurs types de cancers sont deux ou plus de deux cancers sélectionnés dans un groupe constitué d'une tumeur cérébrale, d'un cancer hépatocellulaire, d'un cancer du côlon, d'un cancer gastrique, d'un cancer du corps utérin, d'un cancer du poumon, d'un cancer du sein, d'un cancer de la prostate, et d'un cancer des cellules rénales, et les informations sur les plusieurs types de cancers sont des informations indiquant que des cellules cancéreuses dérivées de l'un quelconque des plusieurs types de cancers existent dans l'échantillon biologique.

7. Procédé selon l'une quelconque des revendications 1 à 6, dans lequel l'étape d'analyse est une étape d'analyse de l'état de méthylation d'un site CpG dans une région du gène GDF7 ayant la séquence de bases SEQ ID NO : 37 dans l'échantillon d'ADN obtenu dans l'étape de préparation.

8. Utilisation in vitro d'un marqueur pour obtenir des informations sur plusieurs types de cancers par une analyse de méthylation, ledit marqueur est au moins un site CpG situé dans une région de promoteur de GDF7.

9. Utilisation in vitro du marqueur selon la revendication 8, dans lequel le marqueur est un polynucléotide obtenu en soumettant un ADN isolé à un traitement au bisulfite, l'ADN isolé ayant une séquence de bases contiguës dans une région de promoteur entière ou partielle de GDF7 et contenant au moins un site CpG dans la région de promoteur et au moins une cytosine non incluse dans les sites CpG.

10. Dispositif de détermination pour obtenir des informations sur plusieurs types de cancers comprenant
    un ordinateur comprenant un processeur et une mémoire, la mémoire étant commandée par le processeur dans lequel
    la mémoire est enregistrée avec un programme informatique pour amener l'ordinateur à exécuter les étapes de la revendication 1 et l'étape de sortie d'un résultat de détermination.

**11.** Dispositif de détermination selon la revendication 10, dans lequel le résultat d'analyse est la présence ou l'absence de méthylation d'au moins un site CpG.

**12.** Dispositif de détermination selon la revendication 11, dans lequel
les informations sur les plusieurs types de cancers sont des informations relatives à la présence ou à l'absence de cellules cancéreuses ; et
l'étape de sortie d'informations est l'étape de sortie d'informations indiquant que des cellules cancéreuses dérivées de l'un quelconque des plusieurs types de cancers existent dans l'échantillon biologique quand le résultat d'analyse indique la présence d'un site CpG méthylé.

**13.** Dispositif de détermination selon les revendications 10 à 12, dans lequel le résultat d'analyse est une fréquence de méthylation.

**14.** Dispositif de détermination selon les revendications 10 à 13 comprenant en outre un dispositif de mesure qui mesure un échantillon d'ADN provenant d'un échantillon biologique prélevé sur un sujet, dans lequel le dispositif de mesure est connecté à l'ordinateur.

**15.** Dispositif de détermination selon la revendication 14, dans lequel le dispositif de mesure est un spectromètre de masse ou un dispositif de balayage d'image de fluorescence.

FIG. 1A

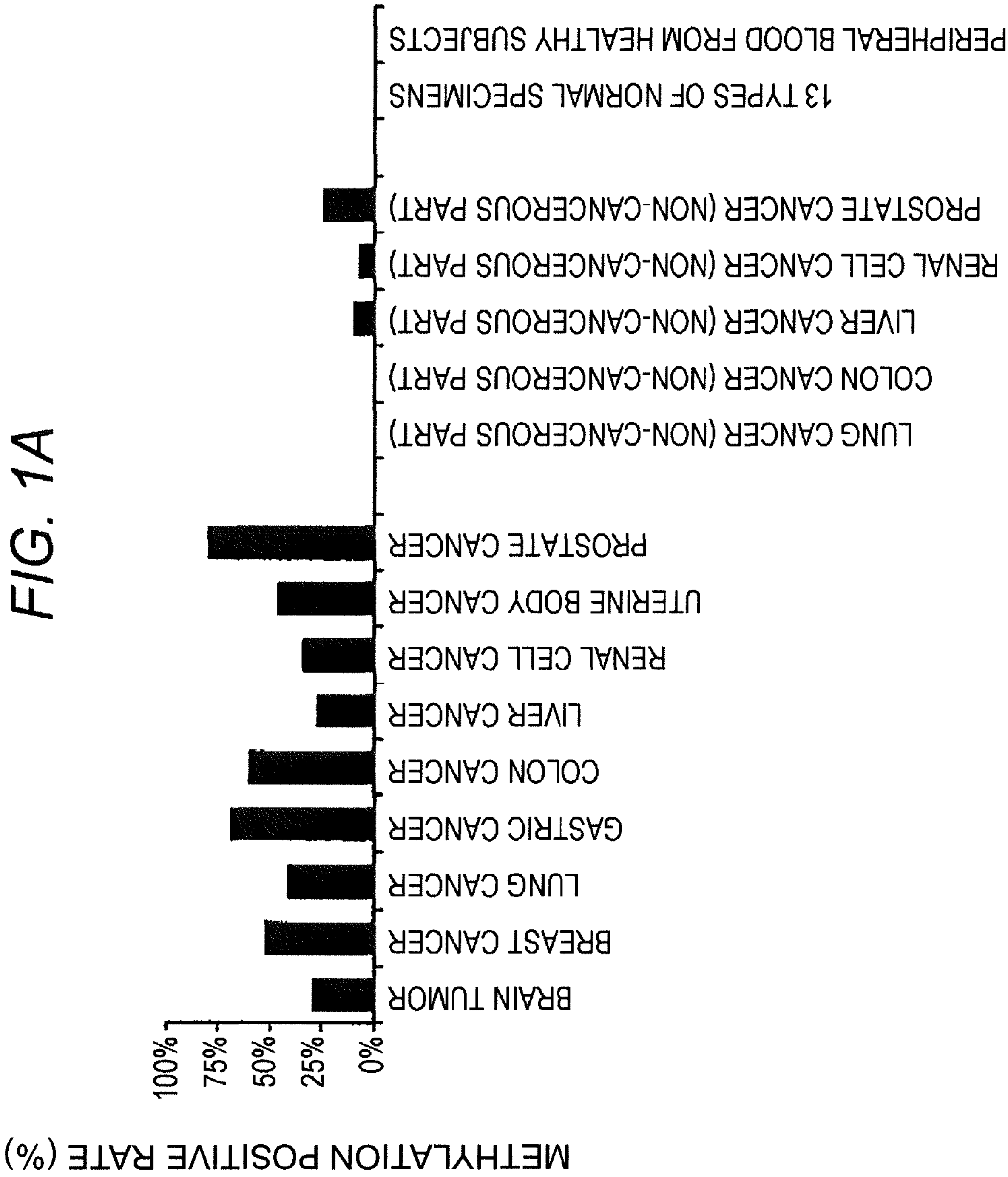
PERIPHERAL BLOOD FROM HEALTHY SUBJECTS
13 TYPES OF NORMAL SPECIMENS
PROSTATE CANCER (NON-CANCEROUS PART)
RENAL CELL CANCER (NON-CANCEROUS PART)
LIVER CANCER (NON-CANCEROUS PART)
COLON CANCER (NON-CANCEROUS PART)
LUNG CANCER (NON-CANCEROUS PART)
PROSTATE CANCER
UTERINE BODY CANCER
RENAL CELL CANCER
LIVER CANCER
COLON CANCER
GASTRIC CANCER
LUNG CANCER
BREAST CANCER
BRAIN TUMOR
100%
75%
50%
25%
0%
METHYLATION POSITIVE RATE (%)

FIG. 1B

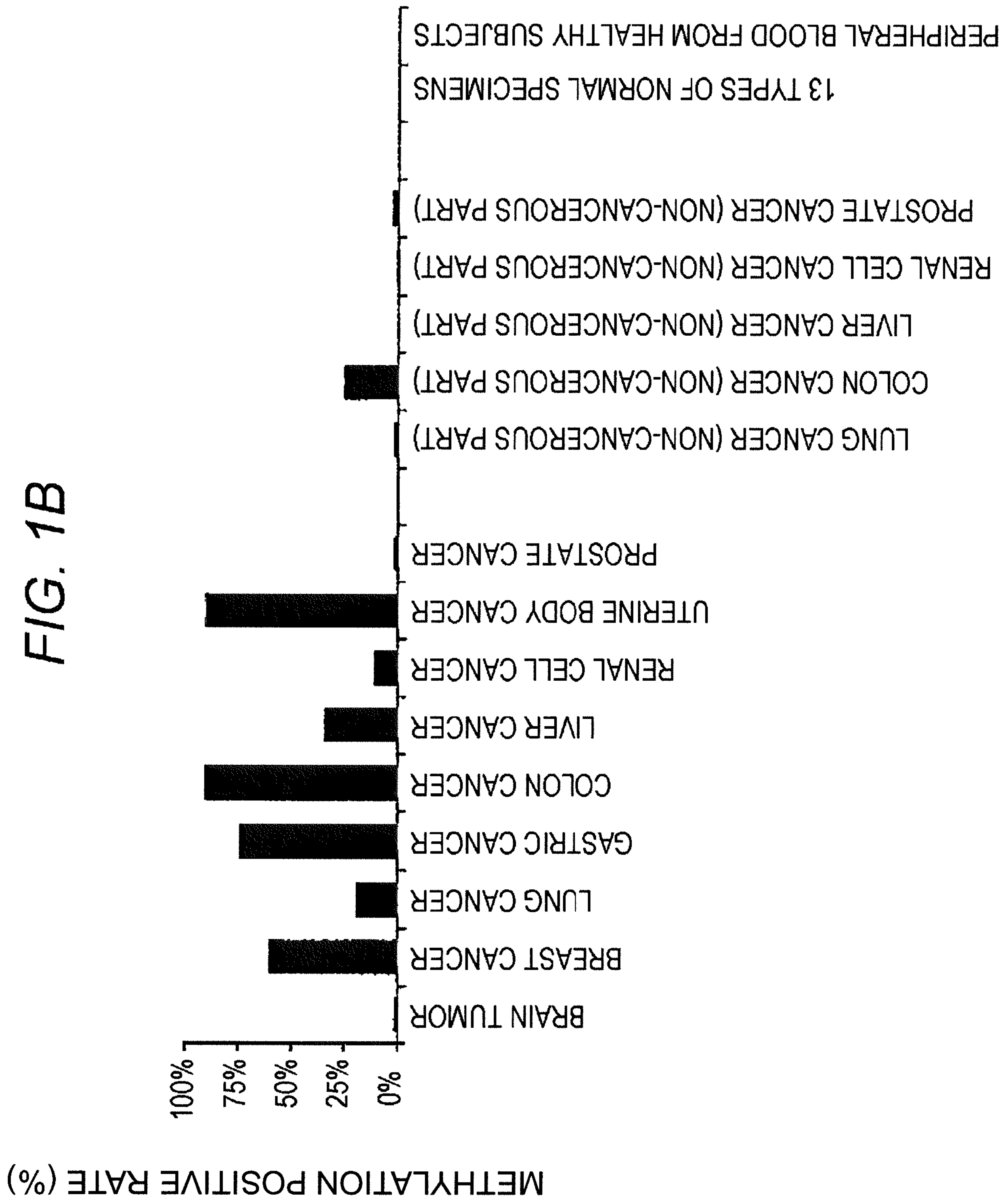
METHYLATION POSITIVE RATE (%)
100%
75%
50%
25%
0%
BRAIN TUMOR
BREAST CANCER
LUNG CANCER
GASTRIC CANCER
COLON CANCER
LIVER CANCER
RENAL CELL CANCER
UTERINE BODY CANCER
PROSTATE CANCER
LUNG CANCER (NON-CANCEROUS PART)
COLON CANCER (NON-CANCEROUS PART)
LIVER CANCER (NON-CANCEROUS PART)
RENAL CELL CANCER (NON-CANCEROUS PART)
PROSTATE CANCER (NON-CANCEROUS PART)
13 TYPES OF NORMAL SPECIMENS
PERIPHERAL BLOOD FROM HEALTHY SUBJECTS

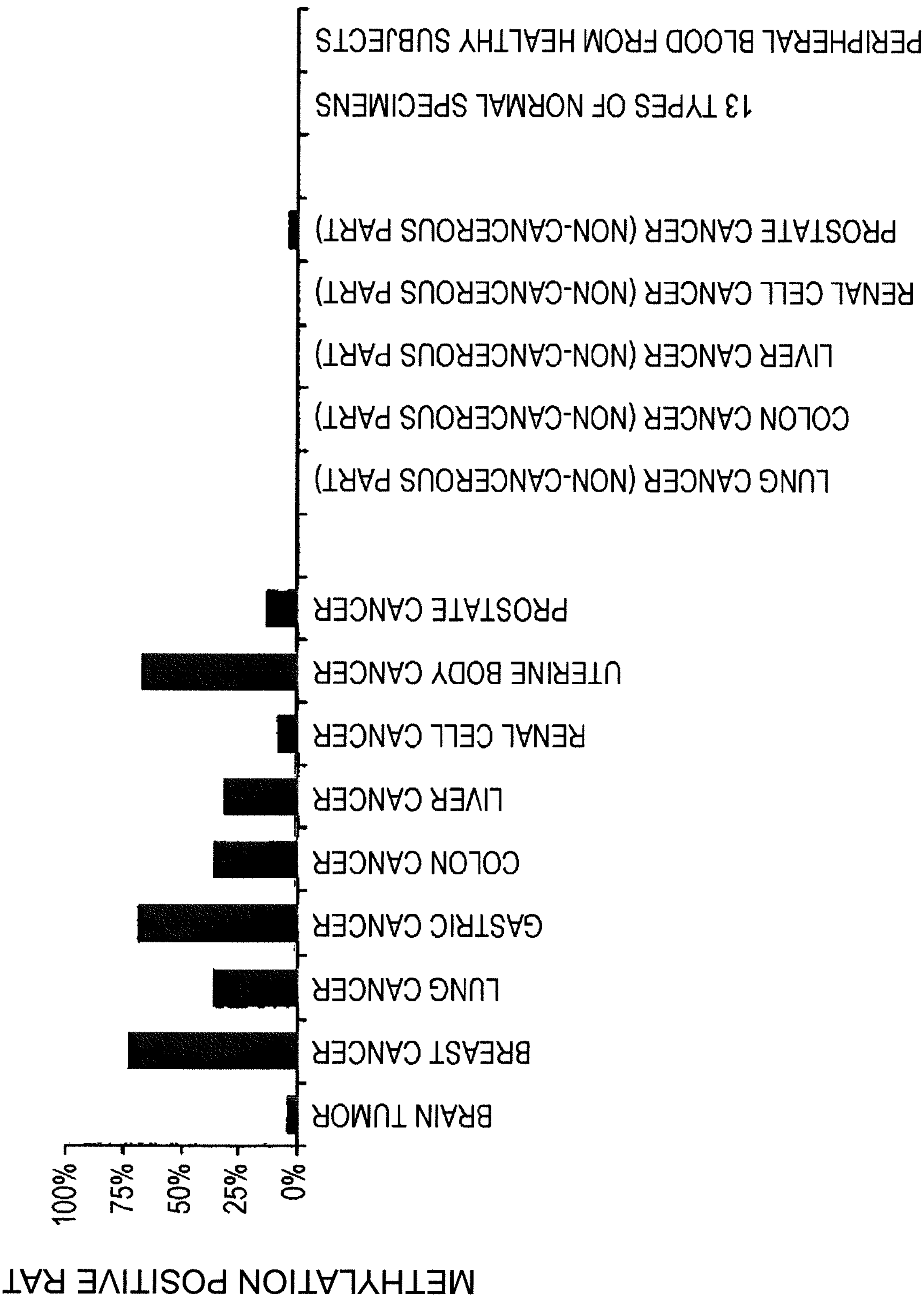
METHYLATION POSITIVE RATE (%)
0%
25%
50%
75%
100%
BRAIN TUMOR
BREAST CANCER
LUNG CANCER
GASTRIC CANCER
COLON CANCER
LIVER CANCER
RENAL CELL CANCER
UTERINE BODY CANCER
PROSTATE CANCER
LUNG CANCER (NON-CANCEROUS PART)
COLON CANCER (NON-CANCEROUS PART)
LIVER CANCER (NON-CANCEROUS PART)
RENAL CELL CANCER (NON-CANCEROUS PART)
PROSTATE CANCER (NON-CANCEROUS PART)
13 TYPES OF NORMAL SPECIMENS
PERIPHERAL BLOOD FROM HEALTHY SUBJECTS

FIG. 1C

FIG. 1D

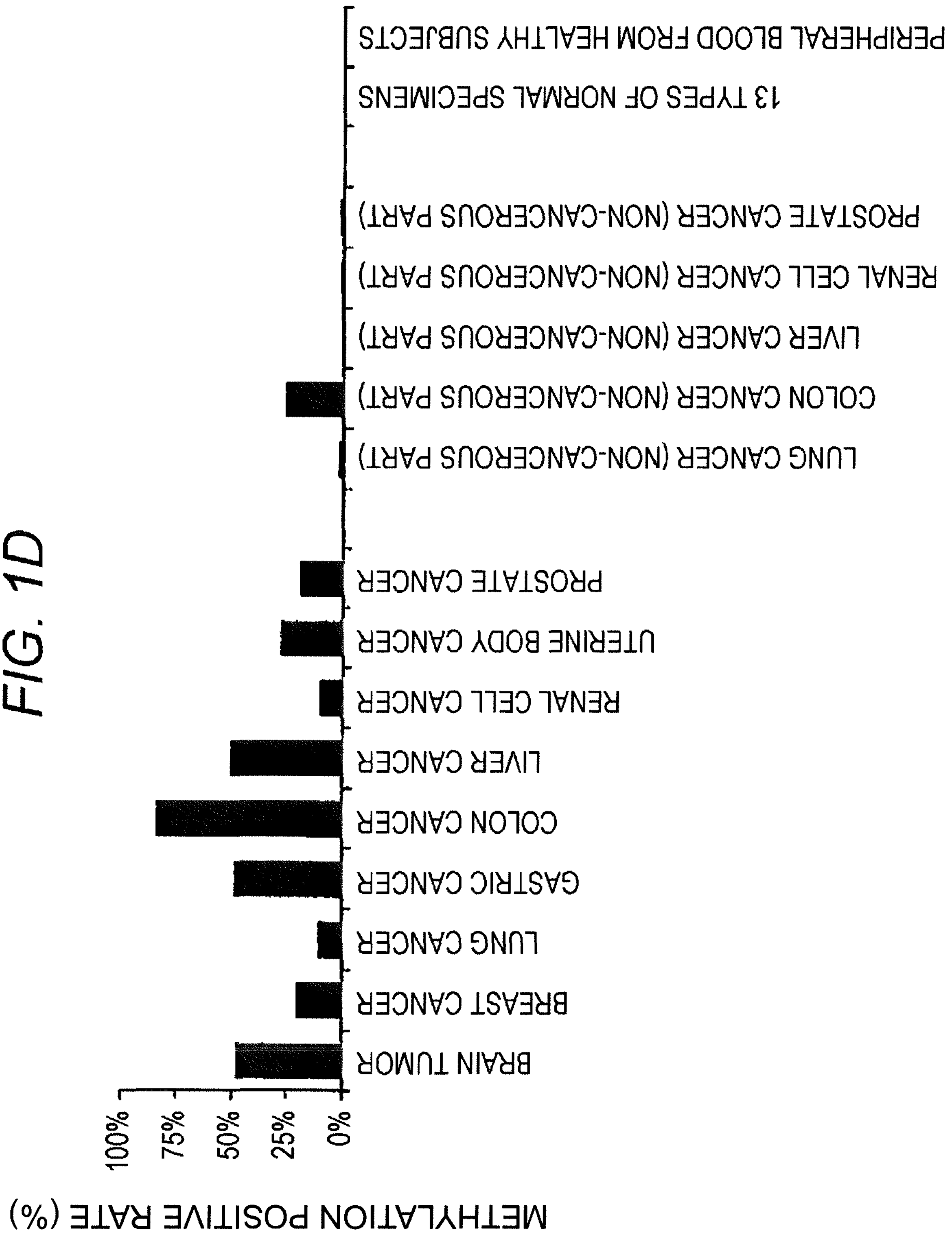
METHYLATION POSITIVE RATE (%)
100%
75%
50%
25%
0%
BRAIN TUMOR
BREAST CANCER
LUNG CANCER
GASTRIC CANCER
COLON CANCER
LIVER CANCER
RENAL CELL CANCER
UTERINE BODY CANCER
PROSTATE CANCER
LUNG CANCER (NON-CANCEROUS PART)
COLON CANCER (NON-CANCEROUS PART)
LIVER CANCER (NON-CANCEROUS PART)
RENAL CELL CANCER (NON-CANCEROUS PART)
PROSTATE CANCER (NON-CANCEROUS PART)
13 TYPES OF NORMAL SPECIMENS
PERIPHERAL BLOOD FROM HEALTHY SUBJECTS

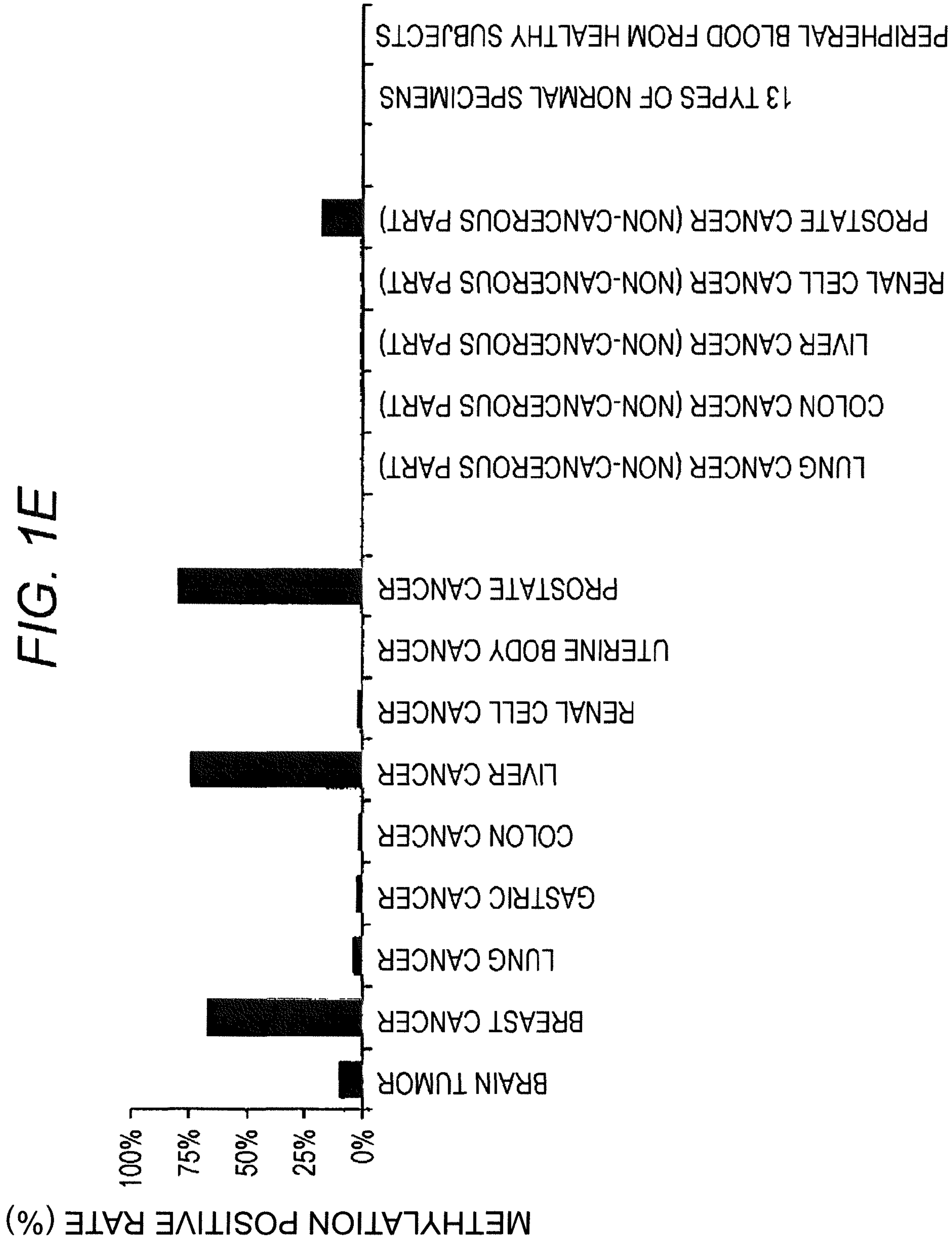
PERIPHERAL BLOOD FROM HEALTHY SUBJECTS
13 TYPES OF NORMAL SPECIMENS
PROSTATE CANCER (NON-CANCEROUS PART)
RENAL CELL CANCER (NON-CANCEROUS PART)
LIVER CANCER (NON-CANCEROUS PART)
COLON CANCER (NON-CANCEROUS PART)
LUNG CANCER (NON-CANCEROUS PART)
PROSTATE CANCER
UTERINE BODY CANCER
RENAL CELL CANCER
LIVER CANCER
COLON CANCER
GASTRIC CANCER
LUNG CANCER
BREAST CANCER
BRAIN TUMOR
100%
75%
50%
25%
0%
METHYLATION POSITIVE RATE (%)

FIG. 1E

FIG. 1F

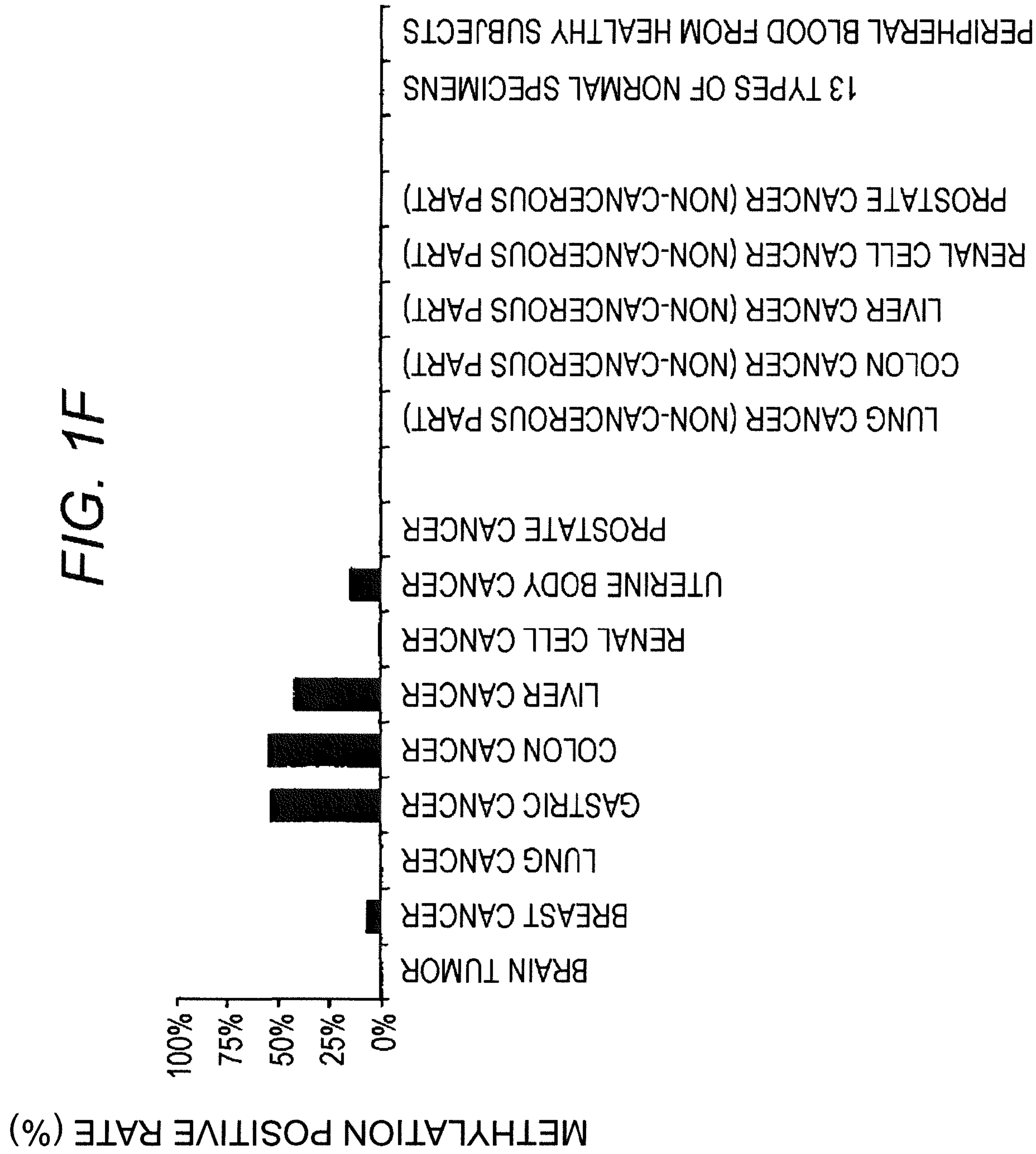

METHYLATION POSITIVE RATE (%)
100%
75%
50%
25%
0%
BRAIN TUMOR
BREAST CANCER
LUNG CANCER
GASTRIC CANCER
COLON CANCER
LIVER CANCER
RENAL CELL CANCER
UTERINE BODY CANCER
PROSTATE CANCER
LUNG CANCER (NON-CANCEROUS PART)
COLON CANCER (NON-CANCEROUS PART)
LIVER CANCER (NON-CANCEROUS PART)
RENAL CELL CANCER (NON-CANCEROUS PART)
PROSTATE CANCER (NON-CANCEROUS PART)
13 TYPES OF NORMAL SPECIMENS
PERIPHERAL BLOOD FROM HEALTHY SUBJECTS

FIG. 1G

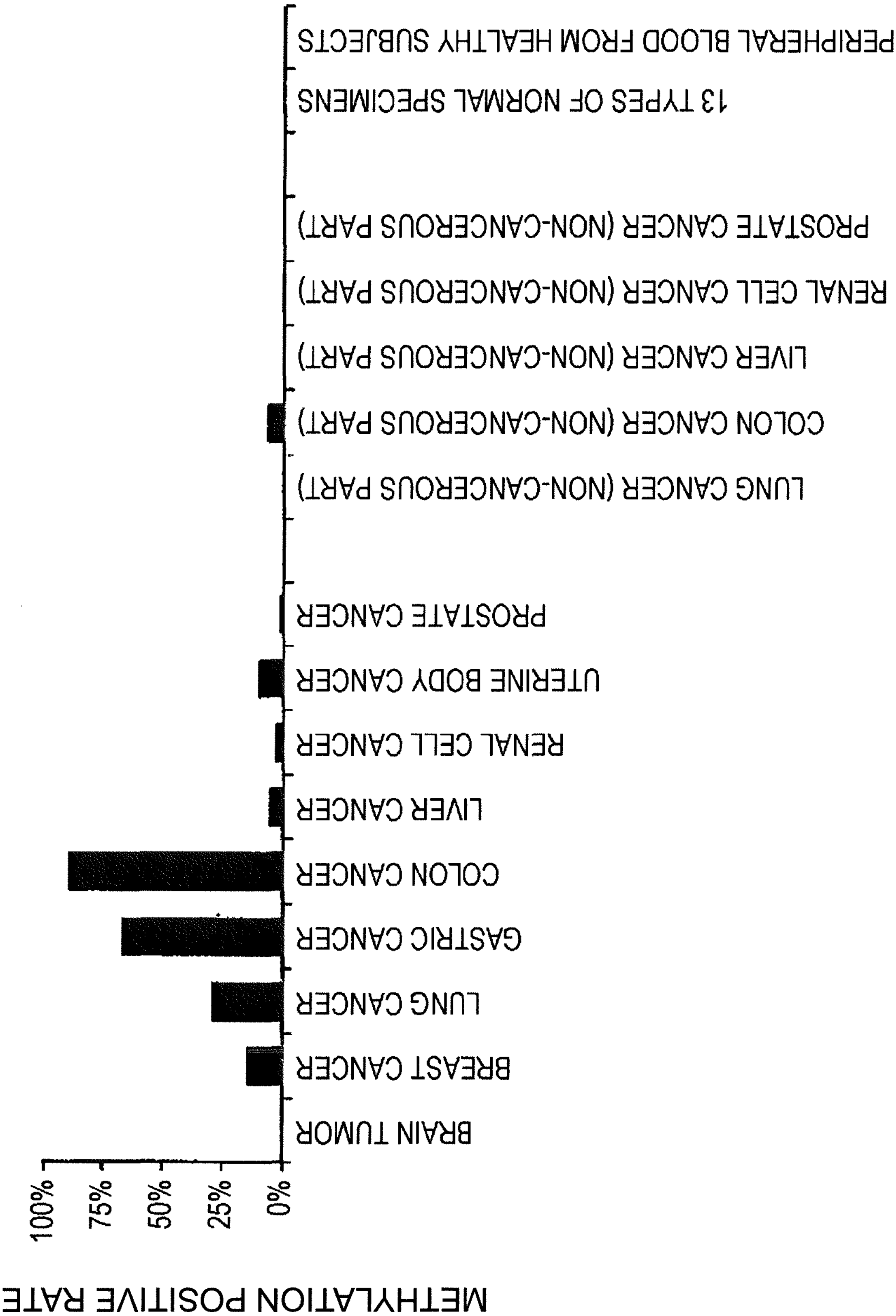
PERIPHERAL BLOOD FROM HEALTHY SUBJECTS
13 TYPES OF NORMAL SPECIMENS
PROSTATE CANCER (NON-CANCEROUS PART)
RENAL CELL CANCER (NON-CANCEROUS PART)
LIVER CANCER (NON-CANCEROUS PART)
COLON CANCER (NON-CANCEROUS PART)
LUNG CANCER (NON-CANCEROUS PART)
PROSTATE CANCER
UTERINE BODY CANCER
RENAL CELL CANCER
LIVER CANCER
COLON CANCER
GASTRIC CANCER
LUNG CANCER
BREAST CANCER
BRAIN TUMOR
100%
75%
50%
25%
0%
METHYLATION POSITIVE RATE (%)

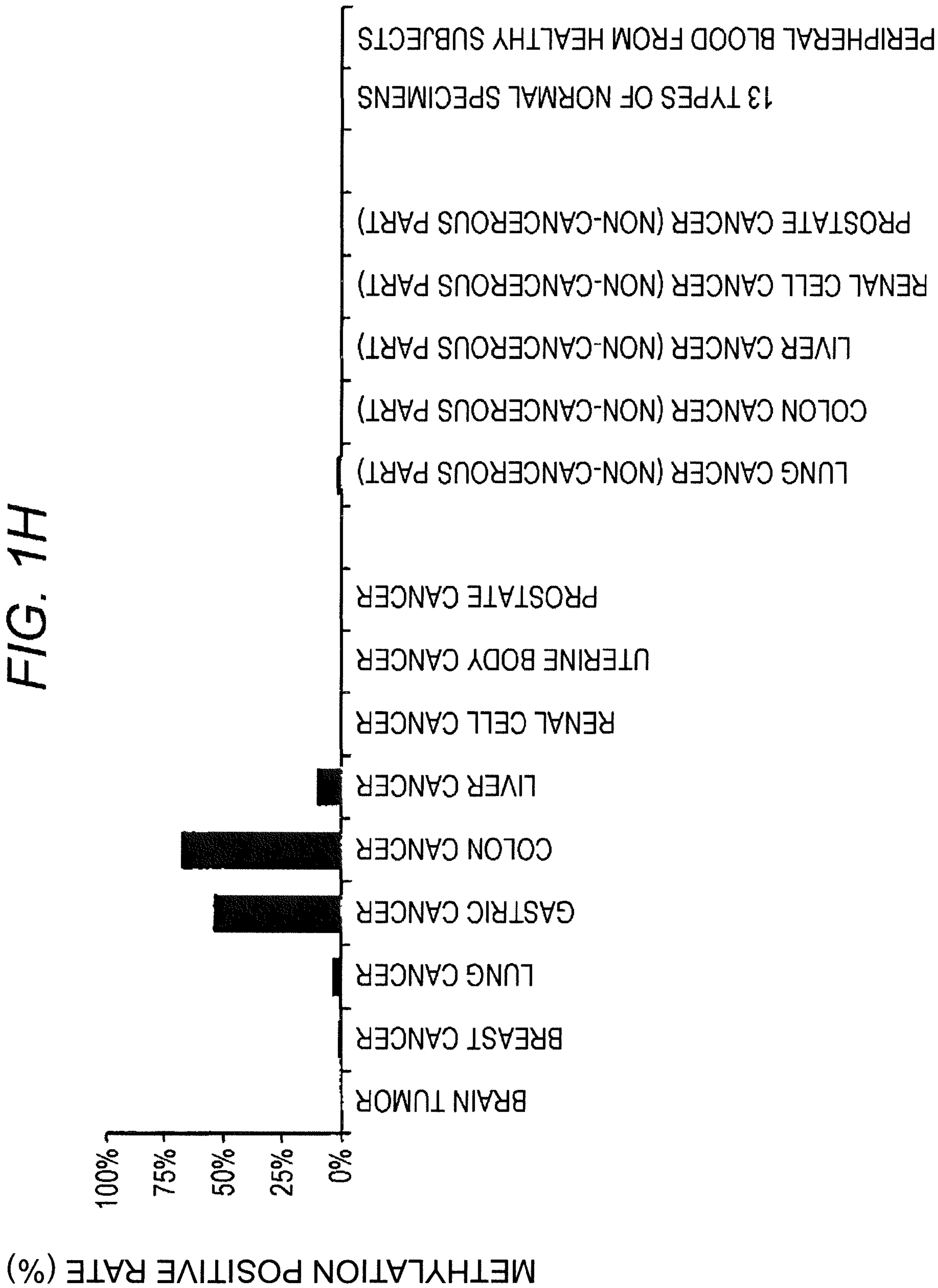
PERIPHERAL BLOOD FROM HEALTHY SUBJECTS
13 TYPES OF NORMAL SPECIMENS
PROSTATE CANCER (NON-CANCEROUS PART)
RENAL CELL CANCER (NON-CANCEROUS PART)
LIVER CANCER (NON-CANCEROUS PART)
COLON CANCER (NON-CANCEROUS PART)
LUNG CANCER (NON-CANCEROUS PART)
PROSTATE CANCER
UTERINE BODY CANCER
RENAL CELL CANCER
LIVER CANCER
COLON CANCER
GASTRIC CANCER
LUNG CANCER
BREAST CANCER
BRAIN TUMOR
100%
75%
50%
25%
0%
METHYLATION POSITIVE RATE (%)

FIG. 1H

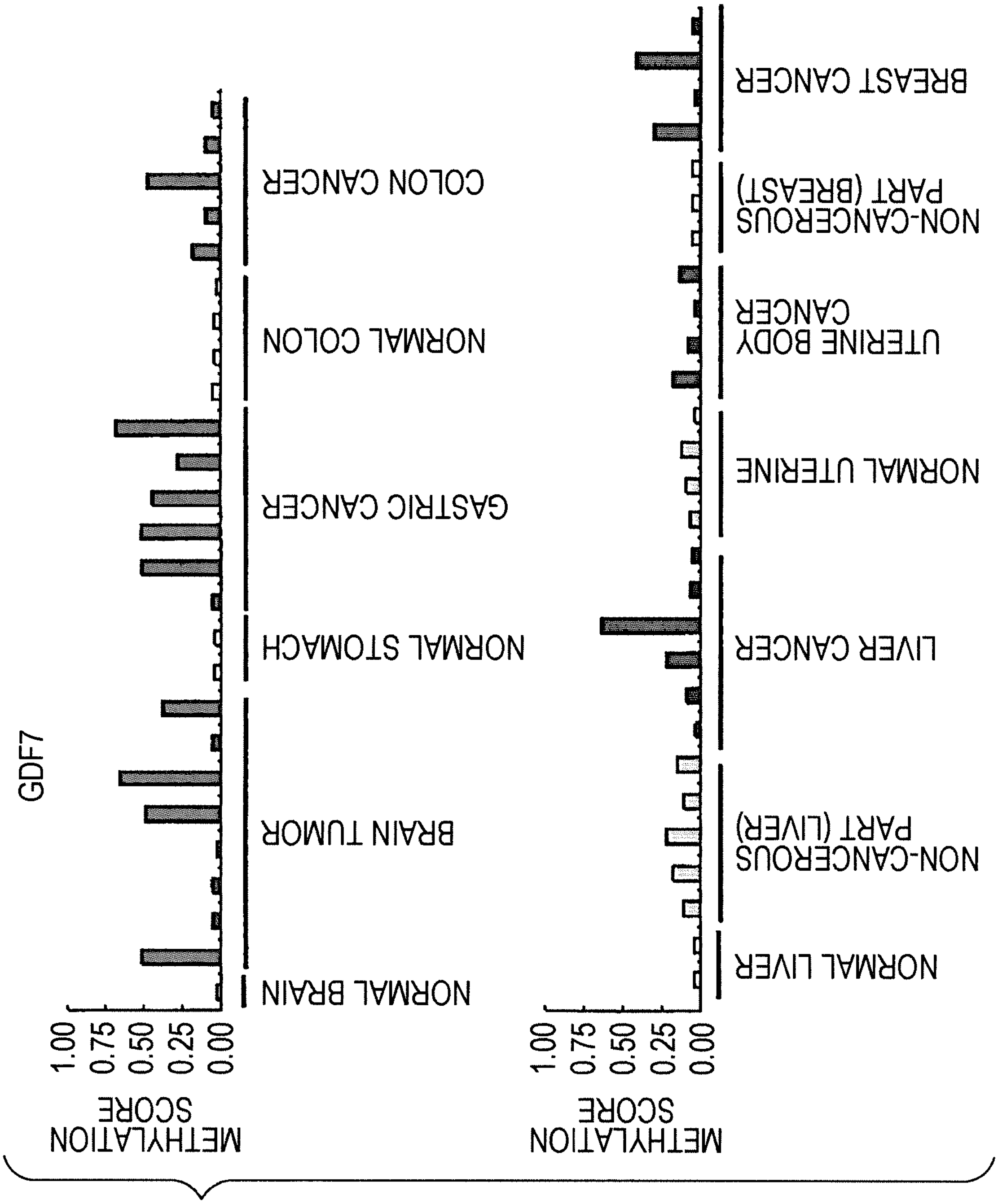
GDF7
METHYLATION SCORE
1.00
0.75
0.50
0.25
0.00
NORMAL BRAIN
BRAIN TUMOR
NORMAL STOMACH
GASTRIC CANCER
NORMAL COLON
COLON CANCER
METHYLATION SCORE
1.00
0.75
0.50
0.25
0.00
NORMAL LIVER
NON-CANCEROUS PART (LIVER)
LIVER CANCER
NORMAL UTERINE
UTERINE BODY CANCER
NON-CANCEROUS PART (BREAST)
BREAST CANCER

FIG. 2A

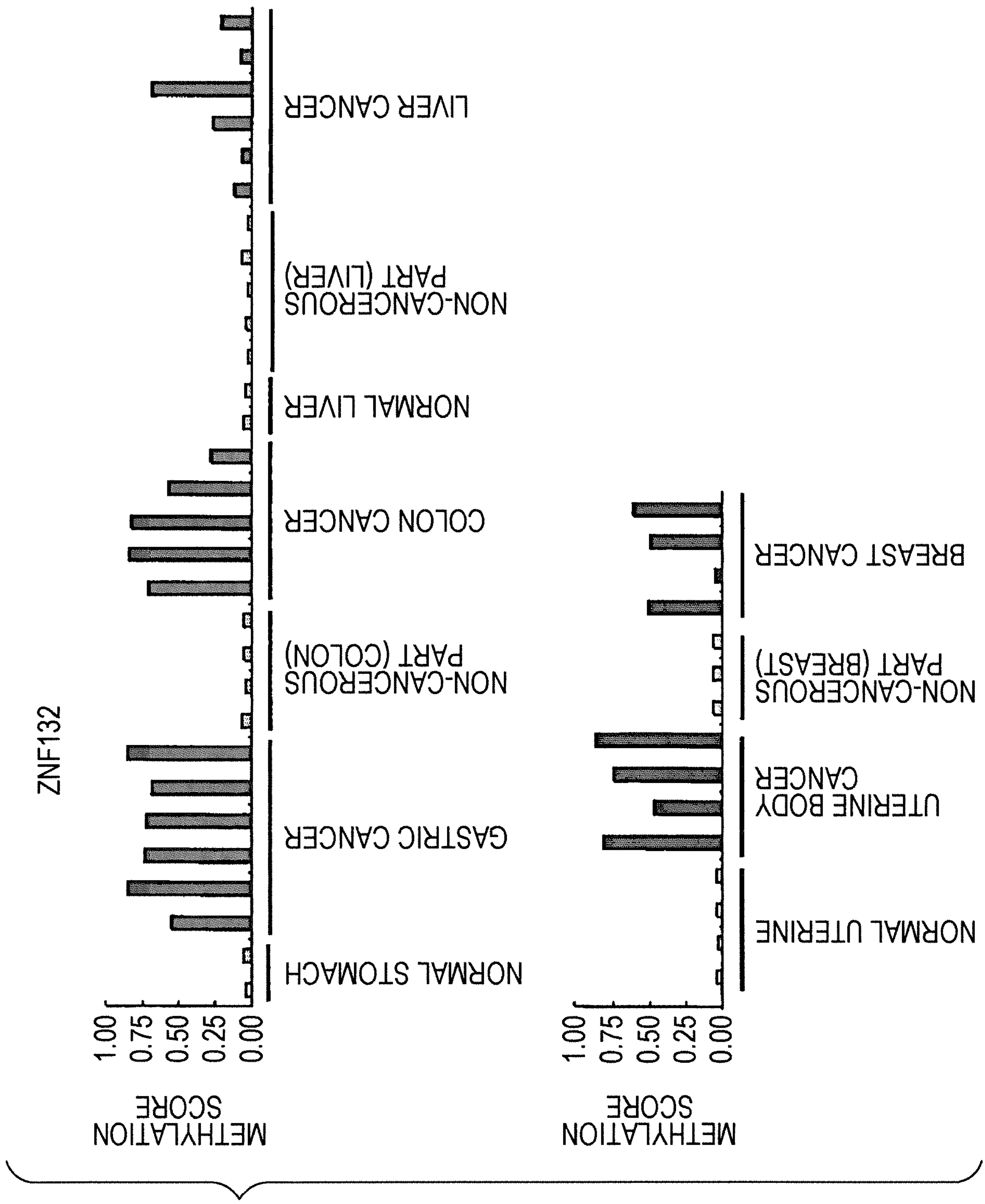
ZNF132
METHYLATION SCORE
1.00
0.75
0.50
0.25
0.00
NORMAL STOMACH
GASTRIC CANCER
NON-CANCEROUS PART (COLON)
COLON CANCER
NORMAL LIVER
NON-CANCEROUS PART (LIVER)
LIVER CANCER
METHYLATION SCORE
1.00
0.75
0.50
0.25
0.00
NORMAL UTERINE
UTERINE BODY CANCER
NON-CANCEROUS PART (BREAST)
BREAST CANCER

FIG. 2B

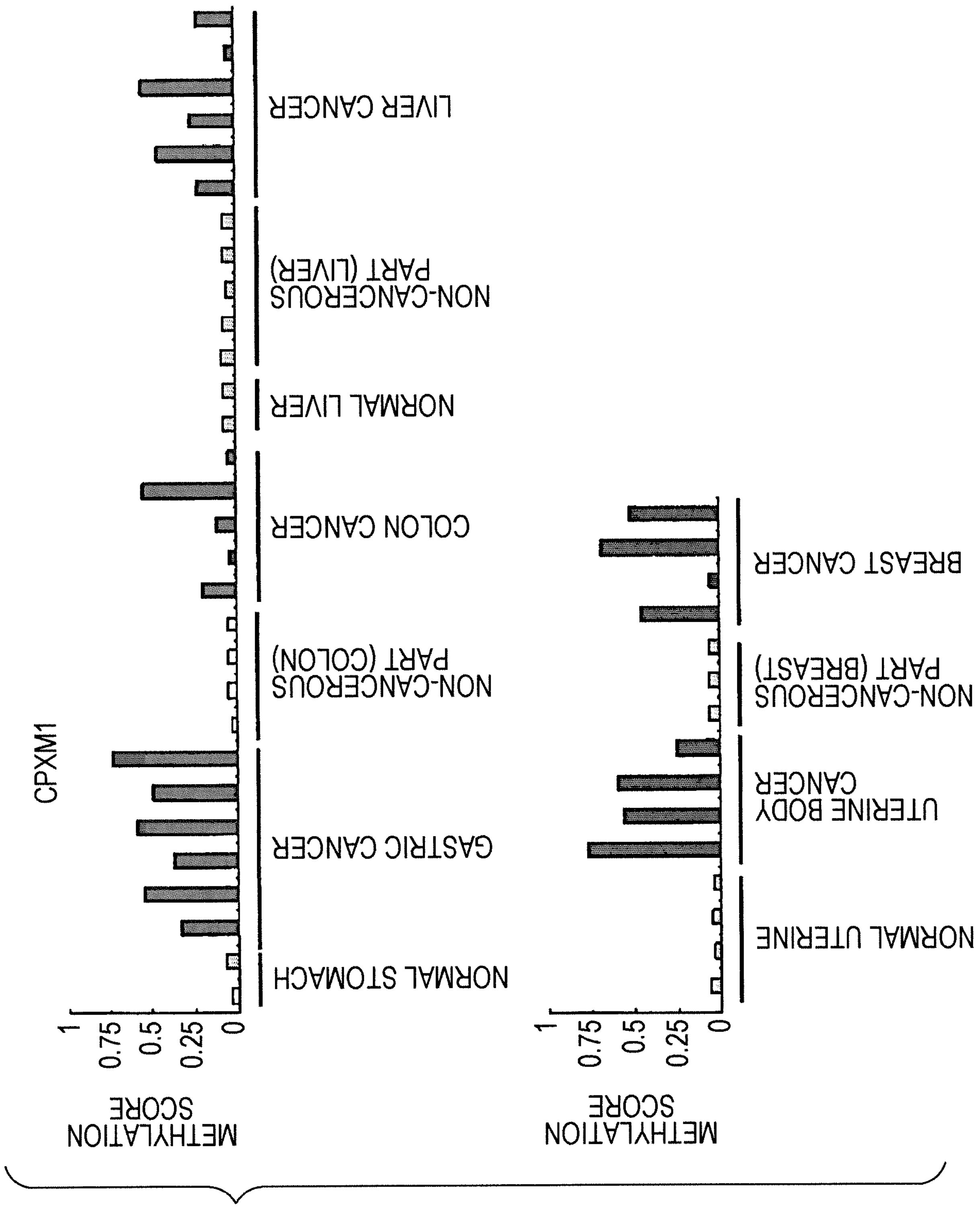
CPXM1
METHYLATION SCORE
0
0.25
0.5
0.75
1
NORMAL STOMACH
GASTRIC CANCER
NON-CANCEROUS PART (COLON)
COLON CANCER
NORMAL LIVER
NON-CANCEROUS PART (LIVER)
LIVER CANCER
METHYLATION SCORE
0
0.25
0.5
0.75
1
NORMAL UTERINE
UTERINE BODY CANCER
NON-CANCEROUS PART (BREAST)
BREAST CANCER

FIG. 2C

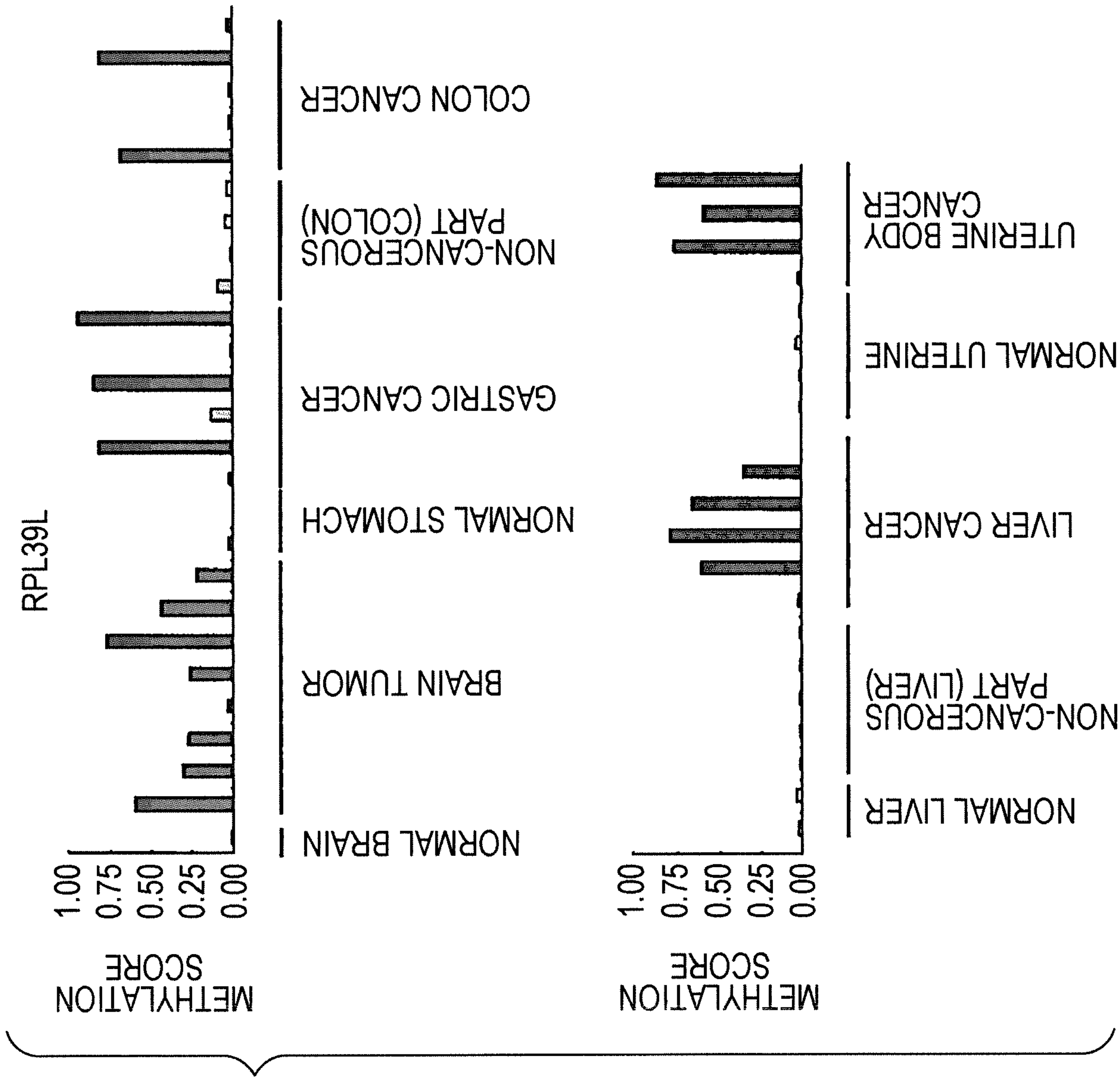
RPL39L
METHYLATION SCORE
1.00
0.75
0.50
0.25
0.00
NORMAL BRAIN
BRAIN TUMOR
NORMAL STOMACH
GASTRIC CANCER
NON-CANCEROUS PART (COLON)
COLON CANCER
METHYLATION SCORE
1.00
0.75
0.50
0.25
0.00
NORMAL LIVER
NON-CANCEROUS PART (LIVER)
LIVER CANCER
NORMAL UTERINE
UTERINE BODY CANCER

FIG. 2D

FIG. 2E

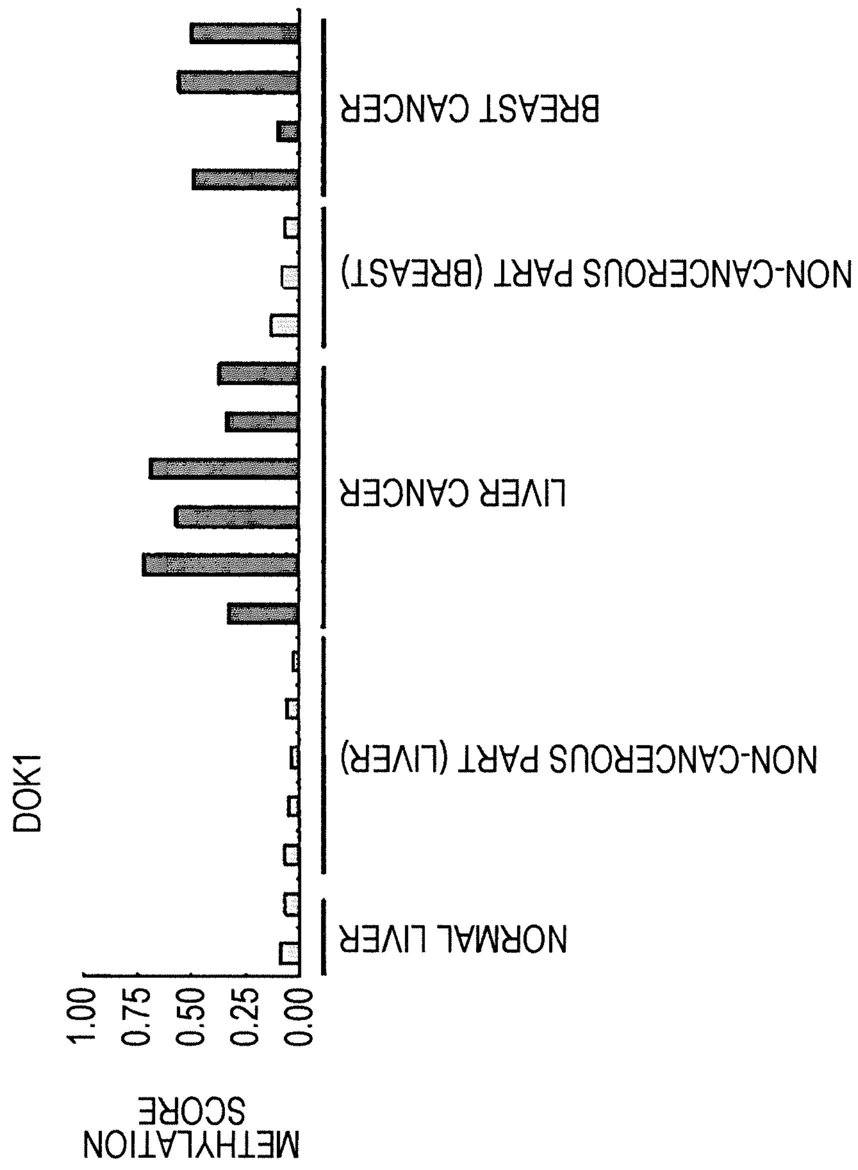
DOK1
METHYLATION SCORE
1.00
0.75
0.50
0.25
0.00
NORMAL LIVER
NON-CANCEROUS PART (LIVER)
LIVER CANCER
NON-CANCEROUS PART (BREAST)
BREAST CANCER

FIG. 2F

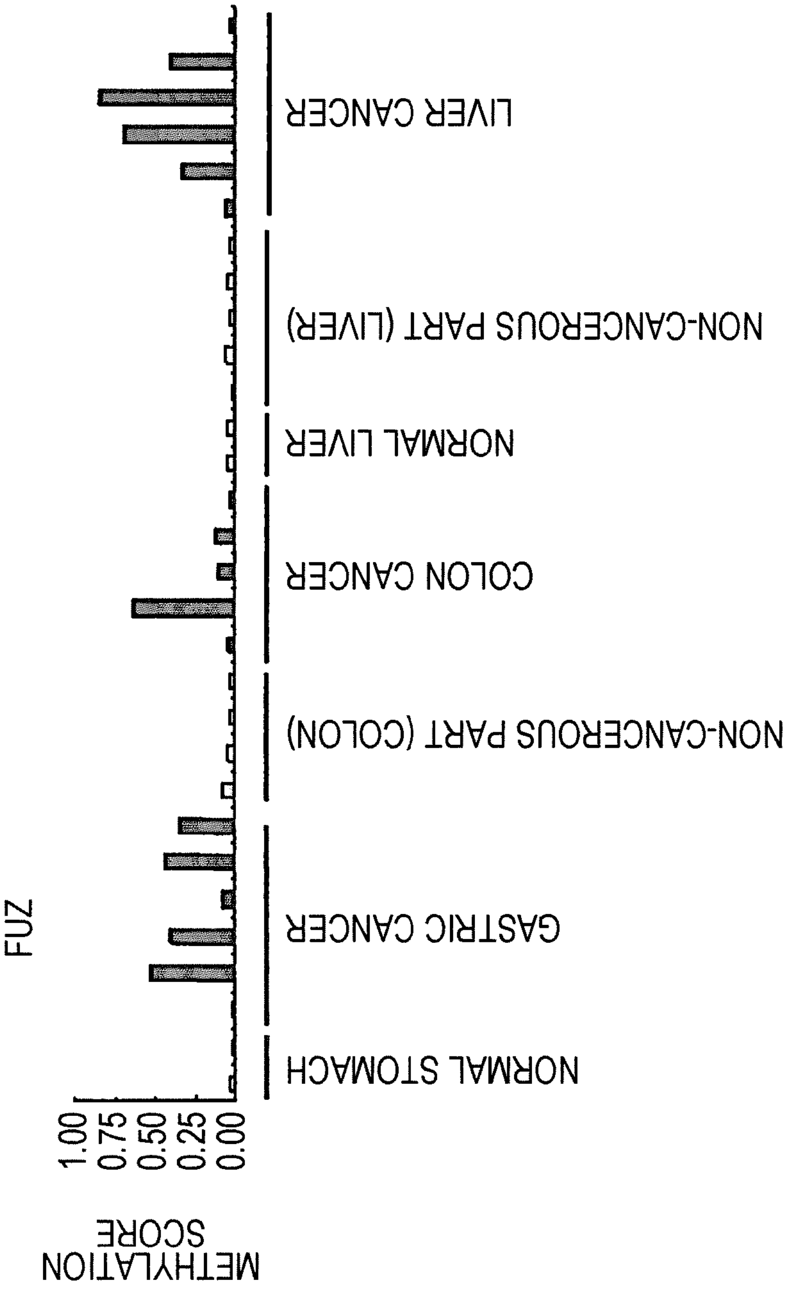
FUZ
METHYLATION SCORE
1.00
0.75
0.50
0.25
0.00
NORMAL STOMACH
GASTRIC CANCER
NON-CANCEROUS PART (COLON)
COLON CANCER
NORMAL LIVER
NON-CANCEROUS PART (LIVER)
LIVER CANCER

FIG. 2G

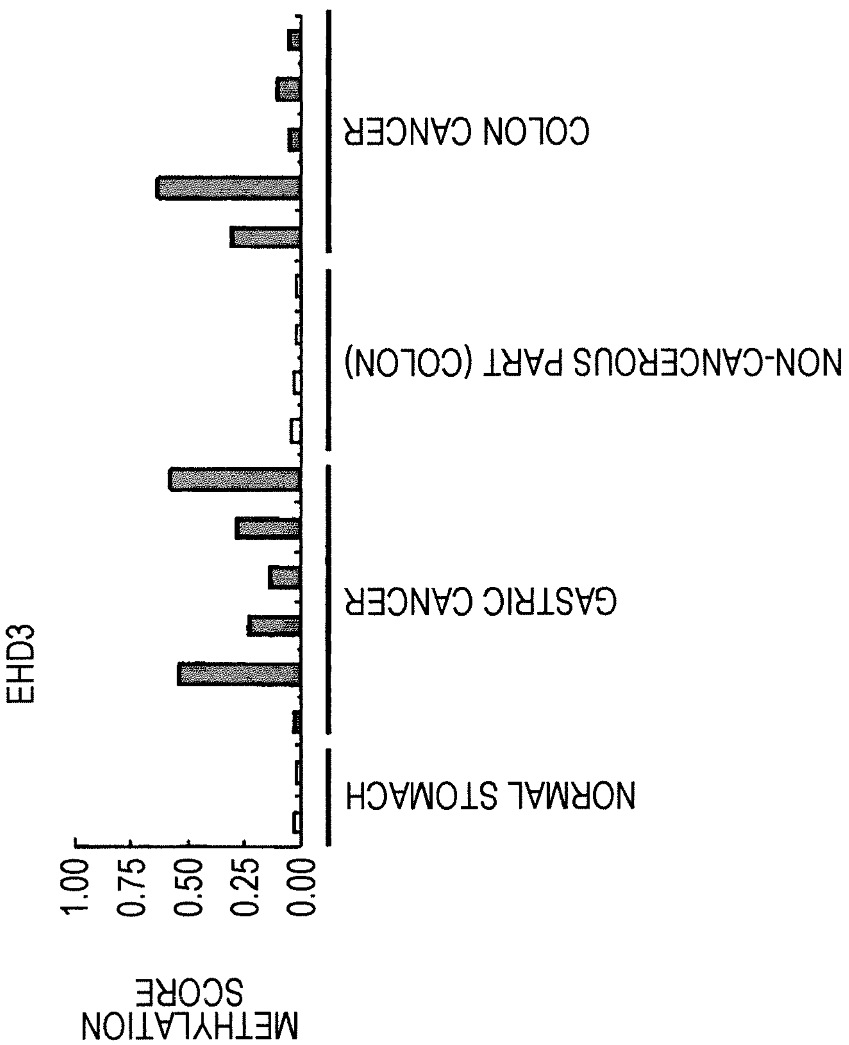
EHD3
METHYLATION SCORE
1.00
0.75
0.50
0.25
0.00
NORMAL STOMACH
GASTRIC CANCER
NON-CANCEROUS PART (COLON)
COLON CANCER

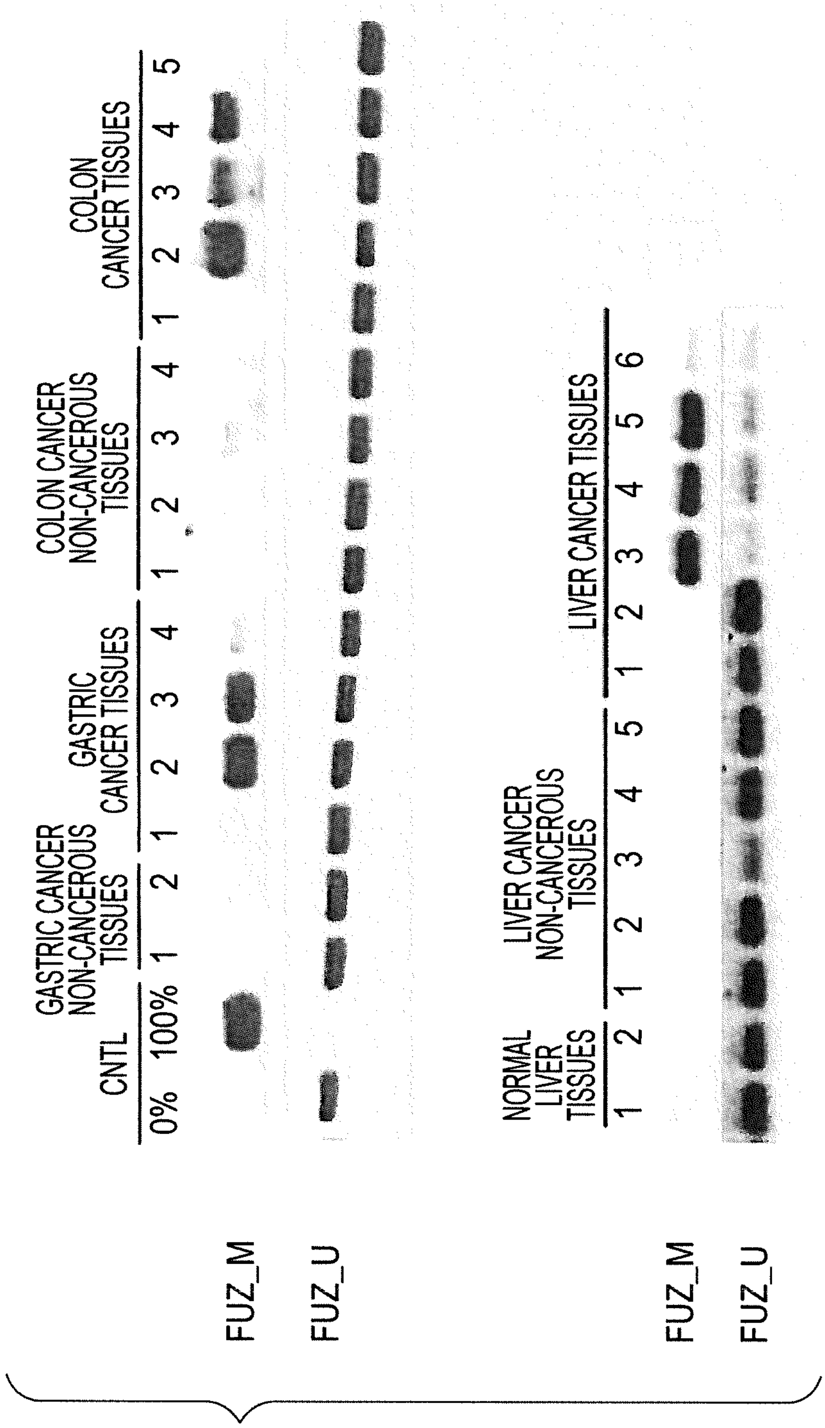
CNTL
0% 100%
GASTRIC CANCER NON-CANCEROUS TISSUES
1 2
GASTRIC CANCER TISSUES
1 2 3 4
COLON CANCER NON-CANCEROUS TISSUES
1 2 3 4
COLON CANCER TISSUES
1 2 3 4 5
FUZ_M
FUZ_U
NORMAL LIVER TISSUES
1 2
LIVER CANCER NON-CANCEROUS TISSUES
1 2 3 4 5
LIVER CANCER TISSUES
1 2 3 4 5 6
FUZ_M
FUZ_U

FIG. 3A

FIG. 3B

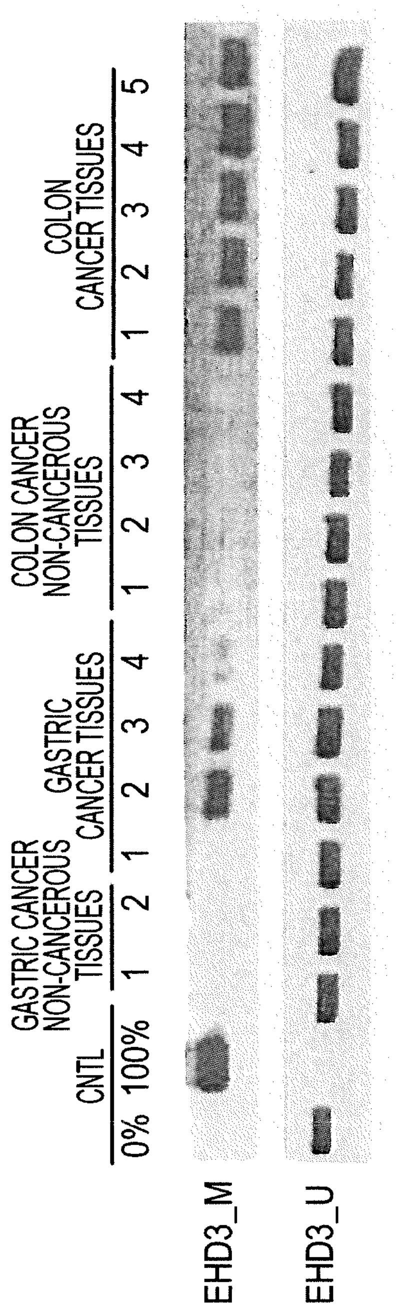
CNTL
0% 100%
GASTRIC CANCER NON-CANCEROUS TISSUES
1 2
GASTRIC CANCER TISSUES
1 2 3 4
COLON CANCER NON-CANCEROUS TISSUES
1 2 3 4
COLON CANCER TISSUES
1 2 3 4 5
EHD3_M
EHD3_U

FIG. 3C

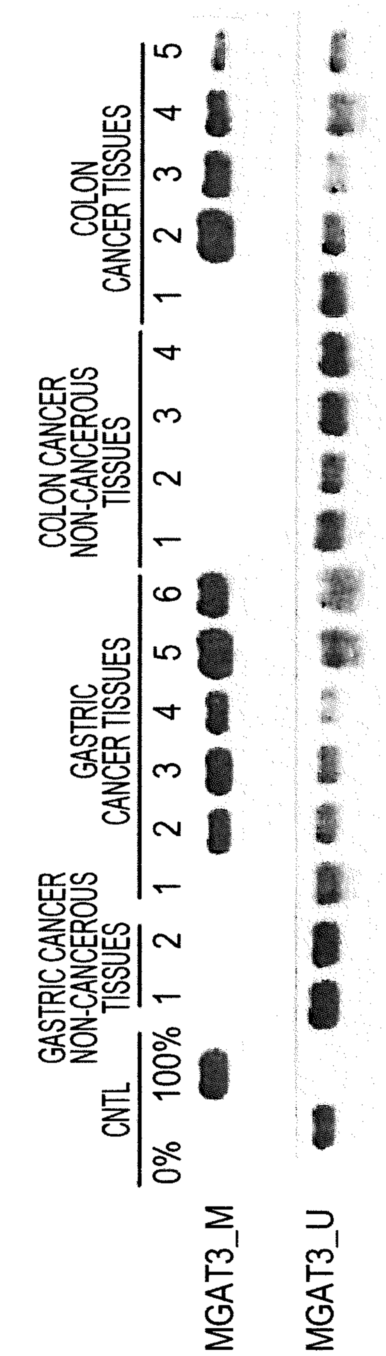
CNTL
0%
100%
GASTRIC CANCER NON-CANCEROUS TISSUES
1
2
GASTRIC CANCER TISSUES
1
2
3
4
5
6
COLON CANCER NON-CANCEROUS TISSUES
1
2
3
4
COLON CANCER TISSUES
1
2
3
4
5
MGAT3_M
MGAT3_U

FIG. 4

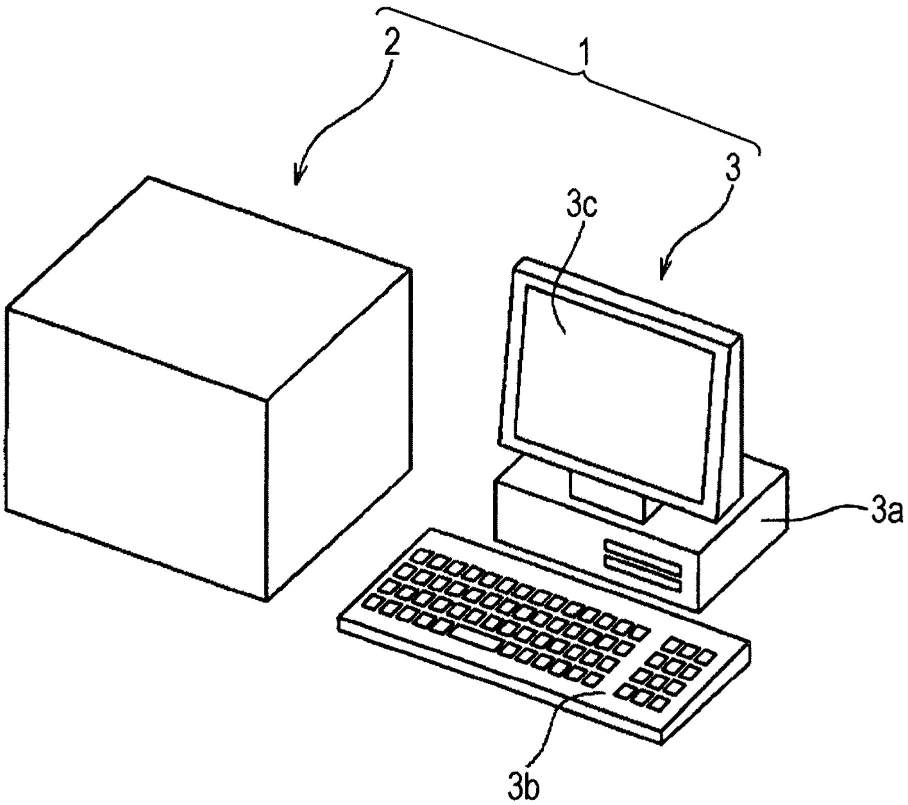

FIG. 5

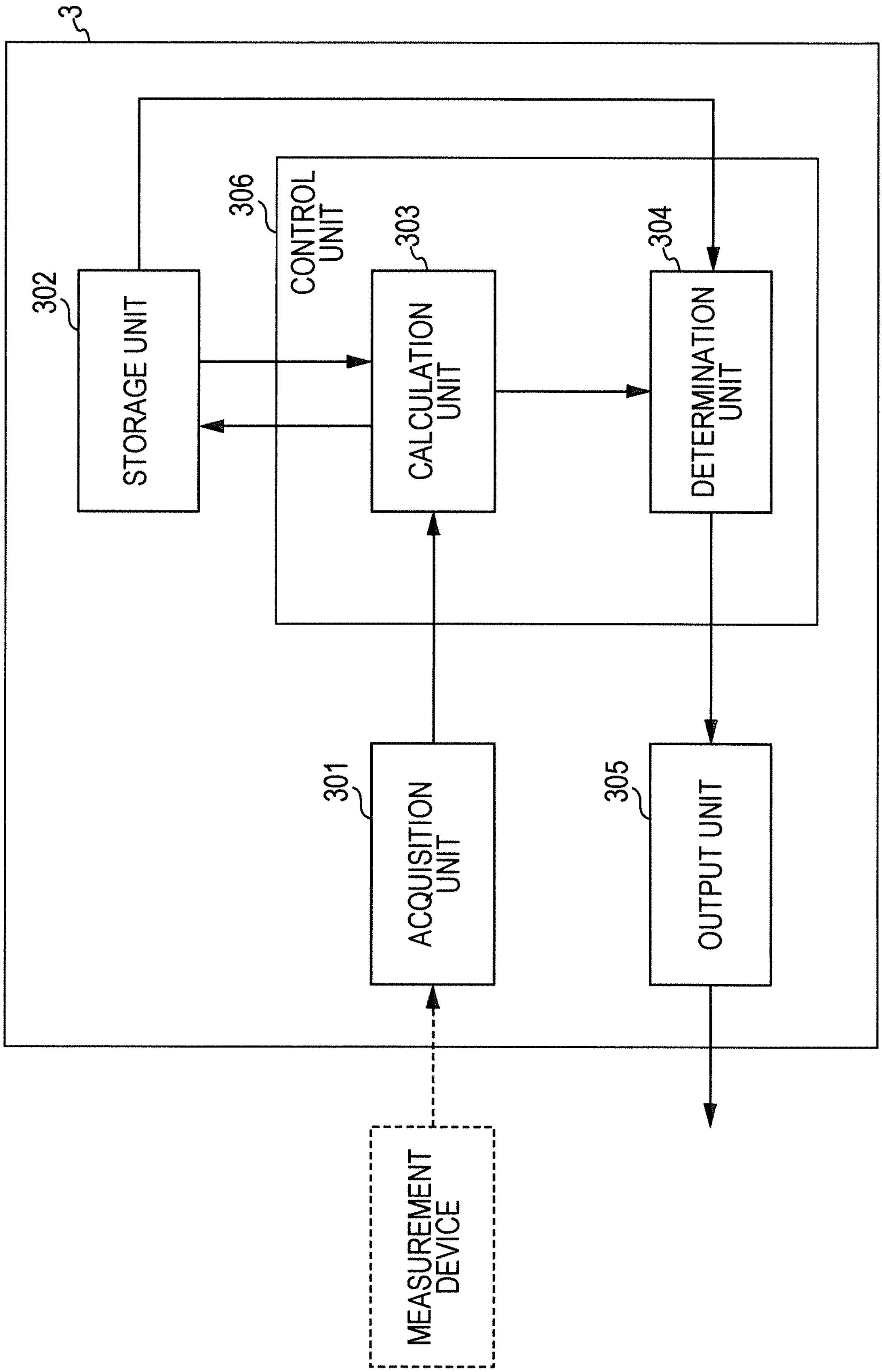
3
302
STORAGE UNIT
306
CONTROL UNIT
303
CALCULATION UNIT
304
DETERMINATION UNIT
301
ACQUISITION UNIT
305
OUTPUT UNIT
MEASUREMENT DEVICE

FIG. 6

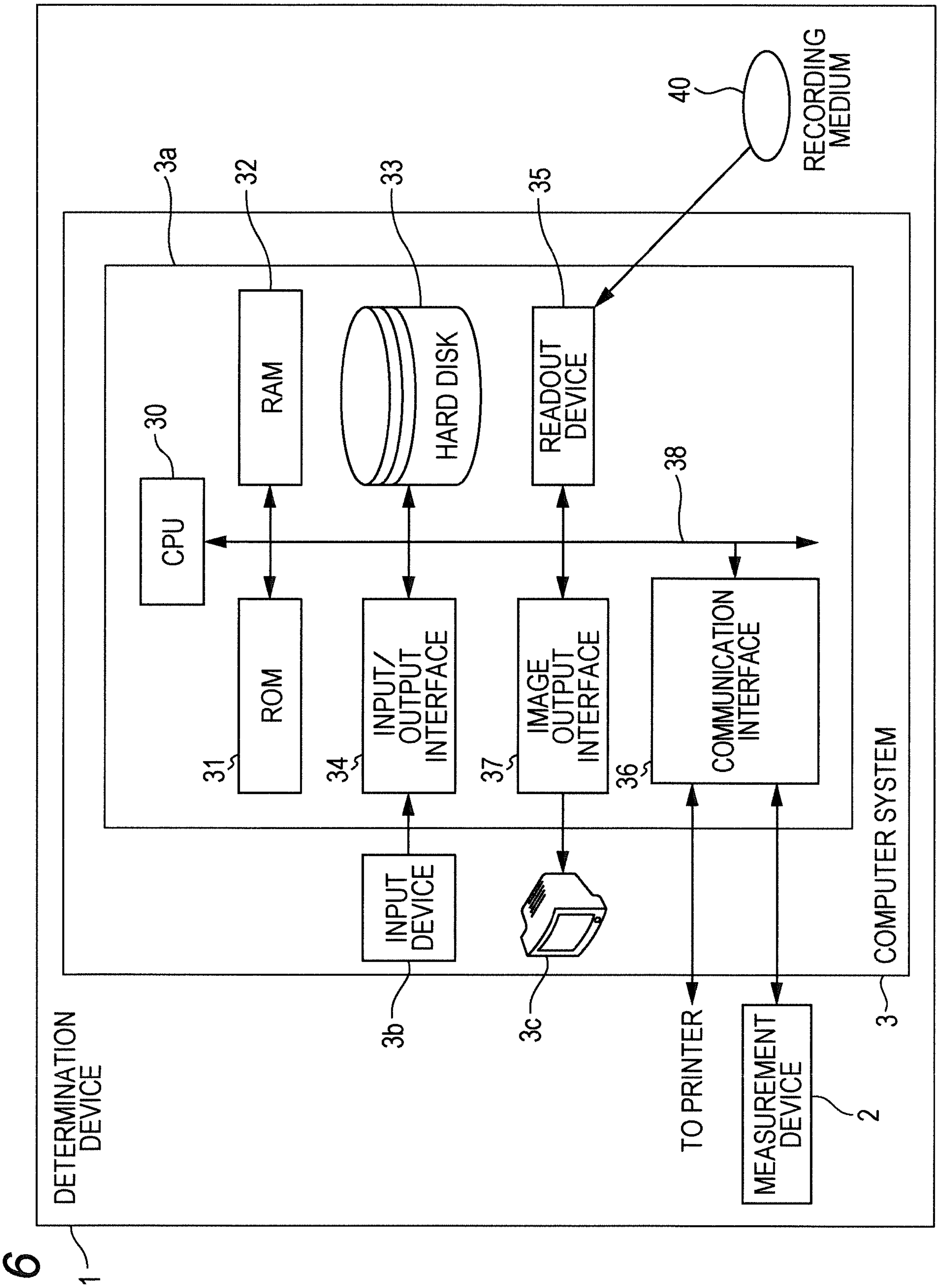
1
DETERMINATION DEVICE
3
COMPUTER SYSTEM
3a
30
CPU
31
ROM
32
RAM
33
HARD DISK
34
INPUT/ OUTPUT INTERFACE
35
READOUT DEVICE
36
COMMUNICATION INTERFACE
37
IMAGE OUTPUT INTERFACE
38
40
RECORDING MEDIUM
3b
INPUT DEVICE
3c
TO PRINTER
2
MEASUREMENT DEVICE

FIG. 7

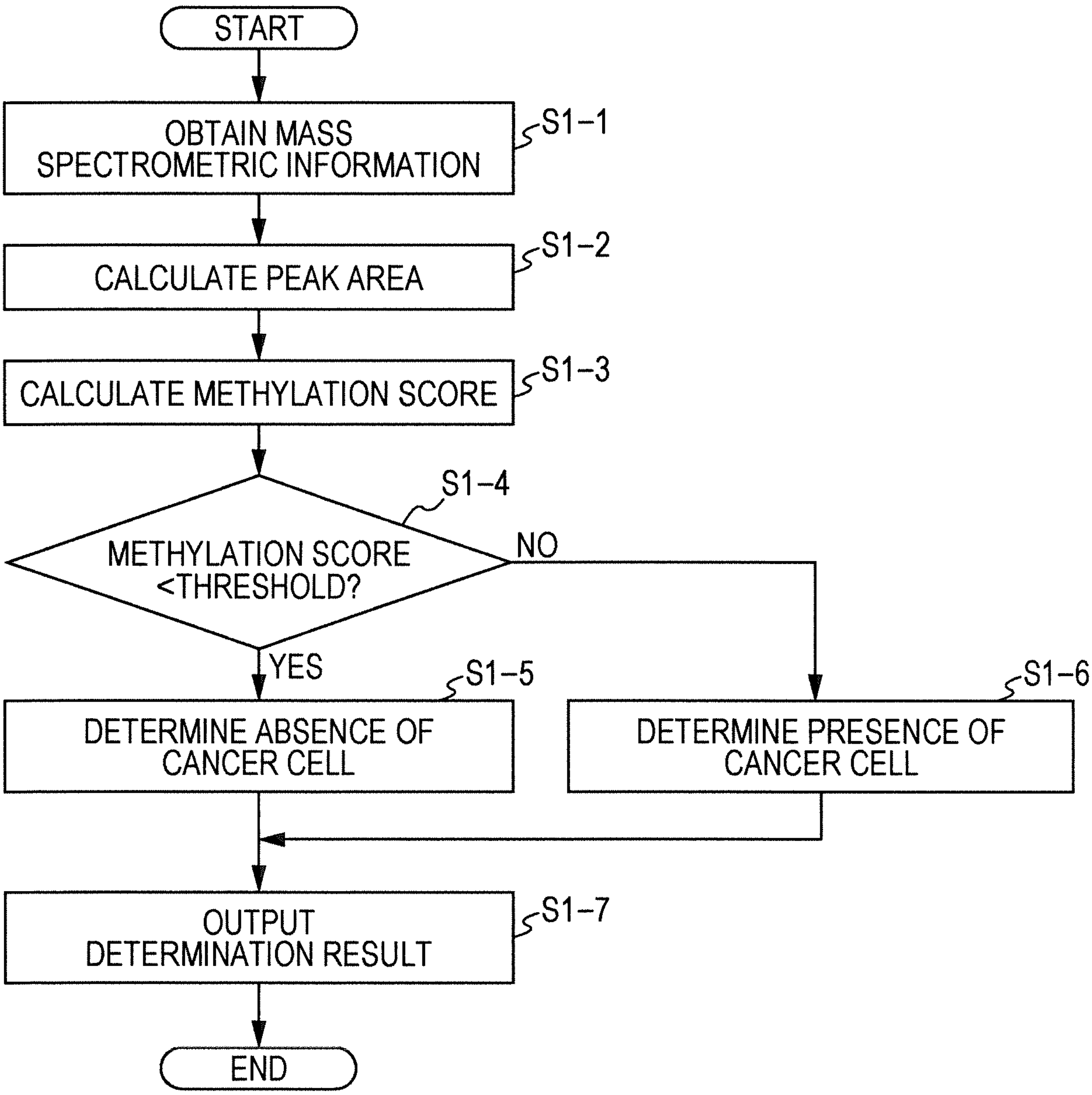
START
OBTAIN MASS SPECTROMETRIC INFORMATION
S1-1
CALCULATE PEAK AREA
S1-2
CALCULATE METHYLATION SCORE
S1-3
METHYLATION SCORE <THRESHOLD?
S1-4
NO
YES
DETERMINE ABSENCE OF CANCER CELL
S1-5
DETERMINE PRESENCE OF CANCER CELL
S1-6
OUTPUT DETERMINATION RESULT
S1-7
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120190024 A
- US 20120178634 A
- US 20130130242 A
- WO 2009128453 A1
- JP 2014143326 A


### Non-patent literature cited in the description


- **SELAMAT SA et al.** Genome-scale analysis of DNA methylation in lung adenocarcinoma and integration with mRNA expression. *Genome Res.,* July 2012, vol. 22 (7), 1197-211
- **KOBAYASHI Y et al.** DNA methylation profiling reveals novel biomarkers and important roles for DNA methyl transferases in prostate cancer. *Genome Res.,* July 2011, vol. 21 (7), 1017-27
- **SALHIA B et al.** DNA methylation analysis determines the high frequency of genic hypomethylation and low frequency of hypermethylation events in plasma cell tumors. *Cancer Res.,* 01 September 2010, vol. 70 (17), 6934-44

SEQUENCE LISTING

<110> SYSMEX CORPORATION
THE UNIVERSITY OF TOKYO

<120> METHOD, USE OF MARKER, AND DETERMINATION DEVICE FOR OBTAINING INFORMATION ON PLURAL TYPES OF CANCERS

<130> 13-024JP

<150> JP 2014-143326
<151> 2014-07-11

<160> 59

<170> PatentIn version 3.5

<210> 1
<211> 4169
<212> DNA
<213> Homo sapiens

<400> 1

```
cacctaagag gaagtcccaa ctgggcaggg cacctggaga cctgggtcat gggcaggtcc      60
tgctgctggt atgacaggct attttccatg gcaagtgttt tccgcttcca gttgcgagca     120
gtggcaggca agagtcccct gggtaacact ttcatagtct tcacctggac tagagaggtc     180
ctttctttgc caagaatttt ctagaaagcc agagaggaaa ggcaggcata ctctgcctct     240
ccactggggt ctggggagca gggcggcaga acaaagcccc tcacattccc atcccccagc     300
cccaagccaa gggttgagca aggatagttg atgactccat gctctgccac tctccaggtg     360
actctgcgca aggtgttgaa ttctttagct caactttcct tttctataga atgggaagaa     420
tacccatctc acaggataga gtagttgtga gaatgaaatg gcatgaggtg tgtggagcag     480
caggcaaggt gcctgacatt caagggacaa cgaatgggca ctatcattat ttacagggag     540
cgagggagac aggcatgacc catgcatcct ctctgcctcc tcctgggctc ggctccaatg     600
tgtcctctgc tgatcacaga gccccccagc ccatcacttt actcggaacc actggatagc     660
acactgtgca tttatgcgct tcctgttggc tgtctcctgt gtccatccta aagctccatg     720
tgctcgccct gtgcgttgct caatccacaa tgcaccgaag gctacctggc aatgagtatt     780
tacataaaga aggaatgaat agagggacgg ataaataaat aaatgactgt gccctcacca     840
tacagaccaa gaagcacagg tgggagaagt gaagtctcag ttggagcaca tacagcgagt     900
ggacagtggc tccagcagca ttagcccacc ccatgtgtgt cctccaaagc cttccaggtg     960
cgtagaagct gcttctctct ctgccatgat cacccctggc actgggaaca cactggtggt    1020
tgggggcaag actacatcac ctcctctgtg ccagcctgtt atgccagcca cacacaggat    1080
gaggtgagaa tccgctcatt ttatagatta ggaaattcgg tctccaagag gctaagtggc    1140
ctgctcccac aatgacagca agggtgcatc cctcattcct aagccccaac gtggcagggg    1200
```

tgtcatgtaa tcaacaaacg tttgtcactg gctgtgccaa gaatgtgcta ggacccttcc 1260
gctctctgct ctacacagcc gggtaaactg aagccaagac aaggcaagcg acttgctgtc 1320
acccaggact ccccgcgtgt ttgcacaaat cttagccgag ttgatttgta aaacacccaa 1380
acactgcggg cgcatggctg caggggggaaa ggaagccaag taacacagta ggacccagca 1440
tccgcgtcag ggagagaccg gggggctggc ggagtggggg cggcggtgtt tgtcaccgac 1500
tctccaatcc cagtcctaga agcagggtgg actatcccag ctcgatcatc ccagattccc 1560
caggctcctg ctaggagaag tccctcagcg ccggcagcgc tgaccctggt ccaggggtcc 1620
gcccttcccg gtaatgtcag gcctttctcc ttggcataga tcggccttgg ggtctgcgag 1680
gactgaacct cactaggcct ctctctccca cccgcaaaca ttctcgagtg ttatgcggct 1740
caatgcccca aggtgagcag gcgccgcgtc tccgcggtgg ccgagagggc aatggctgag 1800
gagttcgttt gggacgaagc agtggagcag gggaactggg gagcctgggc cgggcctctc 1860
tgtgggtccc agcgcagcgc agccaggcag gcgtcgggac ctaggcggct ttggattcgg 1920
cgggcgacct cgctttgaag ctgatcaccc gctcaaggtg atcacgacgt gccgacccag 1980
tctagatttt gagttctgca gaaaatgcaa ttttaaagga aaaggacccg cctgcccttc 2040
ttcggcagtt cgtttggccc aggccttcga tcagctgaaa gtttccgatc taggggggatg 2100
tccaggctgc cggcggagca gagccgataa ccccccctctc ctgcgcgttc ccctgagact 2160
tggcctccgc aacctgtccc agccgcggag caaactgctc tgcggcccgc gcgagtgcgc 2220
cccagggctt cgccgctgcc gggcgtcccc tccatccacc tcgggacccg cgccatcacc 2280
gcggcggggt aagaagctcg gaggcgccat cccggggctc tgcgccgtcc gctctcccgg 2340
ctcctggccg ctcacgcaca cagccggtag ctggttttcg ttagccgctg ccctcgccca 2400
gaaggcgggt ggaaggtcgc cagttggacg cacagccaaa ctcttagcgc actcccgcgc 2460
gagatccccc atcacccggg gctgccgtcc tcatttcgag ctctttctag agcttggctc 2520
ccgctgaagc gcaacttccg cgcccaaacc ttgcttttac gacgggcggt ccgcgtccaa 2580
aggtctctta caggcctgcc ctttcagctc ctcgccctcc cttcctcccc taccggcccc 2640
ctcccctgct ctggccgccc gctgcaatcc tttggggtct gtggctgagt ggggaaccgc 2700
cgacccgcga tccggaacgc gactttgagg ccgccgggag catcctgtgg cctctctctg 2760
cgcggccacc cggccgcggc gcgaagcggt ctggagggcg agcccttccg cggccccaac 2820
tctgccgccc cgttcccggc attgggaacc agggcaggga gggggcgggt gtttctctgc 2880
gggggagtgg ggaggaagct gggcgggtgc gcgcggtgcc ccgagcctgg aaccacggag 2940
ggcgcgttgg tcttgggcgg atggaggggg tgtcgcactg ccgcggggag gcgtgtcggg 3000
aggctggggc cagtggcagt cgcttggcga gggtgggggc gtagcgctgc ggtgggagga 3060
ggcggctccg gccctggtct ccactctagg ccggggtggg gggcgcatag cggccgccgg 3120

agctttcagc agggggcgct gctccgggcg ttgggcgggg gtggggtggg ccaggagggg 3180

gggccgcggg ctggccgcgc acacttcccc cattattaaa cactatgttc aaaaggcgcc 3240

gggggacttc ccggagccac ggagcccgcg ccgcccgccc gcccggccca cggagcccat 3300

ggacctgagc gccgccgccg cgctgtgcct ttggctgctg agcgcctgcc gcccccgcga 3360

cgggctggaa gcggccgccg tgctgcgagc ggcgggggct gggccggtcc ggagcccagg 3420

gggcggcggc ggcggcggcg gcggcgggcg gactcttgcc caggctgcgg gcgccgcggc 3480

tgtcccggcc gccgcggttc cccgggcccg cgccgcgcgc cgcgccgcgg gctccggctt 3540

caggaacggc tcggtggtgc cgcaccactt catgatgtcg ctttaccgga gcctggccgg 3600

gagggctccg gccggggcag ccgctgtctc cgcctcgggc catggtcgcg cggacacgat 3660

caccggcttc acagaccagg cgacccaagg tacttacgcc tcttctgtgc ccgcccatcc 3720

cgtcaggtcc tgggctgaga ccagccccgg agccgtgccg cagctccgtt acatttggag 3780

ccgcggcccc atgcggccca ccctcaggtg atagaaagaa acttcgccga ggccaacact 3840

ctccagcccg ctgcacctgg actgtggcga gagtcgcggc agctcccggg gcccagtccc 3900

agagggctga ctggtccctc tcccaggtct ccctggcctt gcaggccggc tggtcccctc 3960

gaggaggcag gcggaggcca agattcgctt cttggggaat ggtctggagt ggcctcggag 4020

ccctcagaga agaaaggagg gcaagagctg tgttcccggg agtcacggcg agagggactg 4080

cacggtggtg agggtgatgg ctccttcctt gcagcaattt ctgcagctcc cagtctcctg 4140

cgggccacac aaattggggg aagcccagg 4169

<210> 2
<211> 4162
<212> DNA
<213> Homo sapiens

<400> 2

```
tccatccctt ggatgtttcc agcctaaacc cgacataatt tcataatcac atgtttagtg      60
gtctacttgg cacctcaatt tggtagtgtc tgggacatct caaatacaac atggccaaaa     120
tccaactctt tatcttcccc ctgaaatcta ctcctcacac caacttccac atctgaaaga     180
tctaaggtca attttttttt tttttttttt gagacagagt ctcgctctgt cacccaggtt     240
ggagtgcagt ggcgcgatct tggctcactg caagctccgc ctcccgggtt caagccattc     300
tcctgcctca gcctcccgag tagctgggac tacaggcacc caccaccacg cctgactaat     360
tttttgtatt tttagtagag atggggtttc accgtgttag ccaggatggt ctcaatctcc     420
tgacctagtg atccacctgc ctcggcctcc caaagtgctg ggattacagg cgtgagccac     480
cacgcccggc aatttttttt ttttttaag acagagtctc gctctgtcac tcaggctgga      540
gcgcagtggc acgatctcag ctcaatgcaa cctccatctc ccagattcaa acagttcttc     600
```

```
tgcctcagtt tcccaagttg ctgggattac aggtgcacac taccatgtcc tggctaattt     660
ttctgtattt ttagtagagg tggggtttcg ccacgttggc caggctggtc ttgaactcct     720
gacctcaagt gatctggccg ccttggcctc ccaaagtgct gggattacaa gtgtgagcca     780
ctgcactcag ccttaggtca atcttaacat ttcactttct ttcactgtcc cacatcaggt     840
aatccatggc ccagtttggg cagattcaga agccaaccat ctgtcctacc tccacgggca     900
cccctctgtc cagctaccca aagcacacat ctggaccaca tctgtcagct cctcactggc     960
cttcctgctc caccatcacc atccctccag tctgctgccc actaggcatc catagggcgt    1020
ctgttaagac tggagtcaga gcacctactt cctctactcc aaaccttcca tggctcccac    1080
caccatcaga atagaggtcc agctcctcag gctgccattc caggcctaac tattggtttc    1140
ccttccctgt ttccaccaga ctaaatggag accggcagtc cccagaatgc ttccatttca    1200
tcaccactaa acaaggtaca catgctggtc actgtccctg ggacagcctt ccagatctca    1260
ttccacagga ttccagaaat gggaatccct cgatataacc ccttgcctgg attcaggatg    1320
gtaagggtaa gtgactccca gggcagggcc caggacccac gtgtgaggac aggacacgat    1380
caagagtccc aggacaccaa catcccctct atctgggctt caggatcctg agggccggaa    1440
tcccagcggg tgagggcctg gggcacactc cacttacctg cgccgggccc atcagcaacg    1500
ctggcaaccc cattagaacc tgtgggctgg gcagggccat aacaggaggc ccagggctag    1560
ccctgccgct gggccgaacc ctcacacttc cgctgggtga cggtcgcact caggacctgt    1620
cttcccaaat gcaaaatgcg cgcaaggcct ctgggcaccg cagggacgaa ggctgggtat    1680
ggagaccctg gagacgcgtg gcgctggctc cactttcggg aacaccttgg ctcataaaag    1740
cagagtaatt agccgggcac tatggtgcgt gtgaaggcgc ggtgacggtc cctgcaccca    1800
ctcccgtgcc ctggtgtggc tccagaggcc tgctgtagcc caacccacca gcagcaaaat    1860
gaggaccgca atggcggcgc cggaagtccc gcctctcaat gacagctcgg acttgccact    1920
ggtccgagct ttcattggct caaggatccc cgccgtctat gacgctattt ccctatgccc    1980
gtcacgctac tgctaggtcg ttgccaaggt gattgaggaa tggcgtttat tgcgtcgctg    2040
ctcaggcaac gcaaactaca ttatccagaa ggaccctcgc ggtgcctcag ggctggccat    2100
tggcagccga ggagacaggc acttccgggc ggagtgtaag acgctggcca atcacagcct    2160
ggcagcggga cttccgtcgt cgtcctcgga ccatcacttt ggcatttctc gattttgtct    2220
gcttctgaag ggaccgcgtt gtcaggcgag ggacggaatc ttggaggctc cctgggccca    2280
tggaaacagg agcgaggaag gcacgagagt cggggaagtt ccgccttctt gacatacaag    2340
cgcccccacc gcggcgaata tcgcccctga gcccagtgtt cgcccccac aacaccgcac    2400
ccccttccc caccctgtgc cttagataaa gccggcccag tgggaccctc aggttgcacc    2460
```

gcgcggaggt gtccgcggct ctcgggagtc gtcgcccttg gcgttatgtt ggctttaggg 2520
agcgcgaggc cgaggccaag aaaagctacg cattgggagg tcttgaccca gtgtgcgccc 2580
ggctgtaggc atggaacgtg cgccaaaggt ctcctggcac ccactccgct ggactcagca 2640
ccattgtcct aagtctccga ttgagaagta tctgccccag gtcctctgcg ggccccactg 2700
aacagtgtgg agcgttcaca ggttgggggt caccatttca gaatccagtt tgttgagacg 2760
tggagggtac aggaggaggg atctgcatat gcgaagactt ggacttgacc ctgacctgag 2820
cgttcaggca ggctctcatg ccccttggc agcgagtgga gagcgtaaga ggagtcctgc 2880
ctctcctggc acaccgaggg cgcctgggag ccatgaaagg tgttgaacag aaaagggatg 2940
gacatgacct gagttagaat ttggaaagta tttcagtggt tgctgaatga acacactact 3000
ggagaaggtg gttgtagtgg ggctggaagc agggagactg tgggggaggt tattgcagta 3060
ttgttccaga cagttatgat agtggcctgt ccagaacgga agcaagaagg atgggagaag 3120
tgatcagatt cccagtggat cttcaagata gagctaatgg aattttttgc taccttagat 3180
aagtggaata agcatgagta ggggtgaagg accatcccaa gtagttagga agaattagga 3240
tgcctcctgt ggagaataga ggttggcagt tggcaggagg aggatgtttc atggaagatt 3300
agcgatggat tttgtaggta tagagtctga attgtctgga gacctgtgag tggaggtgtc 3360
aaatgggcct ctggatatat agaattctgg tgtggcttca ggacgtccag actgcattat 3420
gttggtggcc tggcccagtg aacggcctga ggataggatt tgggaaggag ggttttaggt 3480
tggaggacca gtggctaggg ctcacgctca gtgcttagat cacatctgtc tgcccattgg 3540
cttggtgttg cagggcttaa tgacctttga ggacagggtc agtacatctt ccaagaatgt 3600
ggggctcctt agtgaggtcc aagggggctt ctacagtgac atgatactac agagctttgt 3660
actccagtgc ccactaggta aggctactgt gtctcctctt tcccctacac atgaaatcta 3720
ggttttcctc cgcctttcca ttgttccctc tgggaggtgc tgctggccct ggcactggcc 3780
agtgtcctgc tgtgtccgga attgtttcct tcctctgggt tcttggtctc gctgacttca 3840
agaataaagc cgcggaccct cgtgttgagt gttacagttc tttttttttt ttttttttga 3900
gacggagtct cgctctgtcg cccaggccgg actgcggact gcagtggcgc aatctcggct 3960
cactgcaagc tccgcttccc gggttcacgc cattctcctg cctcagcctc ccgagcagct 4020
gggactacag gcgcccgcca ccgcacccgg ctaatttttt tttttgtat ttttagtaga 4080
gacggggttt caccttgtta gccaggatgg tctcgatctc ctgacctcat gatccacccg 4140
cctcggcctc ccaaagtgct gg 4162

<210> 3
<211> 4148
<212> DNA

<213> Homo sapiens

<400> 3

```
ggtccctgct ggagtggggg tcacaagggg cccggcagtc accagtgggg tggggcgagt      60
tagagttagc ttcttccgct tcttcataat gaccttttc ttcttgatga ctcgaatccg      120
gacatgctgt tctgaggtcc cttggggtcc gagaggcaca gcatgggga aaggaagaat      180
ggtgaaaccc cggatggaaa ggtctgccag cccaagaaga catgtgagcc cacagcaggc      240
cacaccacca ggatctaaca gccttcacag gacatcaaag gcctgcaatc tgaccccaac      300
ctgcctctcc agtcacttcc ttggctcctc cttctcctcc ctgatgtaac tacatgcttg      360
gccagcctcc cctgtgaacc tctaatccaa tggagagctg cacccagcct ctagaaactg      420
ttcggttgct ccaaagagct gagtctggca ctttacctcc gggcctctgc actagctatc      480
ccctcccctg taatgctacc cctcgcctct gatttttca cctaattccc attcttcctt      540
tgcatctcag cccagatgcc tttccaagga ggctttctta gctccagact cagcatcatg      600
ccccaaaacc cctgtgccgc ttccattgtt gtgtctgctc tctgggctca gatggcctgt      660
cctggaatcc atgtctgcct gggtgtcgag gctttcaggg ctgggcctgc ccgccggccc      720
ttcagcacaa ggctctgccc aggcaggtgc tggggtacc catcgaagct ccacagctgc      780
ctccagaaca ggctgcccat ttccgcatct ggtgcccctg tgcctttgcc cgccgcttcc      840
ttcttggcag ccctctctct ctctctctct ccagactccc ctgactcggg gacatcaccc      900
ttcaagaccc tcctccactt gcctcctcta gaagcccctg agtgccctcc gcttgcgctc      960
ctcctctcct attccacatc tggaaagctg ggaggtcggg ctgccttcct ctcccgctct     1020
gccccatcct cccggcagcc tcagcgaggg gctctccctt ctttcctttt gttctttctc     1080
tccagccctc gccttcaaac tctctcctac gcagtcttgc tctgcacctt gagagcttct     1140
gcccctctgt ctcccctcct gccagttctg gcctgttttg tctttggtga aaagggaagg     1200
tttggggctt ggaagaggca gcatcccact ggcgcctgtc ctgggactgc tgactaggtc     1260
tggcagggaa ctggggctgc cgagtcagca gccattgctc ccagcaggga gttgagggag     1320
tgtgagtgtg tatatgtgtg tgtgcactgt gagtgtgagt gtgcgcgcgc gtgcactgtg     1380
tgtgcgcgca cgcgcgtgtg agtgtgcgtg tgtgtgcatg cgtgtgaatg cgcgcgcgtg     1440
tgagtgcaag tgtgcgcgtg tgtgaatgtg tgtgagtgcg cgcgcgtgtg tgcgcgtgag     1500
tgcgagtgtg cgtgcggggt gagtgtgcat gactgtgagt gtgcgtgggt gtgcgtgtgc     1560
ccgcgtgtta gggagggatc gccccacggg gcaggagagc cgggcagtcg gcttgcgggg     1620
gagcggaccg tcggtcgggg aactcaccgt tagctgtctc cgccggcggc tgtgccgggc     1680
tgctatgcag ggccggggtc gagcctggga ccttggtggt cccgggctgc gcgaggccca     1740
gcaccgagtt cctgggcgcc cccagagccg ggccgacggc cggcgcgaag gcggccaggg     1800
cgagcaggag cccccacatg gcggggattg agtgccaggg gcgcgcgggc tacggcgggt     1860
```

ggcgggtcgg tctcttcctg ccgagtgcgc cgagcccccc gccccttcct gcccccgcc 1920
cctccgcagc ctcctgcccg cccacagccc gctgcccgcc tcggacctcg gagaggagga 1980
gagggccggg aggagcgagc ggggctgacc tccaaggggc cgccccaagt ctcaggaccg 2040
ccgactctgc ccttcctctc cgccgtctgg ggccaacccg cctcgttggc gcgctgcgcc 2100
tttatagtct gcaaagccac actgaagaga ccagagatca cctggtggcc atcaagtcga 2160
ccaacaggag gcaaagcccc ttatctgagc aattcagaat gacacttccc tattgtctaa 2220
agccacatct ggtaccaagc ttccgtccca agaatttata agtaactaga atttctgtac 2280
gtttccggaa tgagtgcacg ttaagaccca ttgtgcaaac tttgctgaca tcaaggcacc 2340
aaaatatcga caaatgtaac catttaccat gacctaagtg actaatgtgg tccaaattac 2400
cctgcagttc cccgcaggga gctcagtcct ctcttgctga agcgcctact gcgctctgct 2460
gcctcgtcct ttccgtctaa taaaactttc cttttcagac ctacacggtt gttgataagt 2520
tcttttacca cccgggagcc aaccacttcc cggttccgga gctctgacac ctcgcctggc 2580
atcttggtgg cccatacggg gacttcactg ggatttccct cttccttttt ctctctgctt 2640
ccttccacgg tctggttctt tactctttgg ggaactgccg gtcccggtgg aggcagctct 2700
tctgtgggat cctgaagccc tagagaaggg atacctggct gtccttgccc ttaggggtga 2760
gggactggcc agggctcttt tctgttttcg gactgccagt gaaccagctt gagttctcgt 2820
tggcaactga cagtttctgg ccaagcgcca tcccccggta ttaccccaag gccaagacaa 2880
aagagctatt gcctgtcaga gtggcagggc actttcattt taacactcca taagccttgt 2940
cctggaagcg gtggatctca attctacaca gggatgcctc agggatcttg tagttccttg 3000
tctaaaccca acttcggttc aattaattca gttccatcca actcgccctt ggattgcctc 3060
cttaaaaatg gtcccattgg tctcattttg acccacaaac tcttaaaaat aagcatataa 3120
ggctgagcac aatggcttat gcctgtaacc ccagcacttt gggaggccaa ggcaggcaga 3180
tcacctgagg tccggagtta aagaccggga gtctactaaa aatgcaaaac ttggccgggc 3240
acagtgactc acacctgtaa tcctagcact ttgggaggcc gaggcgagtg gatcacctga 3300
ggtcaggagt tcgagatcag cctggccaac atggcgaaac cctgtctcta ctaaaaatac 3360
aaaaaaaaaa aaaaaatcag ccaggggggct aattatatgt ggcgggcaca tataatccca 3420
gctactaggg aggctgaggc aggagaatca cttgtacccc gggggcggag gttgcagtgg 3480
gccaagatgg cgccacagca ctccagcctg ggcgacacag caagactctg tctcaaaaaa 3540
tacaaataaa aaataaataa gagtttaaaa agcatatcct tttttctaat attgcttggg 3600
ttcaatacaa gctccctaat caatcaattt ggccaattaa tgaaacttgg gattgcaatg 3660
ctactttaca acttaactga ttttttgccg gaaccttgga aaggattcag aagtcccata 3720

```
tgttcaggtc ttttttgctt tatccccaaa tccaaaccca aaattacata caaattgtca     3780
tgtatgcttc caaggaacgt cctcccttcc cggttctgat gtcttagatg atccttcctt     3840
ttgcctatta catcctcctc aacctgctca ctcttcacct acaccctctc catctgctcc     3900
attccctagt caatctccca cccacatacc caaatacttt ctccccttca catactctca     3960
ggggagtcac atatgctact agtacagagt cctcagaaaa atccccaaaa tattttgcct     4020
ctccctaagg tggctaatga agatttggga acaagccaag ttcatgtccc ttttctgatc     4080
atgtctgatc ttttgcaaat tcaatccaag ttgggttcat ttagtcagga tccctctaag     4140
ttcattca                                                              4148
```

<210> 4
<211> 4151
<212> DNA
<213> Homo sapiens

<400> 4

```
gtgtaatatc aggcttttgg caactagttg cattgttcat caacaacatt aagtcaccaa      60
gcttaaaaca tcacaacaat gaatttaatc aatgaacaat gaaatgttat caagaccatt     120
cacaaaatta aaagcaggcc tggcgtagtg gctcaccccg taatcccaac actttgggag     180
gctgaggcag gtggatgacc aaaggtcagg agttcgagac gagcctggcc aacatggtga     240
atccctgtat ctactaaaaa tacaaaaatt agccaggcat gttggcatgc acctgtaatc     300
ccagctactt gggaagctaa agcatgagaa ttacttgaac ctgggaggcg gaggttgcag     360
tgagattgtg ccactgcact ccagtatggg cgatagagac tcctcaaaga aaaaaaaaac     420
aactatatga attaaagctt acattaacaa caaataagag tttgaatgtg ttatcctgca     480
tatggaaatc agataggtct ttgaagaaaa actgcaacct ccgcctccca ggttcaagcg     540
cttctcctgc ctcagctttc tgagtagcta ggactacagg cacgtaccac cacgtttgac     600
taatttttgt atttttagta gaaacagggt ttcatcatgt tggccaggat agtctcgatc     660
tcctgacctg gtgatccacc cgcttcggcc tcccaaagtg ctgggattac aggcgtgagc     720
cactgcatcc ggccagaaaa acattttaaa atactttgta cactatttga aatgactatg     780
gaatgcttgt ttcaatcaat aaaaaaaaga aatatgaaat gtgaaatgct tgtttcaatc     840
aataaaaaaa agaaatgtga aatgtaaaat ttaataaaat ttaaatttta gatacattta     900
aaatgtcaaa gtcctcctta agcttcaaga aaataaaatc aatctcctta aaatgaacaa     960
attatgatga attcgtcaaa aatatttata ttgtgttaac ctcatttacc aagacatttc    1020
aaatttacat ctttgtgtct gttaaagaca gatttagaaa tacactaatt ttattcaaat    1080
acagttttta gttatgatga ttagatttaa agatatgtgt gatcggtcag tttatgatta    1140
tgtagaataa catgtcaata tttacaggat aatgcaggta ggtaagacac ctgttctact    1200
```

attgcatgat ttcatgattt aactggtgta attaggaaag gttttagatc taattcgaac 1260
tgtcatatcc actttttttt tttttttttt tttttttttt tttttttttt gagatgcagt 1320
ctcactctgt tgcccaggct ggagtacagc tcattgcaac ctctgcctcc agggttcaac 1380
cgattctcgt gcctcagcct cctgagcagc tgggattaca ggcacccgcc accacgcctg 1440
gctagttttt gtatttttgt agagacaggg tttcatcatg ttggccaggc tggtctcgaa 1500
ctcctgacct gaagtaaacc acccaccttg gcctcccgaa atgctgggaa gatatacttc 1560
taataacagc atttccaact acattacttg gttgaaacag ttgaaactgc cattgcagtt 1620
gcagtaaagg taacaagagg taaacttcta atatgtaaca ggagatttga cttttctcat 1680
atgtaacagg agatttgact tttcatctag aattagaacc aaaatcgtta aatttgtata 1740
gatatttgca ttagcagaaa tcccattttg ttcatgttgt ttttaaataa agtgactcgt 1800
ttaaaattta gttgcatttt aaaaatctgt gttatgggag tggtaatact gactccaggc 1860
agggaaatga gacaattgtt agagacgcag ggatggagct gagaggtcct cctggtctcc 1920
aatggcccgg cggtggctcg atgggaggga gagcgccccc tggaggcatt ggcggttcct 1980
ggctcgtgtg ctcgcgggaa cccaagctgt gagcctctgg ccccgccagg gcgtacgaat 2040
cctttcctcg tttatccagt attaagtcac gctgtgtgct aagtaccgtg gccaggcagc 2100
agctagacaa cgcgctgagg taaaatagag aactaaggaa gaatgggggga ccccaaagtc 2160
aacttggtga cacatcacac gcaggagtcg gacccacggc tccccgagcc acagctcagt 2220
ggggcgggac cgcccgggcc tgggcggggc cggggcgggg cctgtcacgg gggaaatcac 2280
cgccctagca tccggggaaa tcgcggtctt agcatccggc gcgcggcggt tgaattgctg 2340
cgcccagcga ggcaaccgcc tccgaacgcc aggtgggggc gaggcgtctc ggagtctcag 2400
agacaccaag gcccctgcga caaggtggct gcagctaggc cgggggcgtc aggacgacgg 2460
agcgggttcg ggtcggtgac acgcagacct gagggagctg ggcccgcctt ttccgcccgc 2520
gccccaggcc cttgcagatc gagatttgcg tcctagagtg ggaaaaaagc agaggccagg 2580
gcgccggtaa gtaaaggttc ctcctcaccc gccgccaggg cccggtccgc ccaacgagca 2640
gggcggcgtg agggtcacgg gctggaaaca acaatgccac agctcaagcc gactccaaca 2700
gtagtagcgt tttgtttgt ttgtttgttt gtttgttttt tgagacaggg tctccctccg 2760
tcgcccaggc tggagtgcag tggtgtgatc tcggctcact gcaacctccg cctccggatt 2820
caagcgattc tcccgcctta gcctcctgag tagctggggt tacaggcgcg cgccaccaca 2880
ctcggctaat ttttgtattt ttagtaaaga cgggtttcac catattggtc acaccagtct 2940
cgaactcccg acctcaggtg atccgcccgc ctcggcctcc caaagtgctg ggattacagg 3000
cgtgaggcac tgctcctggc ctacagtagt ggcttctgga ggccactcag gcaccccctg 3060
tttctagctt agagaaggag gaaatcgaca ataaagtagc cttaaaggga tccatgttcc 3120

gccctgccac ctcttcagat gtcagcgttg tccaactagt gtccctagaa ttacccagga 3180

tgtgagtggc actgattcat cagtggttta atcagcaaaa ccaagtgtct tacctatctg 3240

cctcatctgc tcactgcctc tttccaaggc gttctcacag gcgcaaacac tttcttttcc 3300

tccattaatt ccagtaggta agattttgca gtctccttta aggtccccgt ttctcaaaga 3360

tagcccaagg aatctcgcca cttccttgcc ttttacacat ctggattcat ccagagacat 3420

aaaccctgac caggactagc ttcaaaaggg tcccacccgt gcagtcacat agggctcaga 3480

agggccctgt acttactttc ttgctctgct atggcaatct tgaaattctt aatttttaaa 3540

ccagcacccg cctcccctcc ccgccacctc cacaacattt tcattttgca ctgggcctgg 3600

ccttaattct caattttctc aaaggctgtc atgtaactat ataaaaatat agttaaaaga 3660

gtttcttctc tagacatgtc tttagtcact aggacatcaa gagacaggac ggatgcttct 3720

attaactgag agttgttgga atcataaagg ctttgtaatc caatagtcac cctccacgcc 3780

ttctcaattt cagaacctaa gagagtttgc ttagagaaag aacctgagag gttgtaaagc 3840

tgagctgacc aaagctcatt gaatcacatg tagctattca gcacttgaaa tgtggctggt 3900

ctgaaaaaag atgtgctaca aatgttaaaa cgcacaccag cttttaaagt ctcttttttt 3960

cctttttttt tttttttgag acggagtatc cttctgtcgc tggagtgcag tggcgcaatc 4020

ctggctcact gcaacttctg ctgcctgggt tcaagtgatt ctcccgtctc agcctcccga 4080

gtagctggga ttgtggtagc tgtgcctgcc accacacccg gctaattttt gtatttttag 4140

taaagacggg g 4151

<210> 5
<211> 4189
<212> DNA
<213> Homo sapiens

<400> 5

```
cccagagata agagacaact atgagagatc gagagtcaga gaagggtcaa taaaaagaga      60
tagagactga gcaagagaca cgtagagaga gaggaagacc cagacagaca ggatgtgaga     120
ggaagggaga ctgtgggaat gagagagaga aaaccagaaa gacacagagg agagagacaa     180
cggatagaaa gtgataggca aagaactaag cactcagtat aggggaacca agcacaccgc     240
gggtgagaag gagagggatt gacaagagcc aaggggcgga cgtgggaggg atccagatga     300
ccccatttac tcagagggag gacagacagc tgtactgatt gtgcagagag gcacgtaagg     360
ggccctaggc ggccctggga tcacagatcc cctgaatggg agcccagagt cctaacccag     420
cagattccca ggcagggaag gctggcacca aagctcgcct ggagaacctg ctgcacgccc     480
tccccaacac gcaggctctc agatgccctg cagggtcaag aggcccagga gggccaggcc     540
tcaaggctgg gcgggggatg ggtagggtt gtcccagcct cctgccaaca ccaccatccc     600
```

tgtcaccggg caacgggagg ccaggcgcta tggctcctca gccaaggcct tctctgccta 660
gggctgagaa cagcccctct acagcaagcc cccgtcaaca tgcttccccc aacccaaaca 720
atcaatccaa caaggcagct ttgttctggg ttggggaccc aggggcagcg cctcccggag 780
gagcccctc ccttagggcg ctcctggcgg ccctgttcga acacccgtag cccccgcctc 840
cggtatgtgc ctcctccgtg tactctgtct cacttgccct ttgacaagaa tcaaactctc 900
agttgggtgg cttcccctcg actccatccc acaccacccg ttttccctgg tccctccaca 960
tcctctccat cccctactca ttccctctgc ggttagcgtg actcccccta ccttggccga 1020
gcaggaccct aagggctggg agaagagtgc tggtcctgga gatcagtcct aggacttcca 1080
aaaaaaaaaa aaaatgctcg gccaggggag actggacccc cctctctccc ccttctccgt 1140
tcgtccctcc cacatccccc tgagccctcc cacattccgc cttctccttc cattcctccc 1200
ggccagttcc ctcctctcct cctctgggtc ctccctcccc gtggagctgg ccactgccgc 1260
gcccccctccc cctccctggc cctcccggct cgttcaatga agctttctct tcccgcggca 1320
tggaggccag gcctctgcgg tcagggagct gacatgcaag cggcggccgg gcggcggggc 1380
ggccccggtg gtggcgggaa gcggttcaac cccagccctc cctccgagtc ccgggccagc 1440
ggagtctaaa cagcccggct cgggcggggc tgggggcggg cccggccagg gtaaataaag 1500
ccccggaggc ccgtgtgggt ctctccggtg cccggggcgc tttcggggtg atgtcatggc 1560
tttcactctg acgtcacctt gggcatatga cgtcacgcgc agccaccacg cccagaggcc 1620
agggccctcg cgccaaaacc gaggactttc ggtggagcca ctggcgcgcc cccacgacgg 1680
cgggtagggg cctggggtgc tgcagctccc ggctaatccg gggtctccca ccattccttc 1740
tcgctcctct ccctcacccc accgcgaccc ccccagcggc tgccggcggg ggccggggag 1800
gggcggaaac tcctccaggg aacccggccc cgcctcccgc cccgcctccc gccgcagggc 1860
caggaagcgc ggaaggaacc gccgggggcc atggacggag cagtgatgga agggccgctt 1920
tttttgcaga gtcagcgctt tgggaccaag gtagtctggc gcatggatgc cgaaccttat 1980
ccgggctagt agtgggtgag agagcccaga aacagggaga ggtgggcagc gggctggaga 2040
gcggcgccgg gagttggaga gcctgtgact ttcccgtgaa gttgctttgc acactcccgg 2100
gaagcgtctg tagtctaggg gttctggtcc tggggagacg gagtggcatc gtccttggga 2160
aacttcgccc ccaaccccgt ttggaagccc cagatcccaa atcgacttgc gccgcaacct 2220
ccttccccgt cgggacccgg gccgcctgcg cacgccactc cctctcgagc actctctctc 2280
tctccctaga ggtggaggaa gacctgggcc gtgctctacc cggccagtcc ccacggcgta 2340
gcgcggctcg agttctttga ccataagggg tcgagctctg gggtggccg agggagctcg 2400
cgccgcctgg actgcaaagt gatccgtctg gctgagtgtg tgagtgtggc ccccgtcacc 2460

gtggagaccc cccctgagcc cggcgccact gccttccgcc tggacactgc tcagcgctcg 2520

cacctgctgg cggccgacgc gccgtccagt gcagcctggg tgcagacgct gtgccgaaac 2580

gcctttccgg tgaggagctg cggcgatgcg gggtgggggc agttacagag gcagagaaat 2640

ggggctgcct cagaccgacc cccgctcccc gctgaggaaa ttacgggttt ctcgatgctc 2700

tctactacag aaaggcagct ggactctggc gcctaccgat aacccaccta agctttctgc 2760

cctggagatg ctggagaact ccttgtacag ccctacctgg gaaggtagac gcctcagaag 2820

cccgggcagg gatggagtga agaggaggag ggccgagggc ctttgggaag tgggtggata 2880

cccaggggcc catggggaag taagaagtag gaaggccctg agatctggct tccgagtgag 2940

tgcttggaca ctatgctcat gagtcctctg ggtccccact gctgccccg tccctccccc 3000

gcagctgtct aatcgtgtat gccttccagg atcccaattc tgggtaacgg tgcagaggac 3060

tgaggccgcc gagcgctgtg gcctgcatgg ctcctacgtg ctgagggtgg aggctgaaag 3120

gctgactctc ctgaccgtgg gggcccagag tcagatactg gagccactcc tgtcctggcc 3180

ctacactctg ttgcgtcgct atggccggga caaggtgcag gggctgtccg ggagggcttc 3240

ctgggttggg cagctgtggg aggggtgggg caggacagaa agtgggaagc tctgaccttt 3300

ggatccccct ttcttgccta cccggtgacc ccgcgtctgt tcgcaggtgg tctcttcttt 3360

gtagataccc taactagtgc aggcgtgtga ctcacttcct ctgatggctc ctggtaatgt 3420

gtttccccct ctaccctcct taggtcatgt tctctttcga ggccggccgc cgctgcccct 3480

caggccctgg aaccttcacc ttccagacgg cacagggaaa tgacatcttc caggcagttg 3540

agactgccat ccaccggcag aaggcccagg gaaaggccgg acaggggcac gatgttctca 3600

gagctgactc ccatgaaggg gaggtggcag aggggaagtt gccttcccca cctggccccc 3660

aagagctcct cgacagtccc ccagccctgt atgctgagcc cttagactcc ctgcgcattg 3720

ctccatgccc ttcccaggac tccctatact cagacccctt ggacagcacg tctgctcagg 3780

caggagaggg agtacaacgg aagaaacctc tctattggga cttgtatgag catgcgcagc 3840

agcagttgct gaaggccaag ctgacagacc ccaaagagga tcccatctat gatgaacctg 3900

agggcctggc cccagtccct ccccagggcc tttatgatct gcctcgggag cccaaggatg 3960

catggtggtg ccaagctcgg gtgaaggagg agggctatga gctcccctac aaccctgcca 4020

ctgatgacta cgctgtgcca ccccctcgga gcacaaagcc cctccttgct cccaagcccc 4080

agggcccagc cttccctgaa cctggtactg caactggcag tggcatcaaa agccacaact 4140

cagccctgta cagccaggtc cagaagagcg gggcctcagg gagctggga 4189

<210> 6
<211> 4182
<212> DNA

<213> Homo sapiens

<400> 6

attacttgaa cccgggaggc gcaggctgca gtgagccgag atggtgccat tgtactccag 60
cctgggcaac aagagtgaaa ctacgtctca aataaaaaga gagaaagaaa agaaaaaaga 120
aaagaaagag aaagaaaaga aaaaaaaaaa agaaaaaaga ttcttctggg accaggtggg 180
gataggactg gaagctgaga ggataggagg aggttgggac aatggtccag aagaaagagg 240
atgaggactg agccagggcc agcagaacag gtgtaagagt ttccatagca tccccagtac 300
ctgcaagagg gacccctctg gaggaatcac gcagtccaca cactgggtca ggtccccgat 360
gagctccgag tcccccagga agctgtcgat gaggcaataa ctggcctagg agaggaagaa 420
gggaccagcc taggattcaa gtgggcccag ggtcagacaa ccaagaacct gtgggaccag 480
gctgccccag gggctgctgg gaactgtagt tcccaaatgg cagtgcccag gtatcctaac 540
ttcccaccct catgtcctgc aacctcaccc tcaagtcctt cttcagtctc tccacgttgc 600
ggatattggt cagttcttca agtcccacaa gaaggaccta gaagaatcat ataggagagg 660
atgggcgagg ggctgggact tgaactcctg ggtctgaagg aagaggggc tgggactccc 720
acttctgggt ctggggactg gggagtcaag attcctgggt tctggccggg cgcggaggct 780
catgcctgta attccagcac tttgggaggc caaggtgggt ggatcacttg aggtcaggag 840
tttgagacca gcctggccaa tatggtgaaa ccccgtctct acaaaaaata caaaaattag 900
ctgggcatgg tggcacgtgc ctgtaatccc agctacttgg gaagctgagg cacgagaatc 960
gcttgaacct gggaggcaga ggttgcagtg agccgagatc aagccactgc actccagcct 1020
gggcaacaga gcgagactcc gtctcaaaac aaaacaaaca aacaaacaaa agattcctgg 1080
gttctgagga aggaagtgtt ggtggtctgc actcctaggt ctgagggaaa aggatgctga 1140
gatctcagat tcctggtctg gggagaaaag aggactggtg agtctcacca tggctccaaa 1200
caccatttgg agtagtctct ccagcctcag ctcagagatg cccacctcag atgacagaac 1260
aatgagggtg atgctgtgga atggaagtga gaagaatgag tcaaggcctg gggaatcagg 1320
aaggggtatg ttggagtccg cccattgatc ctagaacacc agatacatcc tctctcagac 1380
caacctgcct ttcttataga gaacatcagg gatcaaagag ggtaactggc ttgctaaaga 1440
tcccatagcc aaattaacag ctgcttttag agagctggaa ggcctccggg gtcatcttca 1500
acagggacta tcacccaggc cctgtccctg tcccacgttc cctcctgggg acctgtcatg 1560
gaagcttttc cacaccacag tcgtgttctc ggtcctcgca gagctcagct gcacctccag 1620
attctgccca aacatgtgga ctccattgag ggaaccgatg acagagaacg ggagctaagg 1680
aggggttagg gacatcagac aagagcaccc ccccatcccc ttccccctta ggacccaagt 1740
gtgccagctc ccagccccat cctctttcca tatgacctgg cagtgcctct ttggggaacc 1800
aggcttattg gtccattgcc tcctcggtcc tacaaattca acactgaaac ttccccagcc 1860

```
cctactgcct tgaagaccca gagtcaaaac accgaacccc cttcggttta gatacaggcg    1920
tccaaggccc cacctgctga cgggcggggg cgccgccgcg actgctcctg cagaataggg    1980
ggaccccgct ggaggccgcg aggcacagca gatgcacagt gccgcccgtc ccctcctccc    2040
ccatttagga ctcccaccgc ggtccctcac gtggggactg tcagtgcggg tcttggagca    2100
tggcggtaat cagagtaact cggcctgtgg tccggagccg ccagagggcg agatggggag    2160
ctgattggaa gaggattaac ttcccgcctt cttcacccaa ctcaaacaga ccctcaaacc    2220
ctattcaggc acctccccca aacccattag gttctcttcc gccctagccg gaagtcacgg    2280
tacccctaac aaagatggcg gcaaattgaa tttgcaaagc tttctagttt ctccgaggag    2340
gtaaagagaa ccaagaagcc ggattgaaga tgatgactgc gccaaggcgt tgcctagcca    2400
ccatcctgta ggctttgatg atttacattc taaacggaaa aaacagctgt gcccggatca    2460
aagattgcga tggtcccaaa gcattgccta gcaaccacat tccctgtagg tttccagtat    2520
atagtagtaa acaatgtgcc ctactcaaag atggcgaccg tggcggggcg ttgccaagca    2580
accaccattc agcctgatat ccattccaag gctgtaaaca gctgtgccct gctcaaagat    2640
ggcggcacgt cgaagtccga gcaccctcct tggggcgaaa tcgagaaaaa caccatctcc    2700
cctacaacct tcgagggtgg ttgagtccag cgatcgttgc ctagtaacca ctaaaaaaaa    2760
ggttgttttt gttattagaa aaaagaaaac aaggaaatcc tttattttcc agaataataa    2820
aaataatatc atttctgtaa tgaatttata catgatagca taccatgtaa aatgtgaaaa    2880
atcccttccc caattttttt cctaaaatac agtttaacgt agactattca tacgttttca    2940
caaagatgat caatatacta acatttccac actttctttt ttgccatgaa aaagtatgtc    3000
tatttcatct taataatgta tttttataat ctattttatg gatgtgacaa attgatttga    3060
ccaattctgt attgatgtac attaggttgt tgaactcctc cagactagcc aactagtgct    3120
gggacagcat tttactgact ttttaaaatg cccttatgaa tatttttatt attttacaca    3180
ggcagcattt acttgtaatg acaaaagtat ttatgattca tttttccctt cctcttcctt    3240
cttgcagatc agcccatctt tctagcataa tttttaattt tcctacttcc taaagtacat    3300
ctttttctct tttttttttt tttgggagag gggacagggt ctcgttctgt cgcccaaggt    3360
ggagtgcagt ggaaccatct cggctaactg caacctccgc ctccaggatt caagcgattc    3420
tcctgcctca gcctcctgag tagctgggat tacaggtgtg cccccaccac gtccagctaa    3480
tttttgtagt ttttgtggag acagggtgtc gccatgttgc ccaagctggt ctcaaactct    3540
ttggctcaaa tgatccaccc acctcagcct ctcaaagtgc tgggactaca ggcaggagcc    3600
accatgcctg gcctaggtac cattttaatc ttccttcttg cctattgata ttttttttcc    3660
tttctctgga ttacttttat cttccttaca actttattaa aatgttagtt acacaggcat    3720
```

aaaagagggg gctgctctag atgaaaagaa acttgagaca taatgtctaa atatgtgtgt 3780

ggtctttgtt tggatcctga tacaaataaa ccaactgtaa aaacacattt tgagacaatt 3840

ggaacgtttg attacacact tactgttgga tgatactaag aagttaattt tgttacaggt 3900

aacaatggca tactagtaat gtaagaaagt gttcataaaa ttggaaagat gcctgccaag 3960

cacacaggag taaaatcata tgattgtatg gatttgaagg cttcagcagg ccaggcgcgg 4020

cggcttacgc ctgtaatccc agcactttgg gaggccaagg tgggcgggtc acctgaggtc 4080

tggagttcga gaccagcctg accaacatgg agaaaccccg gctctactaa aaatacaaaa 4140

ttagctgggc gtggtggcgc atgcctgtaa actcagctac tc 4182

<210> 7
<211> 4074
<212> DNA
<213> Homo sapiens

<400> 7

```
tcccaaagtg ctgggattac aggcatgagc caccatgccc ggcccatgga gatgttataa      60
tggaatgtgc ttagagtgat aggaactagg aatggatctt acactttcaa atggttggga     120
aaacatcaaa aaatgtttct ggacacgtga aaatgacacg aaattcaaat ttttgtgtcc     180
ataaagtttt attggaacac cgccaggctc attcttttac gtattgtcta tggttgattt     240
tatgctacga tggcagagtt gagtagttgg gacagagacc atatggctgg caaagcctaa     300
aagatttaca atcaatctct taaagcaaat ggtttgccca cccttgctta gagagagcct     360
ccaggaagaa aagagcagtg agggtgacgt tgaagaaggg ggcaagacta ggtcaccagg     420
ggctccctgt gccccgccca ggcactgaac ttaagctgag ggcaagtggg agccattgaa     480
ggtgcaggca ggagagtgac ctgctcaagt gaatgttgga gtaggatcac tctgcctcct     540
cagcaaggat ggatgaagct gggcacggag gcacctggag caggtagacc agccctaggc     600
agcccggatg gttcagcaaa ggcctctggg actattctgg agcacattcg ctgggatata     660
gaataggtag cgcatccctg caccttcgac gatggcggcg cagagatgtc tgctgcgtac     720
ccacaatgcc ttgtgcctcg caccgcggga ggaagtggct gctctgtagg ccccagaacg     780
gaaccacttg aaaggcggga aacacgtggg ggacgcctct gagccctgag aggaatggcc     840
tagagcaagg ccacgaggag ccagggcacg acacggtggg ccctcggaga accgctggtg     900
ggcaagtggc aggagagtag gctcaagagg gttggcaggg ccgaccgggc ctagggtggc     960
cgggtgaggg gggcggggga ggggaggccc ccccagttac cccaggagtg cgcaggtggc    1020
ttcgggcatg tgagggacgg agggggtcgg acggaaggac acaggccggt gtacagcggt    1080
cacccacgcc ccaagcagac acacagactc tgcagacacc gtaacaggcc cggcaccgtg    1140
cacacatcac aggcccaggc ctctgcacat ggtcacagat gcctatgcga aatcccggcc    1200
```

caggttcacg cgggcacagg cgcatccacg cacacaccgc aacacggatc tgcacgtcca 1260

tgcacacaca ggcacagact cgggcacacg gagtcacacc gacccgggca cgctgcggaa 1320

cgcacccgtc cacgcataca cagaacggga cttgctgccc cgcacgcaca ccaggcccgc 1380

actccgaact acccgcattc cccgcacacg cccccgcgca ggccgccccg ggccgtgggc 1440

cggcgcacgc acggtcacag gaggggcgcg cgcacactcg cactcacaca cactcgctcg 1500

cacacgcaca cactcgatcc agcacgcgcg ggcggcgcgg ggcggcggcg gccggagtct 1560

gcggtgcgag gcgcccccgg cccggcgcgg cggcggagcc cggctggcgg gggaggggag 1620

ggggttgcgg agggggcggg gtgggggccg gagccgcttg agccggcggg agcgggcacc 1680

cctgcgcgcc gcgctcggcc tcgcctccgc gcccccgcg cgcccgccgc ggtccctccc 1740

ccgcgcccgt ccgctcgccg ggcccccgcc gccgccccgg ggtgcagccg agcggccgcg 1800

ccgggtcccc gggacggggt ggaagtgggg gtgggggag gggatcgggg ccgggccggg 1860

gccgcgctgc ctgcgatgcc gggcgcccgc cgcagccgct gccgccggag cccgggatgg 1920

ggcgagaggc tgcggcggac gccagcatct ccccgccggg gaccccgggg gccgcggagc 1980

cgccgccgcc gctgctgccg ccgttgctga gacccagcgg gcgatgggtg agttgacccc 2040

ggcgtcctgg ggggcgccgc ggcccctcta tccccgcgct gcccgccctc ccgcgctgcg 2100

cttggcgccc cccgcccggc tccgcggccc actccccgag cgtgacctta gggggcgggc 2160

gcgggcgcac tggggctggg ggcccggagg ccgcggtggg ggcgggatgc cggggaagcc 2220

gctcggcgcg ggccccctcc ccctacccgc cgctcctccg agctccccgg tccccggagg 2280

gcagagcctc agcgcccgac cccctcccac ggccgggcgg gggacgtccc cgaggcgcgg 2340

ggctcgagcc gtgctttgtg cgcggcaccc cccagcctcc ggcgcggctc ccccacaacc 2400

tccggcgcgg cgcggggctg gggtgggagg cctccgcgcc cgccttcccc acccccgacc 2460

ccagactgcg gcggcggcag ccgaggccca agcgcagtcg ggggctgggc gggtgcgggg 2520

gctgcggccg tagctacccc tggggaccag gcagaggcaa atgtggagag ggcccccggc 2580

catttaccgg ggtgggggcc cccggctcct tgcattgctc tgcccagggc gggggaggga 2640

acctggctga gggagggcgc tataaatatc tgcaaatagt gagttctgga accgggagtg 2700

gggaggggag tgcgcctggg tgtgtgtatg gtggggttcc tgagctgggg tgcagcagga 2760

agggatgccg cgcggccaga cctagtggtc tgaggtcgga tgacccccag ccccggaaga 2820

tgcccctgaa ggcccctgat ggggtgtggg cagacacact accccagtgg gattcctccc 2880

tggttcccca cttcattcct gggacacacc agtccagatt cgccttgatg gggggagttgt 2940

gagcactggc gtgtcgtttg gggaggggga gcagttatga aagatccaga catggagccc 3000

agcagaagga tggaggggag agcccccacc cccaggagct gcagctctga gggggttttc 3060

agtctaagga gtgctaaggg gagccgctct gggtcctagg aaggtttgaa agaggggtcc 3120

cagcccagga ggaagagaga ggttactgga agagatgacg acaggaagct gccagagatc 3180

tctgggtaga gaaaggtggg aagggcgttg tgggccaagg gaatggcatg agcaaaggct 3240

cagaggtgtg agtgtctggc gtgtgttgag aggtggcagt gggaacagtc aggcactgtg 3300

ggtgcccccct ggaagaagtg agatgaaaac ggggcagagg ctgggcaggg aggccaggtg 3360

gaggtgaggt gggagggcac agcagggagc tggcacttgg tcctgaggac ggcagggagc 3420

caagacctgg ctggggggat gccgctggct cagacccatt ctaagggtct gcctggtgcc 3480

cttggtgtgg tcagggctac ttgaggcaca ctggtgattc ctttttcttt tcttttcttt 3540

tctttttttt tttttttttt tgagatggag cctcactctg tcgcccaggc tggagtgcag 3600

tggtgccatc ctggctcact gcaacctcta cctcctgggt tcaagcaatt gtcccacctc 3660

ggcctcccga gtagctggga ccacaggtgc ccgacaccac acccggctaa tttttgtatt 3720

tttagtagag atggggtttc accaggttgg ccaggctggt ctcgaactcc tgacctcaag 3780

tgatccaccc accttggcct cctaaagtgc tgggattaca ggcgtgagcc acctcgccca 3840

accctgatga tttctttttc tgtagtaact ggaccacctc aggaagtcag ctttctgagc 3900

ctcggtttcc acatctgtga aatggggagg ttttgttttt gtttttgttt tgagacaggg 3960

tctcactctg tgatccaagc tggagtgcag tggtgtgatc ataggtcaca gcagccccaa 4020

cctcctgggc tcaagggatc cttctgcccc aacctcctgg gtagctagga ctac 4074

<210> 8
<211> 4160
<212> DNA
<213> Homo sapiens

<400> 8

```
taacaccagg cgtggcacgg ggccgcaagc cttgctccac taggaaggcc agtgtctgtt      60
ccgggtgttt gattctcatg ccttgtgagt tgttaaatat ctttaatatt actccttagg     120
ttcgtatgtt ggtagtagct aaacaccaag ggtgaaatat taatctgagt gaattctcaa     180
caagagatga tcagttttgg ggttcaagga gggagtcggg agatctgaga tgtaactaag     240
gagattctat tcagcactgg cctctgataa tccccctcc cctgaaatcc ctgcagcttc      300
accacactcc tcttgaagct cctgcaggca cttcaaacgc aacacgtttt agattgaaca     360
cattatctct gtctgctaaa tcagctctct ctctgcctcc cctaattagt ggaagagctt     420
ctccaatgat cttatcccct atggtagaaa attcaaaatc atcctagact gtgcctcacc     480
cttatgtcct cttttttta tcacatacca agatctgaca attctattcc atgaaaaaaa      540
tcttttgagt ctgacctctt ctccaatccc attgcagctg tctcctaagt tttggaatag     600
tttcctaaat ggtctcttgg cctctgtatt cgtctgtttt cacactgctg tgaagaaata     660
cctaagactg ggtaatttat aaaggaaaaa ggtttaatta actcacagtt ctgcatgcct     720
```

agagaggcct caggaaactt acagtcatgg cataaaggga agcaaacacg tccttcataa 780
ggcggcagga gagagaagta tgaggaacca agtgggaaga gccccttata aaaccatcag 840
ctctcacgag aactcactca ctatcatgag aacagcatga atgaaactgt ccctatgatc 900
caatctcctc ccaccagctc tctccctaaa cgcatgggga ttatggggat tacaattaga 960
gatgagattt atgcggggac acaaagcaaa accatatcaa cccccaacct cttttgcccc 1020
tccaacactg ctcttggcat tccagttcct gcctggtcca tagcctactc cttcttcatc 1080
tgtggaaggg agggtagtgg gagtcattag cctgggcagg gaggagtgtt ttatcactac 1140
actgtttaga atggtgccaa tacaaagcac atcaactttc agcaagttta ttattgggtt 1200
taaattctct acacacgatg cactgctttt gcctacacct ggacagtgtt cccactcact 1260
ctccttagta tgatgctgga gataatatga tctgccatga aggttaaata aagcaatgtt 1320
cactgagaac ttagcccagg cccctgcaca tgctgagcat tttgttaatg taacaatgat 1380
tagtaatagc attgctattg gttccccatc tctggcaaag cctttcatgc ttggcctccc 1440
tcactctctt tcaccttcct aaaccctaca ctccagctat gccaaactgt ttactctgcc 1500
ttggacacac caagttcctc aagcctttgc aaatactcct tacgctccta gaacaatgga 1560
tgtcaattca tcatttagga tccagcttac agtcatgctt tccatgaagc cttcctcaaa 1620
ttagccacac aaaaggagtg actcctccct gtattcccat gaccctctgc tcatgcctcc 1680
tctttaaagc ctgccttcac tggagttcct ggaagggaga gtctgttcag cactcatctc 1740
tgtatccaca gtcaccaaaa ttgtaactgc cccataataa attcccaata cttgcaggga 1800
ttttgcttct tggcttgttt tgttttgctg gaatgaatga ataagtaaat gggaacgatt 1860
ttgacttcac cctaagccta gctaaaagtt tgggtagtga cagttttggt tattttgtgt 1920
aacctgatag tggatcccct ttctatgcca aaaaaaaaaa aaatccccca atactagagt 1980
acctgttttc ctagggaatt gacctcagca actgaaggag aggcgattca aagcagggag 2040
tgtgatgaat aggtcaactc tgtcctcgca gccgtccctg agagggcgct gttcccaaat 2100
cgaagcccca gaggcggaga ctgagacacc ggcactctcc cactggactg ggtcagcatc 2160
cctctccccg cgcggccgca ggtctagagc tgagcgcctg cccacaaaca tggcggcgcc 2220
ctgcgcggct tcccgtcgcc gcaaccgtgg ggccggccct gccttgccag tactagggga 2280
cttcctctgc gcgccggctt cctgcccagc tggcatttaa accaccgcct ggggctgcag 2340
gatgctgctg cggatgcaga gctgtccgcg ggctgggcag cgtcgccgtc tcccctgagc 2400
cgcctcggtc cggcaggagc ggagccgaag catcccttgc tgcacgcagg gcagagcagg 2460
cgagggctgg gggccgatcg gggaccccgg cttgggaccc cggcatctgg cagtttcctt 2520
gcaggttcaa ctttaattgc caagatttca cccctcctcc tcaagcccag attatttatc 2580

```
ctccctccgg cctgggctgc tggatgcagc agcggctggg cttggtccca ggagcaggga     2640
gagtgcgctc ccggccctcc tagccgcgtg cccgggccat ggtgcggctg agccccgcgc     2700
ttgggtgagg cggcggcgcg gctcggagcc cggcggaccg gtcctacggg acatcttccc     2760
ctgaggagga gtcttcccct ggggctgcgt gccgggggcg agcggcggcc gcgatgttca     2820
gctggctggg tacggacgac cgcccggagga aggaccccga ggttttccag acggtgagtg     2880
aggggctcaa gaaactctac aagagcaagc tgctgccctt ggaagagcat taccgcttcc     2940
acgagttcca ctcgcccgcc ctggaggatg ccgacttcga caacaagccc atggttctgc     3000
tggtgggcca gtactccact gggaagacca ccttcatcag gtgagccggc ccagggtcct     3060
ggcagcccac cccggagccc agcgcctagt ccctccggtc actcctggtg ctccatccca     3120
gacaggggac caatgggaag ccttcagttg agggctctga agagatctgt gtttgtgcag     3180
gcgtcaaaat cttaccccac aatttttgct ggtccccctt cccatcctac ccaatacctg     3240
tccactctac ttggcccttg gctgtggtgg tcaacctgaa gtattcaagt gtaaagccat     3300
tcccagtctc cccatcttat ggatgatgaa accgaggccc aggaggcaca atggaagagc     3360
tcctggtctc tggagcgcct tatgttggtg ggatgtctt acgtcttcac tttttagcaa     3420
cacatcattc acgggcaaaa cctcttccct tgtttttcaa ggggcttggc actcacatgg     3480
gggcctcaca gttgcagctg agttctgatg ggtgcgggcc tgggaacgga ggtggcctgg     3540
ggatgctact tgtcctcctt ttatcatttc cttctctaaa gcagagctcc tgtgtatata     3600
tcttggagac tcaacagaag ctgagtccag aagcagaatg cttttttttt cccctttctc     3660
ttatttgatc ttaatggctt aataataata ttaaagacgc ttagagatgg aagggatatt     3720
ggcattgtct catccagttg ctgagtttgc aggtgagata accgaggcgc agagaagtta     3780
aatgaattgt gcagagccat ctaaagagtg ggcagcagac ctgggatgtt aagtcctaga     3840
tgcatgtgag gagatgcttg gctaaacgcc tgtaactctg agaatgagtc atccattccc     3900
ttcaattaca gaatccttgg gctggaggga ggctcaagaa gtcaatttgt tcaccctgca     3960
ttcagagaag tccaccagag gcccttctta tctttagaga acaggcttca ctccagtcct     4020
caatcttccc aacttcaagg ccagccattt tcttggcccc ttttatagcc attccaaatt     4080
gttgagaatg ctgtggccct ctgtcttaag actcaggctc ccacagcttg agaatggagt     4140
ttggtggccc cccaaagtca                                                  4160
```

<210> 9
<211> 35
<212> DNA
<213> Artificial Sequence

<220>
<223> Forward primer for amplifying DNA fragments to be subjected to an analysis of the methylation of GDF7 promotor.

<400> 9
aggaagagag gaaaatgtaa ttttaaagga aaagg 35

<210> 10
<211> 54
<212> DNA
<213> Artificial Sequence

<220>
<223> Reverse primer for amplifying DNA fragments to be subjected to an analysis of the methylation of GDF7 promotor.

<400> 10
cagtaatacg actcactata gggagaaggc tcraaaacca aatctcaaaa aaac 54

<210> 11
<211> 31
<212> DNA
<213> Artificial Sequence

<220>
<223> Forward primer for amplifying DNA fragments to be subjected to an analysis of the methylation of ZNF132 promotor.

<400> 11
aggaagagag ttgttaaggt gattgaggaa t 31

<210> 12
<211> 51
<212> DNA
<213> Artificial Sequence

<220>
<223> Reverse primer for amplifying DNA fragments to be subjected to an analysis of the methylation of ZNF132 promotor.

<400> 12
cagtaatacg actcactata gggagaaggc tccaaactat aattaaccaa c 51

<210> 13
<211> 30
<212> DNA
<213> Artificial Sequence

<220>
<223> Forward primer for amplifying DNA fragments to be subjected to an analysis of the methylation of CPXM1 promotor.

<400> 13
aggaagagag ggggttgatt tttaaggggt 30

<210> 14
<211> 57
<212> DNA
<213> Artificial Sequence

<220>
<223> Reverse primer for amplifying DNA fragments to be subjected to an analysis of the methylation of CPXM1 promotor.

<400> 14
cagtaatacg actcactata gggagaaggc tccaccaaat aatctctaat ctcttca 57

<210> 15
<211> 30
<212> DNA
<213> Artificial Sequence

<220>
<223> Forward primer for amplifying DNA fragments to be subjected to an analysis of the methylation of RPL39L promotor.

<400> 15
aggaagagag tttaagttgt gagtttttgg 30

<210> 16
<211> 52
<212> DNA
<213> Artificial Sequence

<220>
<223> Reverse primer for amplifying DNA fragments to be subjected to an analysis of the methylation of RPL39L promotor.

<400> 16
cagtaatacg actcactata gggagaaggc tatcccccat tcttccttaa tt 52

<210> 17
<211> 30
<212> DNA
<213> Artificial Sequence

<220>
<223> Forward primer for amplifying DNA fragments to be subjected to an analysis of the methylation of DOK1 promotor.

<400> 17
aggaagagag agagtttaga aatagggaga 30

<210> 18
<211> 54
<212> DNA
<213> Artificial Sequence

<220>
<223> Reverse primer for amplifying DNA fragments to be subjected to an analysis of the methylation of DOK1 promotor.

<400> 18
cagtaatacg actcactata gggagaaggc taacttccaa acraaattaa aaac 54

<210> 19
<211> 30
<212> DNA

<213> Artificial Sequence

<220>
<223> Forward primer for amplifying DNA fragments to be subjected to an analysis of the methylation of FUZ promotor.

<400> 19
aggaagagag aggtatagta gatgtatagt 30

<210> 20
<211> 51
<212> DNA
<213> Artificial Sequence

<220>
<223> Reverse primer for amplifying DNA fragments to be subjected to an analysis of the methylation of FUZ promotor.

<400> 20
cagtaatacg actcactata gggagaaggc taaattaatc ctcttccaat c 51

<210> 21
<211> 30
<212> DNA
<213> Artificial Sequence

<220>
<223> Forward primer for amplifying DNA fragments to be subjected to an analysis of the methylation of EHD3 promotor.

<400> 21
aggaagagag gattttagta attgaaggag 30

<210> 22
<211> 51
<212> DNA
<213> Artificial Sequence

<220>
<223> Reverse primer for amplifying DNA fragments to be subjected to an analysis of the methylation of EHD3 promotor.

<400> 22
cagtaatacg actcactata gggagaaggc taatactaac ccaatccaat a 51

<210> 23
<211> 25
<212> DNA
<213> Artificial Sequence

<220>
<223> Forward M primer for amplifying DNA fragments to be subjected to an analysis of the methylation of FUZ promotor using Methylation Specific PCR method.

<400> 23
atagtagatg tatagtgtcg ttcgt 25

<210> 24

<211> 24
<212> DNA
<213> Artificial Sequence

<220>
<223> Reverse M primer for amplifying DNA fragments to be subjected to an analysis of the methylation of FUZ promotor using Methylation Specific PCR method.

<400> 24
caaaccgaat tactctaatt accg 24

<210> 25
<211> 27
<212> DNA
<213> Artificial Sequence

<220>
<223> Forward U primer for amplifying DNA fragments to be subjected to an analysis of the methylation of FUZ promotor using Methylation Specific PCR method.

<400> 25
gtatagtaga tgtatagtgt tgtttgt 27

<210> 26
<211> 24
<212> DNA
<213> Artificial Sequence

<220>
<223> Reverse U primer for amplifying DNA fragments to be subjected to an analysis of the methylation of FUZ promotor using Methylation Specific PCR method.

<400> 26
aaccaaatta ctctaattac cacc 24

<210> 27
<211> 24
<212> DNA
<213> Artificial Sequence

<220>
<223> Forward M primer for amplifying DNA fragments to be subjeccted to an analysis of the methylation of EHD3 promotor using Methylation Specific PCR method.

<400> 27
attttagtaa ttgaaggaga ggcg 24

<210> 28
<211> 24
<212> DNA
<213> Artificial Sequence

<220>
<223> Reverse M primer for amplifying DNA fragments to be subjected to an analysis of the methylation of EHD3 promotor using Methylation Specific PCR method.

<400> 28
ccaatccaat aaaaaaatac cgat 24

<210> 29
<211> 25
<212> DNA
<213> Artificial Sequence

<220>
<223> Forward U primer for amplifying DNA fragmnets to be subjected to an analysis of the methylation of EHD3 promotor using Methylation Specific PCR method.

<400> 29
attttagtaa ttgaaggaga ggtga 25

<210> 30
<211> 24
<212> DNA
<213> Artificial Sequence

<220>
<223> Reverse U primer for amplifying DNA fragments to be subjected to an analysis of the methylation of EHD3 promotor using Methylation Specific PCR method.

<400> 30
acccaatcca ataaaaaaat acca 24

<210> 31
<211> 18
<212> DNA
<213> Artificial Sequence

<220>
<223> Forward M primer for amplifying DNA fragments to be subjected to an analysis of the methylation of MGAT3 promotor using Methylation Specific PCR method.

<400> 31
tcgttgttga gatttagc 18

<210> 32
<211> 19
<212> DNA
<213> Artificial Sequence

<220>
<223> Reverse M primer for amplifying DNA fragments to be subjected to an analysis of the methylation of MGAT3 promotor using Methylation Specific PCR method.

<400> 32
aaaataaaaa aaccgcgac 19

<210> 33
<211> 18
<212> DNA
<213> Artificial Sequence

<220>
<223> Forward U primer for amplifying DNA fragments to be subjected to an analysis of the methylation of MGAT3 promotor using Methylation Specific PCR method.

<400> 33

ttgttgttga gatttagt 18

<210> 34
<211> 20
<212> DNA
<213> Artificial Sequence

<220>
<223> Reverse U primer for amplifying DNA fragments to be subjected to an analysis of the methylation of MGAT3 promotor using Methylation Specific PCR method.

<400> 34
aaataaaaaa accacaacac 20

<210> 35
<211> 10
<212> DNA
<213> Artificial Sequence

<220>
<223> Tag sequence fused to 5' end of the forward primers.

<400> 35
aggaagagag 10

<210> 36
<211> 31
<212> DNA
<213> Artificial Sequence

<220>
<223> The sequence of T7 promotor fused to 5' end of the reverse primers.

<400> 36
cagtaatacg actcactata gggagaaggc t 31

<210> 37
<211> 169
<212> DNA
<213> Artificial Sequence

<220>
<223> The sequence of a DNA fragment used for analysing the methylation of GDF7 promotor in MassArray analysis.

<400> 37

**gaaaatgcaa ttttaaagga aaaggacccg cctgcccttc ttcggcagtt cgtttggccc 60**

**aggccttcga tcagctgaaa gtttccgatc tagggggatg tccaggctgc cggcggagca 120**

**gagccgataa cccccctctc ctgcgcgttc ccctgagact tggcctccg 169**

<210> 38
<211> 162
<212> DNA
<213> Artificial Sequence

<220>
<223> The sequence of a DNA fragment used for analysing the methylation of ZNF132 promotor in MassArray analysis.

<400> 38

```
ttgccaaggt gattgaggaa tggcgtttat tgcgtcgctg ctcaggcaac gcaaactaca      60
ttatccagaa ggaccctcgc ggtgcctcag ggctggccat tggcagccga ggagacaggc     120
acttccgggc ggagtgtaag acgctggcca atcacagcct gg                        162
```

<210> 39
<211> 148
<212> DNA
<213> Artificial Sequence

<220>
<223> The sequence of a DNA fragment used for analysing the methylation of CPXM1 promotor in MassArray analysis.

<400> 39

```
ggggctgacc tccaaggggc cgccccaagt ctcaggaccg ccgactctgc ccttcctctc      60
cgccgtctgg ggccaacccg cctcgttggc gcgctgcgcc tttatagtct gcaaagccac     120
actgaagaga ccagagatca cctggtgg                                        148
```

<210> 40
<211> 151
<212> DNA
<213> Artificial Sequence

<220>
<223> The sequence of a DNA fragment used for analysing the methylation of RPL39L promotor in MassArray analysis.

<400> 40

```
cccaagctgt gagcctctgg ccccgccagg gcgtacgaat cctttcctcg tttatccagt      60
attaagtcac gctgtgtgct aagtaccgtg gccaggcagc agctagacaa cgcgctgagg     120
taaaatagag aactaaggaa gaatggggga c                                    151
```

<210> 41
<211> 189
<212> DNA
<213> Artificial Sequence

<220>
<223> The sequence of a DNA fragment used for analysing the methylation of DOK1 promotor in MassArray analysis.

<400> 41

agagcccaga aacagggaga ggtgggcagc gggctggaga gcggcgccgg gagttggaga 60

gcctgtgact ttcccgtgaa gttgctttgc acactcccgg gaagcgtctg tagtctaggg 120

gttctggtcc tggggagacg gagtggcatc gtccttggga aacttcgccc ccaaccccgt 180

ttggaagcc 189

<210> 42
<211> 182
<212> DNA
<213> Artificial Sequence

<220>
<223> The sequence of a DNA fragment used for analysing the methylation of FUZ promotor in MassArray analysis.

<400> 42

aggcacagca gatgcacagt gccgcccgtc ccctcctccc ccatttagga ctcccaccgc 60

ggtccctcac gtggggactg tcagtgcggg tcttggagca tggcggtaat cagagtaact 120

cggcctgtgg tccggagccg ccagagggcg agatggggag ctgattggaa gaggattaac 180

tt 182

<210> 43
<211> 160
<212> DNA
<213> Artificial Sequence

<220>
<223> The sequence of a DNA fragment used for analysing the methylation of EHD3 promotor in MassArray analysis.

<400> 43

gacctcagca actgaaggag aggcgattca aagcagggag tgtgatgaat aggtcaactc 60

tgtcctcgca gccgtccctg agagggcgct gttcccaaat cgaagcccca gaggcggaga 120

ctgagacacc ggcactctcc cactggactg ggtcagcatc 160

<210> 44
<211> 123
<212> DNA
<213> Artificial Sequence

<220>
<223> The sequence of a DNA fragment used for analysing the methylation of FUZ promotor with Methylation Specific PCR method.

<400> 44

acagcagatg cacagtgccg cccgtcccct cctcccccat ttaggactcc caccgcggtc 60

cctcacgtgg ggactgtcag tgcgggtctt ggagcatggc ggtaatcaga gtaactcggc 120

ctg 123

<210> 45
<211> 150
<212> DNA
<213> Artificial Sequence

<220>
<223> The sequence of a DNA fragment used for analysing the methylation of EHD3 promotor with Methylation Specifc PCR method.

<400> 45

acctcagcaa ctgaaggaga ggcgattcaa agcagggagt gtgatgaata ggtcaactct 60

gtcctcgcag ccgtccctga gagggcgctg ttcccaaatc gaagccccag aggcggagac 120

tgagacaccg gcactctccc actggactgg 150

<210> 46
<211> 74
<212> DNA
<213> Artificial Sequence

<220>
<223> The sequence of a DNA fragment for analysing the methylation of MGAT3 promotor with Methylation Specific PCR method.

<400> 46

ccgttgctga gacccagcgg gcgatgggtg agttgacccc ggcgtcctgg ggggcgccgc 60

ggcccctcta tccc 74

<210> 47
<211> 169
<212> DNA
<213> Artificial Sequence

<220>
<223> The sequence of a DNA fragment after bisulfite modification used for analysing the methylation of GDF7 promotor in MassArray analysis.

<400> 47

gaaaatguaa ttttaaagga aaaggauucg uutguuuttu ttcgguagtt cgtttgguuu 60

agguuttcga tuagutgaaa gtttucgatu tagggggatg tuuaggutgu cggcggagua 120

gagucgataa uuuuuututu utgcgcgttu uuutgagaut tgguutucg 169

<210> 48
<211> 162
<212> DNA
<213> Artificial Sequence

<220>
<223> The sequence of a DNA fragment after bisulfite modification used for analysing the methylation of ZNF132 promotor in MassArray analysis.

<400> 48

**ttguuaaggt gattgaggaa tggcgtttat tgcgtcgutg utuagguaac guaaautaua 60**

**ttatuuagaa ggauuutcgc ggtguutuag ggutgguuat tgguagucga ggagauaggu 120**

**auttucgggc ggagtgtaag acgutgguua atuauaguut gg 162**

<210> 49
<211> 148
<212> DNA
<213> Artificial Sequence

<220>
<223> The sequence of a DNA fragment after bisulfite modification used for analysing the methylation of CPXM1 promotor in MassArray analysis.

<400> 49

**ggggutgauu tuuaaggggu cguuuuaagt utuaggaucg ucgaututgu uuttuututu 60**

**cgucgtutgg gguuaauucg uutcgttggc gcgutgcguu tttatagtut guaaaguuau 120**

**autgaagaga uuagagatua uutggtgg 148**

<210> 50
<211> 151
<212> DNA
<213> Artificial Sequence

<220>
<223> The sequence of a DNA fragment after bisulfite modification used for analysing the methylation of RPL39L promotor in MassArray analysis.

<400> 50

**uuuaagutgt gaguututgg uuucguuagg gcgtacgaat uutttuutcg tttatuuagt 60**

**attaagtuac gutgtgtgut aagtaucgtg guuagguagu agutagauaa cgcgutgagg 120**

**taaaatagag aautaaggaa gaatggggga u 151**

<210> 51
<211> 189
<212> DNA
<213> Artificial Sequence

<220>
<223> The sequence of a DNA fragment after bisulfite modification used for analysing the methylation of DOK1 promotor in MassArray analysis.

<400> 51

agaguuuaga aauagggaga ggtggguagc gggutggaga gcggcgucgg gagttggaga 60

guutgtgaut ttuucgtgaa gttgutttgu auautuucgg gaagcgutg tagtutaggg 120

gttutggtuu tggggagacg gagtgguatc gtuuttggga aauttcguuu uuaauuucgt 180

ttggaaguu 189

<210> 52
<211> 123
<212> DNA
<213> Artificial Sequence

<220>
<223> The sequence of a DNA fragment after bisulfite modification used for analysing the methylation of FUZ promotor with Methylation Specific PCR method.

<400> 52

auaguagatg uauagtgucg uucgtuuuut uutuuuuuat ttaggautuu uaucgcggtu 60

uutuacgtgg ggautgtuag tgcgggtutt ggaguatggc ggtaatuaga gtaautcggu 120

utg 123

<210> 53
<211> 150
<212> DNA
<213> Artificial Sequence

<220>
<223> The sequence of a DNA fragment after bisulfite modification used for analysing the methylation of EHD3 promotor with Methylation Specific PCR method.

<400> 53

auutuaguaa utgaaggaga ggcgattuaa aguagggagt gtgatgaata ggtuaautut 60

gtuutcguag ucgtuuutga gagggcgutg ttuuuaaatc gaaguuuuag aggcggagau 120

tgagauaucg guaututuuu autggautgg 150

<210> 54
<211> 74
<212> DNA
<213> Artificial Sequence

<220>
<223> The sequence of a DNA fragment after bisulfite modification used for analysing the methylation of MGAT3 promotor with Methylation Specific PCR method.

<400> 54

ucgttgutga gauuuagcgg gcgatgggtg agttgauuuc ggcgtuutgg ggggcgucgc 60

gguuuututa tuuu 74

<210> 55
<211> 182
<212> DNA
<213> Artificial Sequence

<220>
<223> The sequence of a DNA fragment after bisulfite modification of FUZ promotor in MassArray analysis.

<400> 55

```
agguauagua gatguauagt gucguucgtu uuutuutuuu uuatttagga utuuuaucgc       60
ggtuuutuac gtggggautg tuagtgcggg tuttggagua tggcggtaat uagagtaaut      120
cgguutgtgg tucggagucg uuagagggcg agatggggag utgattggaa gaggattaau      180
tt                                                                     182
```

<210> 56
<211> 160
<212> DNA
<213> Artificial Sequence

<220>
<223> The sequence of a DNA fragment after bisulfite modification used for analysing the methylation of EHD3 promotor in MassArray analysis.

<400> 56

```
gauutuagua autgaaggag aggcgattua aaguagggag tgtgatgaat aggtuaautu       60
tgtuutcgua gucgtuuutg agagggcgut gttuuuaaat cgaaguuuua gaggcggaga      120
utgagauauc gguaututuu uautggautg ggtuaguatu                            160
```

<210> 57
<211> 123
<212> DNA
<213> Artificial Sequence

<220>
<223> The region in FUZ promotor amplified with U primers in Methylation Specific PCR method.

<400> 57

```
guauaguaga tguauagtgu tguutgtuuu utuutuuuuu atttaggaut uuuautgtgg       60
tuuutuatgt ggggautgtu agtgtgggtu ttggaguatg gtggtaatua gagtaauttg      120
guu                                                                    123
```

<210> 58
<211> 152
<212> DNA
<213> Artificial Sequence

<220>
<223> The region in EHD3 promotor amplified with U primers in Methylation Specific PCR method.

<400> 58

```
auutuaguaa utgaaggaga ggtgattuaa aguagggagt gtgatgaata ggtuaautut    60
gtuuttguag utgtuuutga gagggtgutg ttuuuaaatt gaaguuuuag aggtggagau   120
tgagauautg guaututuuu autggautgg gt                                 152
```

<210> 59
<211> 74
<212> DNA
<213> Artificial Sequence

<220>
<223> The region in MGAT3 promotor amplified with U primers in Methylation Specific PCR method.

<400> 59

```
utgttgutga gauuuagtgg gtgatgggtg agttgauuut ggtgtuutgg ggggtgutgt    60
gguuuututa tuuu                                                      74
```